(19) Europäisches Patentamt
European Patent Office
Office européen des brevets

(11) EP 1 642 976 A1

(12) EUROPEAN PATENT APPLICATION
published in accordance with Art. 158(3) EPC

(43) Date of publication:
**05.04.2006 Bulletin 2006/14**

(21) Application number: **04733178.0**

(22) Date of filing: **14.05.2004**

(51) Int Cl.:
***C12N 15/29*** *(1990.01)* ***C12Q 1/68*** *(1980.01)*

(86) International application number:
**PCT/JP2004/006913**

(87) International publication number:
**WO 2004/101794 (25.11.2004 Gazette 2004/48)**

(84) Designated Contracting States:
**AT BE BG CH CY CZ DE DK EE ES FI FR GB GR HU IE IT LI LU MC NL PL PT RO SE SI SK TR**

(30) Priority: **16.05.2003 JP 2003139513**

(71) Applicant: **HOUSE FOODS CORPORATION**
**Higashiosaka-shi,**
**Osaka 577-8520 (JP)**

(72) Inventors:
- **HIRAO, Takashi,**
**c/o House Foods Corporation**
**Higashiosaka-shi,**
**Osaka 577-8520 (JP)**
- **HIRAMOTO, Masayuki,**
**c/o House Foods Corporation**
**Higashiosaka-shi,**
**Osaka 577-8520 (JP)**
- **WATANABE, Satoshi,**
**c/o House Foods Corporation**
**Higashiosaka-shi,**
**Osaka 577-8520 (JP)**
- **SHONO, Jinji,**
**c/o House Foods Corporation**
**Higashiosaka-shi,**
**Osaka 577-8520 (JP)**

(74) Representative: **Denison, Christopher Marcus et al**
**Mewburn Ellis LLP**
**York House**
**23 Kingsway**
**London WC2B 6HP (GB)**

# (54) QUANTITATIVE PCR DETECTION METHOD FOR PLANT OF SPECIFIED GENUS IN FOOD OR FOOD RAW MATERIAL

(57) Provided is a method of quantifying a specific plant genus in a food or a food ingredient by a PCR method, comprising: (i) preparing a sample for correction where a sample derived from the specific plant genus to be detected and a standard plant sample are mixed in a predetermined ratio, and extracting genomic DNA from the sample for correction; (ii) preparing a test sample where a known amount of the standard plant sample is added to the food or the food ingredient to be examined, and extracting genomic DNA from the test sample; (iii) practicing a quantitative PCR method using the genomic DNAs and primers; and (iv) conducting correction with a standard value for correction determined for the sample for correction to calculate the amount of the specific plant ingredient contained in the test sample.

EP 1 642 976 A1

## Description

### Technical Field

The present invention relates to a method of quantitatively detecting a specific plant genus contained in a food or a food ingredient.

### Background Art

The labeling system of allergenic specific ingredients on products has been implemented in Japan since April 2002. Regarding foods, the labeling of five items, wheat, buckwheat, peanuts, milk, and eggs, as specific ingredients, has thus been made mandatory according to conditions below [the Ministry of Health, Labour and Welfare website: "Labeling of Foods Containing Allergens" (http://www.mhlw.go.jp/topics/0103/tp0329-2b.html); and "Journal of the Food Hygienics Society of Japan (SHOKUHIN EISEIGAKU ZASSHI in Japanese) (Japan), The Food Hygienics Society of Japan, 2002, vol. 43, No. 4, p. j-269-j-271"]. Following this, the Ministry of Health, Labour and Welfare has provided notification of a test for the specific ingredients by a quantitative ELISA method for primary screening that utilizes a polyclonal antibody and a qualitative PCR method (wheat, buckwheat, and peanuts) and western blotting (milk and eggs) for confirmatory testing. In the ELISA method for primary screening, a sample having a quantitative value of 10 ppm or more (in terms of the total amount of proteins from a specific ingredient/the final weight of a product) determined with any of two ELISA kits is assessed as being positive and further confirmed by the PCR method (wheat, buckwheat, and peanuts) or the western blotting (milk and eggs) as a qualitative test, in addition to the investigation of its manufacturing records [the Ministry of Health, Labour and Welfare website: "Inspection Method of Foods Containing Allergens" (SHOKU-HATSU NO. 1106001 (Notification No. 001 (Nov. 6, 2002) of Department of Food Safety, Pharmaceutical and Food Safety Bureau of the Ministry of Health, Labour and Welfare)) (http://wwwhourei.mhlw.go.jp/~hourei/cgi-bin/t_docframe.cgi?MODE=tsuchi&DMODE=CO NTENTS&SMODE=NORMAL&KEYWORD=&EFSNO=4642)].

In general, the ELISA method is a highly sensitive method of protein detection and is a routine technique in the art. However, the ELISA method using a polyclonal antibody, which has relatively high cross-reactivity, may detect non-specific proteins (Enzyme Immunoassay, supervised a translation by Eiji Ishikawa (1989)) and may therefore produce false positives. Examination for false positives requires reconfirmation by other methods.

The ELISA method is highly sensitive, while the method has a limited dynamic range in measurement. The limited dynamic range in measurement means that the accurate measurement of a sample with an unknown concentration may involve preparing test solutions by several serial dilutions and selecting a measurement result of the test solution that falls within a standard curve range. In measurement by ELISA, consideration is generally not given to correction for influences such as the extraction efficiency of each sample to be examined and the inhibition of ELISA reaction. Therefore, caution should be exercised when a quantitative value is determined by measuring a sample, especially a food or the like, which is expected to have wide-ranging processing and contaminants.

For example, ELISA for detecting commercially-available buckwheat protein has detection sensitivity as high as 1 ng/ml for a buckwheat protein standard test solution for a standard curve attached to a kit (0.02 to 0.1 ppm in terms of the total amount of proteins from the specific ingredient/the final weight of a product when diluted 20 to 400 times and subjected to ELISA) [Journal of the Food Hygienics Society of Japan (SHOKUHIN EISEIGAKU ZASSHI in Japanese) (Japan), The Food Hygienics Society of Japan, 2002, vol. 43, No. 4, p. j-275-j-277"; Journal of the Food Hygienics Society of Japan (SHOKUHIN EISEIGAKU ZASSHI in Japanese) (Japan), The Food Hygienics Society of Japan, 2002, vol. 43, No. 4, p. j-277-j.279"; FAST KIT (Food Allergen Screening Test) series -ELISA BUCKWHEAT- «Instruction Manual», Nippon Meat Packers, Inc.; and instruction manual of Morinaga buckwheat measurement kit, Morinaga Institute of Biological Science]. However, for example, when a 2-g aliquot is sampled from a sample containing buckwheat flour that attains concentration of this level in terms of the total amount of proteins from buckwheat/the weight of the sample, the particle of the buckwheat flour might not be sampled from the sample unless the specific ingredient to be detected has a quite fine particle size.

On the other hand, a currently known PCR method for detecting contaminating buckwheat has sensitivity of approximately 5 pg in terms of the amount of buckwheat DNA and can detect approximately 10 ppm of buckwheat in a sample where buckwheat is added into wheat. However, this method known in the art is not capable of quantitative analysis ["Journal of the Food Hygienics Society of Japan (SHOKUHIN EISEIGAKU ZASSHI in Japanese) (Japan), The Food Hygienics Society of Japan, 2002, vol. 43, No. 4, p. j-280-j-282"; and "Outline for 84th Academic Lecture Meeting of the Food Hygienics Society of Japan" (Japan), The Food Hygienics Society of Japan, 2002, p. 104].

The present inventors developed a qualitative PCR method that targets an ITS sequence that is detectable with sensitivity of 1 ppm or more (DNA/DNA), as a method for detecting the presence of a specific plant genus, and filed a Japanese patent application (Japanese Patent Application No. 2002-284222) on September 27, 2002. However, this method is not capable of quantitative analysis.

A certain method of quantifying a genetically-modified crop by PCR measures the amount of a genetically-modified maize ingredient in a maize ingredient by measuring the copy number of a gene sequence specific for the genetically-modified maize and conducting correction with the separately measured copy number of an endogenous gene sequence inherent to maize ["Journal of AOAC INTERNATIONAL" (US), AOAC INTERNATIONAL, 2002, Vol. 85, No. 5, p. 1077-1089].

Specifically, a typical cultivar of pure genetically-modified maize is used to determine a value (ratio to an internal standard) of "the copy number of a recombinant DNA sequence/the copy number of an endogenous gene sequence" in DNA extracted from its seed. Next, a value of "the copy number of a recombinant DNA sequence/the copy number of an endogenous gene sequence" is determined for an unknown sample and multiplied by the reciprocal of the ratio to the internal standard and 100 to measure the content of the genetically-modified maize. This method is suitable for quantifying the content of a recombinant in a sample consisting of the same plant species such as a sample consisting of only maize species, because the copy number is equal in a variety of cultivars of maize and an endogenous gene having a nucleotide sequence universal to the cultivars is used as an internal standard.

However, considering the measurement of the amount of an allergenic specific ingredient present in a mixture consisting of various living species ingredients and inanimate ingredients, it is difficult to find an endogenous sequence available as an internal standard from the DNAs of the various living species. Furthermore, it is impossible to find the endogenous sequence from those having no DNA such as inanimate matters.

## Disclosure of the Invention

Thus, the present inventors have attempted to develop a method having fewer disadvantages as a method of quantitatively detecting a specific ingredient contaminating a food or a food ingredient. That is, the present inventors have made a study of the present invention for the purpose of developing a quantifying method with specificity and sensitivity sufficient for quantitatively detecting a specific ingredient contaminating a food or a food ingredient, which allows correction for influences such as the extraction efficiency of each sample to be examined and the inhibition of detection reaction and has a dynamic range wider than those of ELISA methods.

Specifically, the present inventors have completed the present invention by diligently studying the establishment of a quantitative PCR method of detection, characterized by: conducting correction by use of a sample derived from a standard plant (standard plant sample) in contemplation of influences such as the extraction efficiency of each sample to be examined and the inhibition of detection reaction; having a dynamic range of detection wider than those of ELISA methods known in the art; and having sufficient specificity and sensitivity.

That is, the present invention relates to:

1. a method of quantifying a plant belonging to a specific plant genus in a food or a food ingredient by a PCR method, comprising:

    preparing a sample for correction where a sample derived from the specific plant genus to be detected and a standard plant sample are mixed in a predetermined ratio, and extracting genomic DNA from the sample for correction;
    preparing a test sample where a known amount of the standard plant sample is added to the food or the food ingredient to be examined, and extracting genomic DNA from the test sample;
    practicing a quantitative PCR using a primer set for detecting the sample derived from the specific plant genus to be detected and a primer set for detecting the standard plant sample with the genomic DNA extracted from each of the sample for correction and the test sample as a template;
    determining, as a standard value for correction, a value of the copy number of the DNA derived from the standard plant/the copy number of the DNA derived from the specific plant genus for the sample for correction by the quantitative PCR method; and
    determining a value of the copy number of the DNA derived from the specific plant genus/the copy number of the DNA derived from the standard plant for the test sample by the quantitative PCR method, and correcting the value with the standard value for correction to calculate the amount of the plant belonging to the specific plant genus contained in the food or the food ingredient;

2. the method according to the above 1, wherein the quantitative PCR method is a real-time PCR method;
3. the method according to the above 2, characterized in that the real-time PCR method quantifies DNA based on the amount of emitted light by use of a probe with a fluorescent dye at the 5' end and a quencher at the 3' end that hybridizes to an internal region of a genomic DNA site, which is hybridized with each oligonucleotide of a PCR primer set, wherein light emitted from the fluorescent dye at the 5' end of the probe is suppressed by the quencher at the 3' end, while during Taq polymerase-catalyzed DNA extension from the primer in PCR reaction, the probe is degraded by the 5'→3' exonuclease activity of the Taq polymerase to dissociate the fluorescent dye and the quencher, then causing light emission;

4. the method according to any one of the above 1 to 3, wherein the standard plant belongs to a plant species other than upland weeds and food crops;

5. the method according to the above 4, wherein the standard plant is statice;

6. the method according to any one of the above 1 to 5, wherein the specific plant genus to be detected is the genus *Fagopyrum, Arachis, Triticum,* or *Glycine;*

7. the method according to the above 2 or 3, wherein the standard plant is statice, a primer set for detecting the statice is a set consisting of oligonucleotide having a sequence shown in SEQ ID NO: 57 and oligonucleotide having a sequence shown in SEQ ID NO: 58, and a probe for detecting the statice is oligonucleotide having a sequence shown in SEQ ID NO: 59;

8. the method according to the above 2 or 3, wherein the specific plant genus to be detected is the genus *Fagopyrum,* a primer set for detecting the genus *Fagopyrum* is a set consisting of oligonucleotide having a sequence shown in SEQ ID NO: 14 and oligonucleotide having a sequence shown in SEQ ID NO: 15, and a probe for detecting the genus *Fagopyrum* is oligonucleotide having a sequence shown in SEQ ID NO: 64;

9. the method according to the above 2 or 3, wherein the specific plant genus to be detected is the genus *Arachis,* a primer set for detecting the genus *Arachis* is a primer set consisting of oligonucleotide having a sequence shown in SEQ ID NO: 21 and oligonucleotide having a sequence shown in SEQ ID NO: 26, 65, or 66, and a probe for detecting the genus *Arachis* is oligonucleotide having a sequence shown in SEQ ID NO: 34;

10. a primer set for detecting statice consisting of oligonucleotide having a sequence shown in SEQ ID NO: 57 and oligonucleotide having a sequence shown in SEQ ID NO: 58;

11. a primer set for detecting the genus *Fagopyrum* consisting of oligonucleotide having a sequence shown in SEQ ID NO: 14 and oligonucleotide having a sequence shown in SEQ ID NO: 15;

12. a primer set for detecting the genus *Arachis* consisting of oligonucleotide having a sequence shown in SEQ ID NO: 21 and oligonucleotide having a sequence shown in SEQ ID NO: 26, 65, or 66;

13. a kit for use in a method of detecting a plant belonging to a specific plant genus in a food or a food ingredient, comprising a primer set for detecting a standard plant sample;

14. the kit according to the above 13, further comprising a probe for detecting the standard plant sample;

15. the kit according to the above 13 or 14, wherein the standard plant is statice, and a primer set for detecting the statice is a set consisting of oligonucleotide having a sequence shown in SEQ ID NO: 57 and oligonucleotide having a sequence shown in SEQ ID NO: 58;

16. the kit according to the above 15, further comprising a probe for detecting the statice having a sequence shown in SEQ ID NO: 59;

17. the kit according to any one of the above 13 to 16, further comprising a primer set for detecting the specific plant genus to be detected;

18. the kit according to any one of the above 13 to 16, wherein the specific plant genus to be detected is the genus *Fagopyrum,* and a primer set for detecting the genus *Fagopyrum* is a set consisting of oligonucleotide having a sequence shown in SEQ ID NO: 14 and oligonucleotide having a sequence shown in SEQ ID NO: 15;

19. the kit according to the above 18, further comprising a probe for detecting the genus *Fagopyrum* having a sequence shown in SEQ ID NO: 64;

20. the kit according to any one of the above 13 to 16, wherein the specific plant genus to be detected is the genus *Arachis,* and a primer set for detecting the genus *Arachis* is a set consisting of oligonucleotide having a sequence shown in SEQ ID NO: 21 and oligonucleotide having a sequence shown in SEQ ID NO: 26, 65, or 66;

21. the kit according to the above 20, further comprising a probe for detecting the genus *Arachis* having a sequence shown in SEQ ID NO: 34;

22. the kit according to the above 15, further comprising a statice sample as the standard plant sample;

23. the kit according to the above 13, wherein the standard plant is statice and the specific plant genus to be detected is the genus *Fagopyrum,* the kit further comprising a plasmid for standard curves for the statice and the genus *Fagopyrum* that comprises DNA having an amplification target sequence of the statice and DNA having an amplification target sequence of the genus *Fagopyrum* with the DNAs ligated together;

24. the kit according to the above 13, wherein the standard plant is statice and the specific plant genus to be detected is the genus *Arachis,* the kit further comprising a plasmid for standard curves for the statice and the genus *Arachis* that comprises DNA having an amplification target sequence of the statice and DNA having an amplification target sequence of the genus *Arachis* with the DNAs ligated together;

25. a kit for use in a method of detecting a plant belonging to the genus *Fagopyrum* in a food or a food ingredient, comprising a primer set for detecting the genus *Fagopyrum* consisting of oligonucleotide having a sequence shown in SEQ ID NO: 14 and oligonucleotide having a sequence shown in SEQ ID NO: 15;

26. the kit according to the above 25, further comprising a probe for detecting the genus *Fagopyrum* having a sequence shown in SEQ ID NO: 64;

27. a kit for use in a method of detecting a plant belonging to the genus *Arachis* in a food or a food ingredient, comprising a primer set for detecting the genus *Arachis* consisting of oligonucleotide having a sequence shown in SEQ ID NO: 21 and oligonucleotide having a sequence shown in SEQ ID NO: 26, 65, or 66; and

28. the kit according to the above 27, further comprising a probe for detecting the genus *Arachis* having a sequence shown in SEQ ID NO: 34.

The method of the present invention, that is, the method in which correction for influences such as the DNA extraction efficiency of each sample to be examined and the inhibition of PCR reaction is conducted not by externally adding DNA as a standard to conduct correction for influences such as the inhibition of PCR reaction in a reaction solution but by simultaneously extracting DNA derived form a specific plant genus to be detected (the "specific plant genus to be detected" used herein also encompasses even a specific plant genus to be quantified) and DNA derived from a standard plant from a sample externally supplemented with a standard plant sample other than purified DNA to conduct a quantitative PCR method, is disclosed hereby for the first time. This method allows highly reliable quantification because of being capable of measurement under a condition where influences such as DNA extraction efficiency and the inhibition of PCR reaction are uniform between the standard plant sample and the sample derived from the specific plant genus to be detected. The method of the present invention has an advantage that the method is capable of correction for influences such as DNA extraction efficiency and the inhibition of PCR reaction and even for difference in DNA content among samples to be examined. In addition, quantitative analysis by a PCR method can reliably exclude a false positive, if any, by subjecting its PCR amplification product to DNA sequence analysis, and as such, can be said to have excellent industrial applicability. Accordingly, the present invention is useful for quantitatively detecting a plant belonging to an allergenic specific plant genus contained in a food or a food ingredient.

Thus, in the method of the present invention, a primer set for detecting statice used as a standard plant sample and a primer set for detecting the genus *Fagopyrum* or the genus *Arachis* used as a specific plant genus are also included by the present invention. Besides, a probe for use in combination with these primer sets in detection by a real-time PCR method is also encompassed by the present invention. A kit for use in the method of the present invention comprising either or both of a primer set for detecting a standard plant sample or (and) a primer set for detecting a plant belonging to a specific plant genus to be detected is included in the scope of the present invention. This kit may comprise the above-described probe. The kit may further comprise a standard plant sample. A preferred standard plant is statice, and a preferred sample therefrom is a dried powder of statice plant, with a dried powder of its seed particularly preferred. In addition, the kit may comprise a plasmid for standard curves for a standard plant sample and a specific plant genus that comprises DNA having a sequence of the standard plant sample and DNA having a sequence of the specific plant genus to be detected with the DNAs ligated together, which can be amplified by the primer sets included in the kit.

In the present specification, a "primer for detecting" a given plant or plant genus (including a plant belonging to, i.e., included in the genus) or a sample derived from any of them refers to a primer consisting of oligonucleotide for specifically amplifying a portion of the genomic DNA of the given plant or the plant belonging to the given plant genus in a PCR method. A primer pair for use in a PCR method consisting of two oligonucleotides, forward and reverse primers, may be referred herein to as a "primer set."

Although the primer of the present invention can be used in a quantitative PCR method for quantifying each plant genus, it is obvious that the primer can also be used in the non-quantitative (i.e., qualitative) detection of each plant genus. The use of the primer of the present invention allows the detection of every plant species belonging to a plant genus to be detected. The primer and the primer set of the present invention are advantageous in quantitative and non-quantitative PCR methods.

The term "detection" used herein encompasses both qualitative and quantitative detection.

In the present invention, a primer for specifically amplifying DNA derived from a specific plant genus to be detected is designed. That is, a primer capable of hybridizing under stringent conditions to a nucleic acid molecule having a universal nucleotide sequence of a specific plant genus in a 45S rRNA precursor gene sequence is designed, wherein the primer is a primer (A) having the 3' end complementarily binding to nucleotides in the ITS-1 sequence of the specific plant genus or a primer (B) having the 3' end complementarily binding to nucleotides in the ITS-2 sequence of the specific plant genus when the primer hybridizes to the nucleic acid molecule. After PCR that uses one or more of the primers (A) and (B), the presence of the specific plant genus can be detected based on, as an indicator, the presence of a PCR amplification product containing at least a portion of the ITS-1 or ITS-2 sequence of the specific plant genus.

The phrase "hybridizing under stringent conditions" used herein means that two DNA fragments hybridize to each other under standard hybridization conditions as described by Sambrook J. et al (Expression of cloned genes in E. coli (Molecular Cloning: A laboratory manual (1989)) Cold Spring harbor Laboratory Press, New York, USA, 9.47-9.62 and 11.45-11.61). Specifically, the phrase means that hybridization (e.g., approximately 3.0 x SSC or 2.0 x SSC, 30°C or 37°C) is conducted on the basis of, for example, a Tm value determined by an equation below, followed by washing (e.g., approximately 2.0 x SSC, 30°C, 37°C, 40°C, 44°C, or 48°C or higher; or 1.0 x SSC or 0.5 x SSC, 37°C or higher) under more highly stringent conditions than the hybridization conditions. Selections of "stringent conditions" suitable for hybridization and washing based on, for example, nucleotide sequences hybridizing to each other are well known in the art. When merely the term "hybridizing" is described herein, the term means hybridizing under stringent conditions unless conditions are stated otherwise.

$$Tm = 81.5 + 16.6 (\log_{10}[Na^+]) + 0.41 (\text{fraction } G + C) - (600/N)$$

The term "genus" used herein means a group including the whole plants belonging to the genus or including several species selected from among plants belonging to the genus.

The primer set used in the present invention is a primer pair capable of hybridizing under stringent conditions to a nucleic acid molecule having a universal nucleotide sequence of a specific plant genus in a 45S rRNA precursor gene sequence. It is important that at least one of primers in the primer pair is a primer (A) having the 3' end complementarily binding to nucleotides in the ITS-1 sequence of the specific plant genus or a primer (B) having the 3' end complementarily binding to nucleotides in the ITS-2 sequence of the specific plant genus when the primer hybridizes to the nucleic acid molecule. In this context, the primer (A) also includes a primer hybridizing to a bridging region between the ITS-1 sequence and a 5.8S rRNA gene sequence and a primer hybridizing to a bridging region between the ITS-1 sequence and a SSU rRNA gene sequence. Likewise, the primer B also includes a primer hybridizing to a bridging region between the ITS-2 sequence and a 5.8S rRNA gene sequence and a primer hybridizing to a bridging region between the ITS-2 sequence and a LSU rRNA gene sequence. The primers (A) and (B) each consists of preferably at least 15 bases, more preferably 15 to 30 bases. Because the ITS-1 and ITS-2 sequences contain variable sequences specific to species, the primer (A) or (B) universal and specific to a specific plant genus can be obtained by preferably selecting a nucleic acid molecule having a nucleotide sequence universal and specific to the specific plant genus in the ITS-1 or ITS-2 sequence, as the nucleic acid molecule having a universal nucleotide sequence of a specific plant genus in a 45S rRNA precursor gene sequence. Moreover, one or two or more of the primers (A) and (B) may be used. The use of two or more of the primers (A) and (B) further enhances specificity to the specific plant genus.

In another aspect, the primer (A) is used in combination with a primer (C) capable of hybridizing under stringent conditions to a nucleic acid molecule having a partial nucleotide sequence in a sequence where the ITS-1, 5.8S rRNA gene, ITS-2, and LSU rRNA gene of a specific plant genus are consecutively ligated. Alternatively, the primer (A) is used in combination with a primer (E) capable of hybridizing under stringent conditions to a nucleic acid molecule having a partial nucleotide sequence in a sequence where the SSU rRNA gene and ITS-1 of a specific plant genus are consecutively ligated. In yet another aspect, the primer (B) is used in combination with a primer (D) capable of hybridizing under stringent conditions to a nucleic acid molecule having a partial nucleotide sequence in a sequence where the SSU rRNA gene, ITS-1, 5.8S rRNA gene, and ITS-2 of a specific plant genus are consecutively ligated. Alternatively, the primer (B) is used in combination with a primer (F) capable of hybridizing under stringent conditions to a nucleic acid molecule having a partial nucleotide sequence in a sequence where the ITS-2 and LSU rRNA gene of a specific plant genus are consecutively ligated. In this context, the 5.8S rRNA gene is highly conserved and contains many sequences universal to a large variety of plants. Therefore, the primer (C) is preferably selected from primers capable of hybridizing under stringent conditions to a nucleic acid molecule having a partial nucleotide sequence of the 5.8S rRNA gene and having the 3' end complementarily binding to a nucleotide sequence in the 5.8S rRNA gene sequence when the primer hybridizes to the nucleic acid molecule, or alternatively, the primer (D) is preferably selected from primers capable of hybridizing under stringent conditions to a nucleic acid molecule having a partial nucleotide sequence of the 5.8S rRNA gene and having the 3' end complementarily binding to a nucleotide sequence in the 5.8S rRNA gene sequence when the primer hybridizes to the nucleic acid molecule, thereby making it possible to commonly use the primer to a variety of plants. These primers are fixed, and a primer universal and specific to a specific plant genus desired to be detected is selected from the ITS-1 or ITS-2 region. In this way, primers for detecting the contamination of a plant belonging to the specific plant genus with high sensitivity can easily be designed. The primers (C) to (F) each consist of preferably at least 15 bases, more preferably 15 to 30 bases.

For designing these primers, the primers may be designed based on, for example, "Recent Advances in PCR Methodology: Basic Methodology and it's Application" (Protein, Nucleic Acid and Enzyme, 1996 Supplement, Kyoritsu Shuppan), "Visual Experimental Note Series, Biotechnology Experiments Illustrated 3, PCR for Real Amplification (Hontouni Hueru PCR in Japanese) in Cell Technology Supplement" (Nakayama, H., 1996, Syujunsha), "PCR Technology--Principles and Applications of DNA Amplification--" (Erlich, H.A.(ed.), supervised by Kato, K., Takara Shuzo). However, the primers may be those that can yield an amplification product within 700 bases when a specific plant genus is detected from unprocessed products, whose DNA is less likely to be fragmented. Alternatively, when a specific plant genus is detected from processed products, its DNA is likely to be short due to possible fragmentation. From this point of view, primers that can yield an amplification product within 200 bases are preferred in that high sensitivity can be attained.

Thus, the primer (C) or (D) is preferably a primer capable of hybridizing under stringent conditions to a nucleic acid molecule having a nucleotide sequence shown in SEQ ID NO: 1 or a complementary nucleotide sequence thereof. Because the 5.8S rRNA gene sequence is highly homologous among plants almost across the gene sequence, any primer hybridizing to any region in the gene sequence can be employed. However, the above-described primer is preferred for the reason that the region shown in SEQ ID NO: 1 has especially high homology. More preferred is a primer capable of hybridizing under stringent conditions to a nucleic acid molecule having a nucleotide sequence at positions 11 to 63 in SEQ ID NO: 1 or a complementary nucleotide sequence thereof. Such a primer preferable as the primer (C) is any of oligonucleotides shown in SEQ ID NOs: 2 to 4 (which hybridize to the nucleic acid molecule having the sequence shown in SEQ ID NO: 1). Alternatively, such a primer preferable as the primer (D) is any of oligonucleotides shown in SEQ ID NOs: 5 to 7 (which hybridize to the nucleic acid molecule having the complementary strand of the sequence shown in SEQ ID NO: 1). The above-described primer should specifically hybridize under stringent conditions to the target nucleic acid molecule. Moreover, nucleotides at the 3' end of the primer should be complementary to that of a target DNA sequence portion in order that the hybridized primer may function as a primer to bring about extension reaction. Thus, the primer may be oligonucleotide indicated by any of nucleotide sequences shown in SEQ ID NOs: 2 to 7 with the deletion, substitution, or addition of one or several base(s) as long as the primer meets these requirements.

The nucleotide sequence universal and specific to a specific plant genus in the ITS-1 or ITS-2 sequence can be identified by obtaining the ITS-1-5.8S rRNA gene-ITS-2 sequences of a variety of plants of the specific plant genus to be detected and other plant genera from GenBank, and conducting alignment to search for a region universal and highly specific to the specific plant genus. In addition, a primer sequence can be selected from this identified region by adapting nucleotides at the 3' end of the primer sequence to retain specificity especially to the specific plant genus and its possible related plant species.

For example, when a specific plant genus is the genus *Fagopyrum,* a nucleotide sequence universal and specific to the genus *Fagopyrum* in the ITS-1 sequence of the genus *Fagopyrum* may be selected from the sequence of *Fagopyrum esculentum* (common buckwheat) because a large variety of commercially-available buckwheat are *Fagopyrum esculentum* (common buckwheat) and the actual sequence of commercially-available buckwheat consistent with the sequence of *Fagopyrum esculentum* registered in GenBank. Specific examples of the nucleotide sequence can include a nucleotide sequence shown in SEQ ID NO: 8, 9, or 10 or a complementary nucleotide sequence thereof. Preferred examples thereof can include a nucleotide sequence at positions 11 to 61 in SEQ ID NO: 8 or a complementary nucleotide sequence thereof and a nucleotide sequence at positions 11 to 67 in SEQ ID NO: 9 or a complementary nucleotide sequence thereof. The nucleotide sequence shown in SEQ ID NO: 10 is particularly useful as a region from which a primer for specifically detecting members of the genus *Fagopyrum, F. esculentum* (common buckwheat), *F. tataricum* (Dattan buckwheat), *F homotropicum,* and *F. cymosum,* is selected.

A preferred primer (A) for the genus *Fagopyrum* is any of oligonucleotides shown in SEQ ID NOs: 11 to 16 (which respectively hybridize under stringent conditions to the complementary strand of the nucleotide sequence of SEQ ID NO: 8 (in the case of the oligonucleotides shown in SEQ ID NOs: 11 to 14) and to the nucleotide sequence of SEQ ID NO: 9 (in the case of the oligonucleotides shown in SEQ ID NOs: 15 and 16)). Alternatively, the primer may be oligonucleotide indicated by any of nucleotide sequences shown in SEQ ID NOs: 11 to 16 with the deletion, substitution, or addition of one or several base(s). Examples of a nucleotide sequence universal and specific to the genus *Fagopyrum* in the ITS-2 sequence can include a nucleotide sequence shown in SEQ ID NO: 36 or 37 or a complementary nucleotide sequence thereof These nucleotide sequences are particularly useful as a region from which a primer for specifically detecting members of the genus *Fagopyrum, F. esculentum* (common buckwheat), *F. tataricum* (Dattan buckwheat), *F. homotropicum,* and *F. cymosum,* is selected. The combination of any of the primers of SEQ ID NOs: 11 to 14 with any of the primers of SEQ ID NOs: 15 and 16 or SEQ ID NOs: 2 to 4 is preferably used.

When a specific plant genus is the genus *Arachis,* a nucleotide sequence universal and specific to the genus *Arachis* in the ITS-1 sequence of the genus *Arachis* may be selected from the sequence of *A. villosa* because the actual sequences of commercially-available peanuts consistent with the sequences of *A. correntina* and *A. villosa* registered in GenBank (although a large variety of commercially-available peanuts are *Arachis hypogaea).* Specific examples of the nucleotide sequence can include nucleotide sequences shown in SEQ ID NOs: 17 to 20 or complementary nucleotide sequences thereof. Preferably, the nucleotide sequence is a nucleotide sequence at positions 11 to 62 in SEQ ID NO: 17 or a complementary nucleotide sequence thereof, a nucleotide sequence at positions 11 to 47 in SEQ ID NO: 18 or a complementary nucleotide sequence thereof, a nucleotide sequence at positions 11 to 50 in SEQ ID NO: 19 or a complementary nucleotide sequence thereof, or a nucleotide sequence at positions 11 to 58 in SEQ ID NO: 20 or a complementary nucleotide sequence thereof.

A preferred primer (A) for the genus *Arachis* is any of oligonucleotides shown in SEQ ID NOs: 21 to 31, 65, and 66 (which respectively hybridize under stringent conditions to the complementary strand of the nucleotide sequence of SEQ ID NO: 17 (in the case of the oligonucleotides shown in SEQ ID NOs: 21 to 23), to the complementary strand of the nucleotide sequence of SEQ ID NO: 18 (in the case of the oligonucleotides shown in SEQ ID NOs: 24 and 25), to the complementary strand of the nucleotide sequence of SEQ ID NO:. 20 (in the case of the oligonucleotides shown in SEQ ID NOs: 30 and 31), and to the nucleotide sequence of SEQ ID NO: 19 (in the case of the oligonucleotides shown in SEQ ID NOs: 26 to 29, 65 and 66)). Alternatively, the primer may be oligonucleotide having any of nucleotide sequences shown in SEQ ID NOs: 21 to 31, 65 and 66 with the deletion, substitution, or addition of one or several base(s), as long as the oligonucleotide hybridizes under stringent conditions to each corresponding sequence as described above. Examples of a nucleotide sequence universal and specific to the genus *Arachis* in the ITS-2 sequence of the genus *Arachis* can include a nucleotide sequence shown in SEQ ID NO: 38 or a complementary nucleotide sequence thereof. Preferably, the nucleotide sequence is a nucleotide sequence at positions 11 to 47 in SEQ ID NO: 38 or a complementary nucleotide sequence thereof. A preferred primer (B) for the genus *Arachis* is oligonucleotide shown in SEQ ID NO: 39 (which hybridizes under stringent conditions to the nucleotide sequence of SEQ ID NO: 38). Alternatively, the primer may be oligonucleotide indicated by a nucleotide sequence shown in SEQ ID NO: 39 with the deletion, substitution, or addition of one or several base(s). The combination of any of the primers of SEQ ID NOs: 21, 24, and 25 with any of the primers of SEQ ID NOs: 2 to 4, any of the primers of SEQ ID NOs: 21, 24, and 25 with the primer of SEQ ID NO: 39, the primer of SEQ ID NO: 39 with any of the primers of SEQ ID NOs: 5 to 7, or any of the primers of SEQ ID NOs: 21 to 23, 30, and 31 with any of the primers of SEQ ID NOs: 26 to 29, 65, and 66 is preferably used. However, more preferred is the combination of any of the primers of SEQ ID NOs: 21, 24, and 25 with any of the primers of SEQ ID NOs: 2 to 4 or the primer of SEQ ID NO: 21 with any of the primers of SEQ ID NOs: 26, 65, and 66.

When a specific plant genus is the genus *Triticum,* examples of a nucleotide sequence universal and specific to the genus *Triticum* in the ITS-2 sequence of the genus *Triticum* can include nucleotide sequences shown in SEQ ID NOs: 40 to 42 or complementary nucleotide sequences thereof. Preferably, the nucleotide sequence is a nucleotide sequence at positions 11 to 50 in SEQ ID NO: 40 or a complementary nucleotide sequence thereof, a nucleotide sequence at positions 11 to 47 in SEQ ID NO: 41 or a complementary nucleotide sequence thereof, or a nucleotide sequence at positions 11 to 47 in SEQ ID NO: 42 or a complementary nucleotide sequence thereof.

A preferred primer (B) for the genus *Triticum* is any of oligonucleotides shown in SEQ ID NOs: 43 to 45 (which respectively hybridize under stringent conditions to the complementary strand of the nucleotide sequence of SEQ ID NO: 40 (in the case of the oligonucleotide shown in SEQ ID NO: 43), to the nucleotide sequence of SEQ ID NO: 41 (in the case of the oligonucleotide shown in SEQ ID NO: 44), and to the nucleotide sequence of SEQ ID NO: 42 (in the case of the oligonucleotide shown in SEQ ID NO: 45)). The primer may be oligonucleotide indicated by any of nucleotide sequences shown in SEQ ID NOs: 43 to 45 with the deletion, substitution, or addition of one or several base(s). The combination of the primer of SEQ ID NO: 43 with one or more of the primers of SEQ ID NOs: 44 and 45 is preferably used.

When a specific plant genus is the genus *Glycine,* examples of a nucleotide sequence universal and specific to the genus *Glycine* in the ITS-2 sequence of the genus *Glycine* can include nucleotide sequences shown in SEQ ID NOs: 46, 47 and 48 or complementary nucleotide sequences thereof. Preferably, the nucleotide sequence is a nucleotide sequence at positions 11 to 48 in SEQ ID NO: 46 or a complementary nucleotide sequence thereof, a nucleotide sequence at positions 11 to 55 in SEQ ID NO: 47 or a complementary nucleotide sequence thereof, or a nucleotide sequence at positions 11 to 52 in SEQ ID NO: 48 or a complementary nucleotide sequence thereof.

A preferred primer (B) for the genus *Glycine* is any of oligonucleotides shown in SEQ ID NOs: 49 to 56 (which respectively hybridize under stringent conditions to the complementary strand of the nucleotide sequence of SEQ ID NO: 46 (in the case of the oligonucleotide shown in SEQ ID NO: 49), to the nucleotide sequence of SEQ ID NO: 47 (in the case of the oligonucleotides shown in SEQ ID NOs: 50 to 65), and to the nucleotide sequence of SEQ ID NO: 48 (in the case of the oligonucleotide shown in SEQ ID NO: 56)). The primer may be oligonucleotide indicated by any of nucleotide sequences shown in SEQ ID NOs: 49 to 56 with the deletion, substitution, or addition of one or several base (s). The combination of the primer of SEQ ID NO: 49 with one or more of the primers of SEQ ID NOs: 50 to 56 is preferably used.

For designing these primers and evaluating the designed primers, a PCR simulation may be utilized.

For example, in the design of the primer for detecting the genus *Fagopyrum,* a region universal and highly specific to 21 sequences of plants belonging to the genus *Fagopyrum* including edible buckwheat (common buckwheat and Dattan buckwheat) is found in the ITS-1-5.8S rRNA gene-ITS-2 sequence portions, and a primer sequence can be selected from the region by adapting nucleotides at the 3' end of the primer sequence to retain specificity to other plants. However, the site and number of nucleotides deleted in the ITS-1-5.8S rRNA gene-ITS-2 sequence portion vary according to each species of the genus *Fagopyrum.* Therefore, additional selection is required for obtaining the same size of amplification products from all of the 21 sequences of plants belonging to the genus *Fagopyrum.* If the same size of amplification products can be obtained, the presence of the genus *Fagopyrum* can easily be detected. The simulation demonstrates that the same size of amplification products can be obtained from all of the 21 sequences of plants belonging to the genus *Fagopyrum,* especially by selecting the primer (A) and the primer (C) or two primers (A) for the genus *Fagopyrum.* This allows the design of a primer capable of size-based discrimination against non-specific products with ease.

In the present invention, the above-described primers are used to detect a plant belonging to a specific plant genus to be detected by a PCR method. Alternatively, the plant is quantified by a quantitative PCR method.

For PCR, conditions such as the temperature and time of each of denaturation, annealing, and extension steps, the type and concentration of an enzyme (DNA polymerase), the concentrations of dNTP, primer, and magnesium chloride, and the amount of template DNA are appropriately modified and optimized on the basis of ordinary methods described in, for example, Saiki RK, et al., Science, 230: 1350-1354 (1985) and "Plant Cell Technology Suppl., Plant Cell Technology Series, PCR Experimental Protocols of Plants (Shimamoto, K. and Sasaki, T eds. (1995)).

Alternatively, PCR amplification can be conducted at an annealing temperature of primers and template DNA used in the PCR amplification that is set to a temperature higher than the Tm values of the primers calculated by commercially-available software such as HYB Simulator™ version 4.0 (Advanced Gene Computing Technologies, Inc.) and Primer Express™ version 1.5 (Applied Biosystems), preferably at a temperature of the Tm values + 10 to + 3°C, followed by additional PCR amplification at an annealing temperature that is set to a temperature around the Tm values of the primers, preferably at a temperature of the Tm values + 7 to ± 0°C.

A quantifying method that employs a real-time PCR method is preferred as the quantitative PCR method. Examples of the real-time PCR method include, but not limited to, SYBR Green, Fluorogenic probe (e.g., TaqMan™ probe), Molecular Beacon, and LightCycler™ probe methods. Recently, a variety of real-time PCR methods are energetically developed, and those skilled in the art can practice any of the methods. In order to design a probe used in the method, the probe is selected from a sequence capable of hybridizing under stringent conditions to an internal region of a site hybridized with each PCR primer for an amplification target sequence.

An especially preferred real-time PCR method is a method characterized by quantifying DNA based on the amount of emitted light by use of the specific plant genus-specific primer set designed as described above as well as a probe with a fluorescent dye at the 5' end and a quencher at the 3' end that hybridizes under stringent conditions to an internal region of a site hybridized with each oligonucleotide of a PCR primer set for an amplification target sequence, wherein light emitted from the fluorescent dye at the 5' end of the probe is suppressed by the quencher at the 3' end, while during Taq polymerase-catalyzed DNA extension from the primer in PCR reaction, the probe is degraded by the 5'→3' exonuclease activity of the Taq polymerase to dissociate the fluorescent dye and the quencher, which then emits light. It is not required that the entire probe sequence is encompassed in the internal region of the site hybridized with the PCR primers. There may be a 1 to 10-base or 1 to 5-base overlap between the 3'-terminal bases of the designed probe and the 3'-terminal bases of the primer designed on the antisense strand of the strand to which the probe hybridizes. It is more preferable to select the probe sequence from a region having a sequence universal to a specific plant genus to be detected. A TaqMan™ probe is preferred as the above-described probe. A method of designing the TaqMan probe is known in the art (see e.g., Applied Biosystems, Japan, "Simple Operational Guideline for Primer Express Software, Simple Operational Guideline for TaqMan Probe Search in Primer Express Software: Rev. C" (http://www.appliedbiosystems.co.jp/website/jp/product/ctlgpage-jsp?MODELCD=19775&PL CD=17689&BUCD=131)). A probe that can be used in quantitative PCR for a given plant or a plant belonging to a given plant genus is referred herein to as a "probe for detecting" the given plant or the plant belonging to the given plant genus. That is, in the present specification, the "probe for detecting" refers to a probe that can detect a plant belonging to each plant genus by using the probe in combination with a primer set for detecting the plant genus. In this context, detection encompasses both qualitative and quantitative detection, as described above. It should be appreciated that such a probe is also advantageous in the quantitative detection.

Known examples of the fluorescent dye used in the probe include, but not limited to, FAM, HEX, TET, and FITC. Moreover, known examples of the quencher include, but not limited to, TAMRA and Dabcyl, and non-fluorescent quenchers.

The probe is preferably 13 to 30 bases in length, particularly preferably 13 to 25 bases in length. It is more preferable to use a probe having a quencher additionally labeled with MGB (Minor Groove Binden) at the 3' end so as to maintain a high Tm value even if the base length of the probe is short. Concretely, the probe for the genus *Fagopyrum* can be exemplified by oligonucleotide shown in SEQ ID NO: 64. The probe for the genus *Arachis* can preferably be exemplified by oligonucleotide that hybridizes under stringent conditions to a complementary nucleotide sequence of a nucleotide sequence shown in SEQ ID NO: 32 or 33 in the case of the combination of any of primers of SEQ ID NOs: 24 and 25 with any of primers SEQ ID NOs: 2 to 4, or otherwise, by oligonucleotide shown in SEQ ID NO: 34 in the case of the combination of any of primers of SEQ ID NOs: 21 to 23 with any of primers of SEQ ID NOs: 26 to 29, 65, and 66. In the case of the combination of any of primers of SEQ ID NOs: 30 and 31 with any of primers of SEQ ID NOs: 26 to 29, the probe is preferably oligonucleotide that hybridizes under stringent conditions to a nucleotide sequence shown in SEQ ID NO: 35 or a complementary nucleotide sequence thereof, in addition to oligonucleotide shown in SEQ ID NO: 34. It is especially preferred to use the oligonucleotide shown in SEQ ID NO: 34 as the probe together with the combination of the primer of SEQ ID NO: 21 with any of the primers of SEQ ID NOs: 26, 65, and 66.

Such a probe may be constructed using a commercially-available kit after oligonucleotide having the designed sequence is synthesized. Alternatively, the construction of the probe may be outsourced and custom-ordered, and many contract manufactures for probes are known in the art (e.g., Applied Biosystems, Japan (http//www.appliedbiosystems.co.jp)).

The quantifying method of the present invention uses a sample for correction where a sample derived from a specific plant genus to be detected (especially, to be quantified) and a standard plant sample are mixed in a predetermined ratio, and a test sample where a known amount of the standard plant sample is added to a food or a food ingredient to be examined. The method comprises extracting genomic DNA from the sample for correction and the test samples by the same approach; practicing a quantitative PCR method under the same condition; determining, as a standard value for correction, a value of the copy number of the DNA derived from the standard plant (Lo)/the copy number of the DNA derived from the specific plant genus (Fo) for the sample for correction by the quantitative PCR method; and determining a value of the copy number of the DNA derived from the specific plant genus (Fs)/the copy number of the DNA derived from the standard plant (Ls) for the test sample, and correcting the value with the standard value for correction to calculate the amount (μg) of a plant belonging to the specific plant genus contained in the food or the food ingredient (1 g) by an equation below.

$$\text{Amount of plant belonging to specific plant genus (ppm }(\mu g/g)) = Fs / Ls \times Lo / Fo \times 1{,}000{,}000$$

Thus, the method allows correction for influences such as the DNA extraction efficiency of each food or food ingredient to be examined and the inhibition of PCR reaction and even for difference in DNA content among samples to be examined. This method also allows the proper quantitative detection of a plant belonging to a specific plant genus in a DNA-free food ingredient such as salts and a food containing the ingredient.

When the assessment of whether PCR amplification products are false positive or not is required, the assessment can strictly be conducted by subjecting, to DNA sequence analysis, the PCR amplification products contained in a reaction solution after the completion of PCR.

Since it is desirable that the influences of a variety of components on DNA extraction efficiency should be as uniform as possible, the standard plant sample used in the present invention is preferably any of those being in a state similar to the state of a specific plant genus to be detected. Preferably the standard plant sample is derived from plant species unlikely to contaminate a food or a food ingredient to be examined. In light of current circumstances where the possibility of upland weeds' contaminating food crops during the cultivation process is quite difficult to eliminate and a trace amount of a weed-derived substance contaminates food crops, it is preferred to use a plant species other than plant species recognized as the upland weeds as the standard plant sample. That is, the standard plant sample should be selected from plant species unlikely to be contaminated with a plant used in the food or the food ingredient and unlikely to contaminate the food or the food ingredient.

Various upland weeds are known as the above-described upland weeds. Major examples thereof include the family *Poaceae,* the subfamily *Bambusoideae,* the family *Typhaceae,* the family *Cyperaceae,* the family *Asteraceae,* the family *Polygonaceae,* the family *Commelinaceae,* the family *Equisetaceae,* the family *Moraceae,* the family *Pontulacaceae,* the family *Caryophyllaceae,* the family *Chenopodiaceae,* the family *Leguminosae,* the family *Oxalidaceae,* the family *Euphorbiaceae,* the family *Apiaceae,* the family *Convolvulaceae,* the family *Lamiacea,* the family *Plantaginaceae,* the family *Solanaceae,* and the family *Cucurbitaceae.* For detailed information, see, for example, description in The Weed Science Society of Japan website.

For example, uniform materials that can be obtained in large amounts at a time and can be stored, such as commercially-available seeds, are more preferable as the standard plant sample.

The standard plant sample may be derived from any plant tissue (such as seeds, leaves, and rhizomes). When a sample to be detected is derived from, for example, buckwheat, wheat, and peanut seeds, the standard plant sample is preferably a seed, as with the sample to be detected. From these points of view, plant species such as watermelon, papaya, and melon, whose pulp contains a great number of seeds separated by the pulp and the rind from the outside world are preferred in the examination of a food without , for example, watermelon, papaya, and melon. Plant species that are not cultivated as food crops are also preferred even though their seeds are not separated from the outside world. Considering these conditions, examples of the standard plant sample used in the present invention include, but not particularly limited (as long as satisfying the conditions) to, those derived from *Nemophila* (the family *Hydrophyllaceae), Gloxinia* (the *family Gesneriaceae),* and statice *(Limonium)* (the family *Plumbaginaceae).* Particularly preferred is a statice seed.

It is preferred to avoid the use of, as the standard plant sample, those containing a large amount of components showing inhibitory activity in DNA extraction or PCR reaction, in view of, for example, DNA extraction efficiency and the sensitivity and/or precision of a quantitative PCR method.

By way of example, the use of a statice seed as the standard plant sample is illustrated in the Examples herein. As described above, upland weeds are highly likely to contaminate food crops and are therefore unsuitable as the standard plant sample. Consequently, the present inventors investigated the designations of families for all of the 860 types of plants described as upland weeds in The Weed Science Society of Japan website (http//wssj.ac.affrc.go.jp), and selected statice as a plant belonging to a family not included in the families. Primers specifically detecting the ITS-1 sequence of statice were used to examine general food ingredients, that is, five types of commercially-available wheat, five types of commercially-available corn grits, and three types of commercially-available mustard, for the presence or absence of contamination with the statice. However, the contamination was not detected at all in any of these food ingredients. Therefore, the statice was expected to be preferable as the standard plant sample of the present invention.

The present inventors have confirmed that the use of rice among the family *Poaceae* that contains a great number of upland weeds as the standard plant sample instead of statice is not preferred. This may be because upland weeds, plants belonging to the family *Poaceae,* contaminate cultivated ingredient plants during the cultivation.

The pulverized powder of a plant material (e.g., a statice seed) selected as the standard plant sample and the powder of a plant (e.g., buckwheat) selected as a sample derived from a specific plant genus to be detected are mixed in a predetermined ratio to prepare a sample for correction. Apart from this sample for correction, the pulverized powder of the same standard plant sample as above is added to a food or a food ingredient to be examined to prepare a test sample. In the pulverization process, it is important to make sufficient considerations so as not to cause the contamination of other food ingredients and especially the contamination of the sample derived from the specific plant genus to be detected and the standard plant sample with each other. For example, the washing of apparatuses and the like used in pulverization should completely be conducted. For preparing the sample for correction and the test sample, it is preferred that the sample derived from the specific plant genus in the sample for correction and the sample of the food or the food ingredient in the test sample should be used in almost the same amounts, and that the standard plant sample in the sample for correction and the sample derived from the standard sample in the test sample should be used in almost the same amounts.

Next, DNA is extracted from the sample for correction and the test sample. This DNA extraction can be conducted by a variety of methods known in the art and can also be performed using a commercially-available kit or pre-packed column. For example, Genomic-tip manufactured by QIAGEN may be used with reference to QIAGEN Genomic DNA Handbook and User-Developed Protocol: Isolation of genomic DNA from plants using the QIAGEN Genomic-tip.

Subsequently, the extracted DNAs are subjected to a quantitative PCR method. Although a variety of PCR techniques for quantitative analysis are known in the art, a quantitative real-time PCR method that uses a TaqMan™ probe would be convenient and advantageous.

Primers for detecting (including quantitative detection) a standard plant sample are preferably primers that bring about the specific amplification of the DNA of the standard plant sample. In addition, preferred are primers that meet the following requirements: the copy number of the DNA derived from the standard plant hardly differs from the copy number of the DNA derived from the specific plant genus in the quantitative PCR method performed for the genomic DNA extracted from the sample for correction where the sample derived from the specific plant genus to be detected and the standard plant sample are mixed in a predetermined ratio; and the difference between both of the copy numbers is within 100 times, preferably within 10 times. This is because the above-described Lo/Fo ratio is stable.

For example, when statice is used as a standard plant sample, available primers for the statice consist of the following sequences derived from a portion of the ITS-1 sequence of the statice:

5'-TTG GAC GTG TAT CCC TTG TGG TTC-3' (SEQ ID NO: 57);

and

5'-CAC GAA GGT GAA AGT TGC GTT CAT-3' (SEQ ID NO: 58).

As described above, any of those hybridizing an internal region of a site hybridized with each PCR primer for an amplification target sequence may be used as a TaqMan probe for detecting statice. For example, a probe having the following sequence derived from a portion of the ITS-1 sequence:

5'-TGT GCG ACG CGG AAT G-3' (SEQ ID NO: 59)

can be labeled with a fluorescent dye and used as the TaqMan probe.

The copy number of the DNA derived from the standard plant and the copy number of the DNA derived from the specific plant genus to be detected are calculated for the sample for correction and the test sample based on the standard curves by a quantitative real-time PCR method.

Those skilled in the art can readily practice the generation of the standard curves by a variety of methods. The standard curves can be generated by practicing a quantitative PCR method using, as a template, DNAs with a known length comprising amplification target sequences by the quantitative PCR method for the standard plant sample and the sample derived from the specific plant genus to be detected.

In addition, standard curves having higher reproducibility and fewer errors can be generated by constructing a plasmid for standard curves comprising amplification target sequences by a quantitative PCR method for the standard plant sample and the sample derived from the specific plant genus to be detected and using this plasmid as a template. DNA comprising the amplification target sequence of the sample derived from the specific plant genus to be detected and DNA comprising the amplification target sequence of the standard plant sample are inserted into one plasmid vector to construct a plasmid for standard curves. The plasmid can be amplified in *E. coli* or the like, thereby obtaining a template for standard curves.

For example, the amplification target sequences by a quantitative PCR method for the standard plant sample and the sample derived from the specific plant genus to be detected can be ligated using the method by Jayaraman K. et al. (1992. BioTechniques 12: 392-398) that uses outer and bridging primers.

The amplification target sequences of the standard plant sample and the sample derived from the specific plant genus to be detected can be incorporated into one plasmid, thereby reducing the errors of the concentrations of both sequences due to dilution. Alternatively, by using a short plasmid DNA molecule, errors due to dilution can also be reduced.

Since the template for standard curves used has a known base length, a copy number contained in a solution of the template for standard curves can be determined according to a concentration by weight and a base length. In light of this copy number, a copy number contained in the test sample is calculated.

Such a concept of the quantitative PCR method of detection of the present invention can be applied to the case in which a specific ingredient to be detected is derived from an animal such as livestock products and the case in which the specific ingredient to be detected is derived from a microorganism. When the specific ingredient to be detected is derived from an animal such as livestock products, it is preferred that an ingredient derived from an animal should be used as a standard sample. Alternatively, when the specific ingredient to be detected is derived from a microorganism, it is preferred that an ingredient derived from a microorganism should be used as a standard sample.

The present specification encompasses contents described in the specification and/or drawing of the Japanese Patent Application No. 2003-139513 to which the present application claims the priority.

<u>Brief Description of the Drawings</u>

Fig. 1A is a result of examining Shirahana buckwheat for the sensitivity of PCR. Following PCR, the resulting PCR reaction solution was subjected to 2% agarose gel electrophoresis and staining with ethidium bromide and analyzed with a fluorescent image analyzer;

Fig. 1B is a result of examining Dattan buckwheat for the sensitivity of PCR. Following PCR, the resulting PCR reaction solution was subjected to 2% agarose gel electrophoresis and staining with ethidium bromide and analyzed with a fluorescent image analyzer;

Fig. 2 is a result of examining the specificity of buckwheat PCR. Following PCR, the resulting PCR reaction solution was subjected to 2% agarose gel electrophoresis and staining with ethidium bromide and analyzed with a fluorescent image analyzer;

Fig. 3 is a result of examining the seeds of other plants for the specificity of statice PCR. Following PCR, the resulting PCR reaction solution was subjected to 2% agarose gel electrophoresis and staining with ethidium bromide and analyzed with a fluorescent image analyzer;

Fig. 4 is a result of examining a variety of food ingredients for the specificity of statice PCR. Following PCR, the resulting PCR reaction solution was subjected to 2% agarose gel electrophoresis and staining with ethidium bromide and analyzed with a fluorescent image analyzer;

Fig. 5A is a result of conducting a quantitative PCR method for buckwheat DNA. The quantitative PCR method was conducted for 500 pg of buckwheat DNA and 50 ng each of wheat, peanut, soybean, maize, mustard, pepper, and rice DNAs. However, the ingredients other than the buckwheat were not detected in the quantitative detection region. Thus, it was confirmed that only buckwheat could specifically be quantified;

Fig. 5B is a result of conducting a quantitative PCR method for buckwheat DNA. Although the quantitative PCR method was conducted for 500 pg of buckwheat DNA and 50 ng of statice DNA, it was confirmed that statice was not detected in the quantitative detection region;

Fig. 6 is a result of conducting a quantitative PCR method for buckwheat DNA. The quantitative PCR method was conducted for black bindweed DNA. Even though 50 ng of black bindweed DNA was used as a template, its amplification rate was obviously slow as compared with that of 10 copies of plasmid for standard curves used as a template and an amplification signal did not reach a threshold line. Black bindweed was not detected in the quantitative detection region. Thus, it was confirmed that only buckwheat could specifically be quantified;

Fig. 7 is a result of conducting a quantitative PCR method for buckwheat DNA by use of a plasmid for standard curves;

Fig. 8 is a graph obtained from the result shown in Fig. 7;

Fig. 9 is a result of conducting a quantitative PCR method for statice DNA. The PCR was conducted with 500 pg of statice DNA as a template. Although the quantitative PCR method was conducted for 50 ng each of wheat, peanut, soybean, maize, mustard, pepper, rice, black bindweed DNAs, they were not detected in the quantitative detection region. Thus, it was confirmed that only statice could specifically be quantified;

Fig. 10 is a result of conducting a quantitative PCR method for statice DNA by use of a plasmid for standard curves;

Fig. 11 is a graph obtained from the result shown in Fig. 10;

Fig. 12 is a result of examining a variety of food ingredients for the specificity of peanut PCR. Following PCR, the resulting PCR reaction solution was subjected to 2% agarose gel electrophoresis and staining with ethidium bromide and analyzed with a fluorescent image analyzer;

Fig. 13 is a result of conducting a quantitative PCR method for peanut DNA. The quantitative PCR method was conducted for 500 fg of peanut DNA and 50 ng each of wheat, buckwheat, soybean, maize, apple, adzuki bean, and statice DNAs. However, the ingredients other than the peanut were not detected in the quantitative detection region. Thus, it was confirmed that only a peanut could specifically be quantified;

Fig. 14 is a result of conducting a quantitative PCR method for peanut DNA by use of peanut DNA; and

Fig. 15 is a graph obtained from the result shown in Fig. 14.

## Best Mode for Carrying Out the Invention

Hereinafter, the present invention will be described more specifically with reference to the Examples.

### Example 1

#### A. Plant samples used in DNA extraction

(1) Buckwheat seed:

Shirahana buckwheat (common buckwheat; *Fagopyrum esculentum,* diploid) and Dattan buckwheat (tatary buckwheat; *Fagopyrum tataricum,* diploid) seeds from Takano were used.

(2) Wheat, peanut, soybean, maize, mustard, and statice seeds, and white pepper and rice (brown rice):

Commercially-available products were used.

(3) Wheat, soybean, maize, mustard, and black bindweed leaves:

Leaves germinated from commercially-available seeds were used.

#### B. DNA extraction

(1) DNA extraction from buckwheat seed and white pepper

DNA extraction was conducted using Genomic-tip manufactured by QIAGEN with reference to QIAGEN Genomic DNA Handbook and User-Developed Protocol: Isolation of genomic DNA from plants using the QIAGEN Genomic-tip according to procedures below.

In a 15-ml tube, 1 g of a pulverized sample was introduced, 4 ml of Carlson Lysis Buffer (0.1 M Tris-HCl (pH 9.5), 2% CTAB, 1.4 M Polyethylene Glycol #6000, and 20 mM EDTA), 8 μl of RNase A (100 mg/ml), 10 μl of 2-mercaptoethanol, and 80 μl of proteinase K (20 mg/ml) were added and mixed, followed by incubation at 74°C for 20 minutes. After being returned to room temperature, 5 ml of phenol:chloroform:isoamyl alcohol (25:24:1) was added to the resulting mixture and well mixed. An aqueous layer was then collected therefrom by centrifugation. This aqueous layer was supplemented and well mixed with the same amount of chloroform:isoamyl alcohol (24:1). An aqueous layer was then collected therefrom by centrifugation. After the same amount of chloroform:isoamyl alcohol (24:1) was again added to the aqueous layer and mixed, an aqueous layer was collected therefrom by centrifugation.

A 1/2 aliquot was taken from the obtained aqueous layer and subjected to isopropanol precipitation to collect the resulting precipitate. The precipitate was dissolved in 500 μl of Buffer QBT and applied to Genomic-tip 20/G Column equilibrated with 1 ml of Buffer QBT, to which DNA was then adsorbed. Then, the Column was washed with 5 ml of Buffer QBT and subsequently with 2 ml of Buffer QC. Finally, a precipitate collected by elution with 1.7 ml of Buffer QF and isopropanol precipitation was dissolved in 40 μl of sterilized ultrapure water. A DNA concentration in the resulting solution was measured, and the DNA solution appropriately diluted with sterilized ultrapure water was used as a template DNA sample for PCR.

(2) DNA extraction from wheat, soybean, maize, mustard, and statice seeds, and rice (brown rice)

DNA extraction was conducted using DNeasy Plant Maxi Kit manufactured by QIAGEN with reference to DNeasy Plant Maxi Kit Handbook according to procedures below.

In a 50-ml tube, 2 g of a pulverized sample was introduced, 10 ml of Buffer AP 1 and 20 μl of RNase A (100 mg/ml) were added and mixed. The resulting mixture was incubated at 65°C for 15 minutes and then centrifuged at approximately 3,000 x g for 10 minutes. A 4-ml aliquot of the resulting supernatant was collected into a 15-ml tube, to which 1.8 ml of Buffer AP2 was in turn added. The resulting mixture was left in ice for 10 minutes and centrifuged at approximately 3,000 x g for 10 minutes. The resulting supernatant was applied to QIAshredder Spin Column and centrifuged at approximately 3,000 x g for 5 minutes. A 5-ml aliquot of the resulting flow-through solution was collected into a 50-ml tube, to which 7.5 ml of Buffer AP3/E was in turn added and mixed. The resulting mixture was applied to DNeasy Spin Column and centrifuged at approximately 3,000 x g for 5 minutes to have DNA adsorbed to the Column. Then, 12 ml of Buffer AW was added to the Column and centrifuged at approximately 3,000 x g for 5 minutes, followed by the washing of the Column. Again, 12 ml of Buffer AW was added thereto and centrifuged at approximately 3,000 x g for 10 minutes, followed by the washing of the Column. Finally, 1 ml of Buffer AE preincubated at 65°C was added to the Column and left for 10 minutes. The Column was then centrifuged at approximately 3,000 x g for 5 minutes to elute DNA from the Column. A DNA concentration in the resulting solution was measured, and the DNA solution appropriately diluted with sterilized ultrapure water was used as a template DNA sample for PCR.

(3) DNA extraction from peanut seed:

DNA extraction was conducted using DNeasy Plant Maxi Kit manufactured by QIAGEN in combination with NucleoSpin Extract 2 in 1 manufactured by MACHEREY-NAGEL with reference to QIAGEN Genomic DNA Handbook and NucleoSpin Extract 2 in 1 For Direct Purification of PCR Products according to procedures below.

In a 15-ml tube, 1g of a pulverized sample was introduced, 10 ml of Buffer G2, 100 μl of proteinase K (20 mg/ml), and 10 μl of RNase A (100 mg/ml) were added and mixed, followed by incubation at 50°C for 1 hour. The resulting mixture was centrifuged at approximately 3,000 x g for 10 minutes to obtain its supernatant. The obtained supernatant was applied to Genomic-tip 20/G Column equilibrated with 1 ml of Buffer QBT, to which DNA was then adsorbed. Then, the Column was washed with 4 ml of Buffer QC. DNA was then eluted with 1 ml of Buffer QF preheated to 50°C. To the resulting eluate, 4 volumes of Buffer NT2 was added and mixed. Then, 650-μl/run of the resulting mixture solution was applied to two NucleoSpin Extract Columns and centrifuged at approximately 6,000 x g for 1 minute to have DNA adsorbed to the Columns. This was repeated until the whole amount of the mixture solution was treated. Then, 600 μl of Buffer NT3 was added to the Column and centrifuged at approximately 6,000 x g for 1 minute, followed by the washing of the Column. Again, 600 μl of Buffer NT3 was added thereto and centrifuged at the maximum speed for 1 minute to completely remove the Buffer NT3 remaining in the Column. Finally, 100 μl of Buffer NE was added to the Column and centrifuged at the maximum speed for 1 minute to elute DNA from the Column. A precipitate collected by isopropanol precipitation was dissolved in 50 μl of sterilized ultrapure water. A DNA concentration in the resulting solution was measured, and the DNA solution appropriately diluted with sterilized ultrapure water was used as a template DNA sample for PCR.

(4) DNA extraction from wheat, soybean, maize, mustard, and black bindweed leaves:

DNA extraction was conducted using DNeasy Plant Mini Kit manufactured by QIAGEN with reference to DNeasy Plant Mini Kit Handbook according to procedures below.

In a 15-ml tube, 0.5 g of a pulverized sample was introduced, 3 ml of Buffer AP 1 and 30 μl of RNase A (100 mg/ml) were added and mixed, followed by incubation at 65°C for 15 minutes. To this mixture, 975 μl of Buffer AP2 was added and left on ice for 10 minutes. The mixture was centrifuged to obtain its supernatant. The obtained supernatant was applied to QIAshredder Spin Column, which was in turn centrifuged to obtain a flow-through solution from the Column. To this flow-through solution, 0.5 volumes of Buffer AP3 and 1 volume of ethanol were added and mixed. Then, 650-μl/run of the resulting mixture solution was applied to two DNeasy Spin Columns and centrifuged at approximately 6,000 x g for 1 minute to have DNA adsorbed to the Columns. This was repeated until the whole amount of the mixture solution was treated. Then, 500 μl of Buffer AW was added to the Column and centrifuged at approximately 6,000 x g for 1 minute, followed by the washing of the Column. Again, 500 μl of Buffer AW was added thereto and centrifuged at the maximum speed for 1 minute to completely remove the Buffer AW remaining in the Column. Finally, 120 μl of Buffer AE preincubated at 65°C was added to the Column and centrifuged at approximately 6,000 x g for 1 minute to elute DNA from the Column. A DNA concentration in the resulting solution was measured, and the DNA solution appropriately diluted with sterilized ultrapure water was used as a template DNA sample for PCR.

C. PCR that detects a portion of ITS-1-5.8S rRNA gene sequence of buckwheat

(1) Primers for detecting genus *Fagopyrum:*

Sequences universal to the ITS-1-5.8S rRNA gene sequences of the following 21 sequences registered in GenBank of plants belonging to the genus *Fagopyrum* were used as primer sequences:

1: Fagopyrum urophyllum (AB000342)
2: Fagopyrum urophyllum (AB000341)
3: Fagopyrum tataricum (sub#species:potanini) (AB000340)
4: Fagopyrum tataricum (AB000339)
5: Fagopyrum statice (AB000338)
6: Fagopyrum statice (AB000337)
7: Fagopyrum pleioramosum (AB000336)
8: Fagopyrum lineare (AB000335)
9: Fagopyrum leptopodum (AB000334)
10: Fagopyrum homotropicum (AB000333)
11: Fagopyrum gracilipes (AB000332)
12: Fagopyrum esculentum ancestralis (AB000331)
13: Fagopyrum esculentum (AB000330)
14: Fagopyrum cymosum (AB000329)
15: Fagopyrum cymosum (AB000328)
16: Fagopyrum cymosum (AB000327)
17: Fagopyrum cymosum (AB000326)
18: Fagopyrum cymosum (AB000325)
19: Fagopyrum cymosum (AB000324)
20: Fagopyrum capillatum (AB000323)
21: Fagopyrum callianthum (AB000322)

Then, oligo DNA primers (manufacture by QIAGEN, OPC-purified oligonucleotides) having the following sequences were synthesized and used as primers for PCR that detect a portion of the ITS-1-5.8S rRNA gene sequence of buckwheat (hereinafter, referred to as buckwheat PCR):

5'-CGC CAA GGA CCA CGA ACA GAA G-3' (SEQ ID NO: 14);

and

5'-CGT TGC CGA GAG TCG TTC TGT TT-3' (SEQ ID NO: 15).

(2) Specificity of primers for detecting genus *Fagopyrum* (PCR simulation):

A PCR simulation software Amplify 1.0 (Bill Engels) was used to confirm whether a result of the simulation showed that a PCR amplification product was obtained with the primers for detecting buckwheat, based on 21 sequences of plants belonging to the genus *Fagopyrum,* 8 sequences of likely-to-be-allergenic plants other than buckwheat (peanut, wheat, soybean, walnut, matsutake mushroom, peach, apple, and orange), 4 sequences of plants frequently used as food ingredients (maize, rice, pepper, and mustard), and 27 sequences of related plant species of buckwheat. The related plant species of buckwheat used herein refer to plants other than the genus *Fagopyrum,* which attained Score 60 bits or more when the ITS-1 sequence portion in the nucleotide sequence (AB000330) of common buckwheat, *Fagopyrum esculentum,* registered in GenBank was subjected to BLAST homology search. This time, the sequence of a species attaining the highest score in a genus to which each of the plants belonged was selected as a representative sequence of the genus. The PCR simulation was conducted for the ITS-1-5.8S rRNA gene-ITS-2 sequence region of that sequence. The GenBank Accession Number of the sequence used in the simulation and a result of the simulation are shown in Tables 1A to 1C. Abbreviated letters and symbols in Tables 1A to 1C are as shown below:

Filled-in asterisk: those expected to yield a PCR amplification product having a size around a target size (±10 bp)
W value: Possibility of yielding a PCR amplification product
High possibility...W6>W5>W4>W3>W2...Low possibility
Numeric (bp): the size (bp) of a PCR amplification product

A value where 2 was subtracted from a value obtained in the amplification

- : those expected to yield no PCR amplification product

[Table 1A]

| Primers for detecting buckwheat (SEQ ID NOs: 14 and 15): amplification product | | | | | | | |
|---|---|---|---|---|---|---|---|
| Scientific name (Common name) | | GenBank Accession No. | W6 | W5 | W4 | W3 | W2 |
| Genus Fagopyrum | ★*Fagopyrum urophyllum* | AB000342 | 101bp | - | 439bp | - | - |
| | ★*Fagopyrum urophyllum* | AB000341 | 101bp | - | - | - | - |
| | ★*Fagopyrum tataricum* (Dattan buckwheat) | AB000340 | 101bp | - | - | - | - |
| | ★*Fagopyrum tataricum* (Dattan buckwheat) | AB000339 | 101bp | - | - | - | - |
| | ★*Fagopyrum statice* | AB000338 | 101bp | - | - | - | - |
| | ★*Fagopyrum statice* | AB000337 | 101bp | - | - | - | - |
| | ★*Fagopyrum pleioramosum* | AB000336 | 101bp | - | - | - | - |

| | | | | | | | |
|---|---|---|---|---|---|---|---|
| | ★*Fagopyrum lineare* | AB000335 | 101bp | - | - | - | - |
| | ★*Fagopyrum leptopodum* | AB000334 | 101bp | - | - | - | - |
| | ★*Fagopyrum homotropicum* | AB000333 | 101bp | - | - | - | - |
| | ★*Fagopyrum gracilipes* | AB000332 | 101bp | - | - | - | - |
| | ★*Fagopyrum esculentum* (Common buckwheat) | AB000331 | 101bp | - | - | - | - |
| | ★*Fagopyrum esculentum* (Common buckwheat) | AB000330 | 101bp | - | - | - | - |
| | ★*Fagopyrum cymosum* | AB000329 | 101bp | - | - | - | - |
| | ★*Fagopyrum cymosum* | AB000328 | 101bp | - | - | - | - |
| | ★*Fagopyrum cymosum* | AB000327 | 101bp | - | - | - | - |
| | ★*Fagopyrum cymosum* | AB000326 | 101bp | - | - | - | - |
| | ★*Fagopyrum cymosum* | AB000325 | 101bp | - | - | - | - |
| | ★*Fagopyrum cymosum* | AB000324 | 101bp | - | - | - | - |
| | ★*Fagopyrum capillatum* | AB000323 | 101bp | - | - | - | - |
| | ★*Fagopyrum callianthum* | AB000322 | 101bp | - | 440bp | - | - |

[Table 1B]

| Primers for detecting buckwheat (SEQ ID NOs: 14 and 15): amplification product | | | | | | | |
|---|---|---|---|---|---|---|---|
| Scientific name (Common name) | | GenBank Accession No. | W6 | W5 | W4 | W3 | W2 |
| Allergenic Specific ingredient | *Arachis hypogaea* (Peanut) | AF156675 | - | - | - | - | - |
| | *Triticum aestivum* (Wheat) | AJ301799 | - | - | - | - | - |
| | *Glycine max* (Soybean) | U60551 | - | - | - | - | - |
| | *Juglans regia* (Walnut) | AF303809 | - | - | - | - | - |
| | *Tricholoma matsutake* (Matsutake mushroom) | U62964 | - | - | - | - | - |
| | *Prunus persica* (Peach) | AF185621 | - | - | - | - | - |
| | *Malus x domestica* (Apple) | AF186484 | - | - | - | - | - |
| | *Citrus sp.* (Valencia orange) | E08821 | - | - | - | - | - |
| Principal food ingredient | *Zea mays* (Maize) | U46648 | - | - | - | - | - |
| | *Oryza sativa* (Rice) | AF169230 | - | - | - | - | - |
| | *Piper nigrum* (Pepper) | AF275197 | - | - | - | - | - |
| | *Sinapis alba* (Mustard) | X15915 | - | - | - | - | - |
| Related species of Polygonaceae | *Aconogonum sp. Won 152* | AF189731 | - | - | - | - | - |
| | *Fallopia scandens* | AF040069 | - | - | - | - | - |
| | *Polygonum virginianum* | U51274 | - | - | - | - | - |
| | *Rumex acetosella* | AF189730 | - | - | - | - | - |

[Table 1C]

| Primers for detecting buckwheat (SEQ ID NOs: 14 and 15): amplification product | | | | | | | |
|---|---|---|---|---|---|---|---|
| | Scientific name (Common name) | GenBank Accession No. | W6 | W5 | W4 | W3 | W2 |
| Related species other than Polygonaceae | *Talinum paraguayense* | L78056 | - | - | - | - | - |
| | *Bruinsmia styracoides* | AF396438 | - | - | - | - | - |
| | *Talinella pachypoda* | L78054 | - | - | - | - | - |
| | *Rehderodendron kwangtungense* | AF396448 | - | - | - | - | - |
| | *Pterostyrax corymbosus* | AF396445 | - | - | - | - | - |
| | *Anredera cordifolia* | L78086 | - | - | - | - | - |
| | *Cistanthe quadripetala* | L78062 | - | - | - | - | - |
| | *Xenia vulcanensis* | L78060 | - | - | - | - | - |
| | *Talinopsis frutescens* | L78058 | - | - | - | - | - |
| | *Talinaria palmeri* | L78052 | - | - | - | - | - |
| | *Portulaca sp.* | L78049 | - | - | - | - | - |
| | *Phemeranthus confertiflorus* | L78039 | - | - | - | - | - |
| | *Montiopsis umbellata* | L78033 | - | - | - | - | - |
| | *Grahamia bracteata* | L78028 | - | - | - | - | - |
| | *Herniaria glabra* | AJ310965 | - | - | - | - | - |
| | *Alluaudia dumosa* | L78011 | - | - | - | - | - |
| | *Sinojackia xylocarpa* | AF396451 | - | - | - | - | - |
| | *Halesia macgregori* | AF396442 | - | - | - | - | - |
| | *Changiostyrax dolichocarpa* | AF396439 | - | - | - | - | - |
| | *Alectryon subdentatus* | AF314765 | - | - | - | - | - |
| | *Anacampseros recurvata* | L78014 | - | - | - | - | - |
| | *Weinmannia racemosa* | AF485597 | - | - | - | - | - |
| | *Bursera tecomaca* | AF080029 | - | - | - | - | - |

As shown in Tables 1A to 1C, it was expected from the result of the simulation that a PCR amplification product having a target size of 101 bp was obtained from the 21 sequences of plants belonging to the genus *Fagopyrum.* In addition, it was expected that a PCR amplification product having the target size and a non-specific PCR amplification product were not obtained from the 8 sequences of likely-to-be-allergenic plants other than buckwheat (peanut, wheat, soybean, walnut, matsutake mushroom, peach, apple, and orange), the 4 sequences of plants frequently used as food ingredients (maize, rice, pepper, and mustard), and the 27 sequences of related plant species of buckwheat.

(3) Buckwheat PCR:

Buckwheat PCR was conducted using HotStarTaq Master Mix Kit manufactured by QIAGEN according to procedures below.

Primers of SEQ ID NOs: 14 and 15 (0.5 μM each at a final concentration) and template DNA were added to 12.5 μl of 2 × HotStartTaq Master Mix (HotStar Taq DNA Polymerase, PCR buffer with 3mM $MgCl_2$, and 400μM each dNTP), whose final volume was adjusted with sterilized ultrapure water to 25 μl to make a reaction solution, which was in turn placed in a 0.2-ml microtube and reacted using a thermal cycler GeneAmp PCR System 9600 manufactured by Applied Biosystems according to the following PCR steps: enzyme activation at 95°C for 15 minutes; 45 cycles of denaturation at 95°C for 1 minute, annealing at 66°C for 2 minutes, and extension 72°C for 1 minute; and final extension at 72°C for 4 minutes. The resulting PCR reaction solution was subjected to ethidium bromide-containing 2% agarose gel electrophoresis and analyzed with a fluorescent image analyzer FluorImager 595 manufactured by Amersham Biosciences. The results are shown in Figs. 1A, 1B, and 2. Abbreviated letters and symbols in Figs. 1A, 1B, and 2 are as shown below:

M: 100-bp DNA Ladder Marker
(-): No addition of template DNA
Numeric: Amount of template DNA added
Arrow: Target band (approximately 101 bp) of PCR amplification product
The extracted plant DNA was confirmed to have a purity level capable of PCR amplification by obtaining a PCR amplification product with primers for amplifying a portion of plant chloroplast DNA (data not shown).

(4) Sensitivity and specificity of buckwheat PCR:

As a result of buckwheat PCR, a PCR amplification product having a size of approximately 101 bp expected from the target ITS-1-5.8S rRNA gene sequence of buckwheat was obtained from 500 to 50 fg of Shirahana buckwheat (common buckwheat) and Dattan buckwheat DNAs, as shown in Figs. 1A and 1B. Sensitivity that allows the detection of 500 to 50 fg of buckwheat DNA corresponds to a sensitivity level at which, when PCR is conducted with 50 ng of DNA extracted from a certain sample as a template, 10 to 1 ppm of buckwheat DNA contained in the sample DNA can be detected.

As a result of buckwheat PCR, a PCR amplification product having the target size and a non-specific PCR amplification product were not obtained from 50 ng each of the DNAs of the wheat leaf, peanut seed, soybean leaf, maize leaf, mustard leaf, and white pepper, and rice, as shown in Fig. 2. Similarly, it was also confirmed that a PCR amplification product was not obtained from salmon sperm DNA (data not shown). As shown in Fig. 2, although a PCR amplification product having the target size but a faint band was obtained from 50 to 5 ng of the DNA of the leaf of black bindweed that was one of related species of buckwheat, a PCR amplification product having the target size and a non-specific PCR amplification product were not obtained from 500 pg or less thereof. Specificity that does not detect 500 pg or less of black bindweed DNA as a false positive corresponds to a specificity level at which, when PCR is conducted with 50 ng of DNA extracted from a certain sample as a template, 1% or less black bindweed DNA, if any, in the sample DNA is not detected as a false positive. Moreover, there is the possibility that a change in PCR conditions results in no amplification product having the target size even from 50 to 5 ng of black bindweed DNA.

(5) Nucleotide sequence analysis of buckwheat PCR amplification product:

The nucleotide sequence of the Shirahana buckwheat DNA-derived PCR amplification product thus obtained was analyzed by double-strand direct sequencing using primers of SEQ ID NOs: 14 and 15. The obtained nucleotide sequence was compared with the nucleotide sequence (AB000330) of common buckwheat, *Fagopyrum esculentum,* registered in GenBank to confirm that the nucleotide sequence of the Shirahana buckwheat DNA-derived PCR amplification product matched 100% to the target site of the nucleotide sequence (AB000330) of common buckwheat *(Fagopyrum esculentum)* registered in GenBank. This demonstrated that PCR using the primers amplified and detected a portion of the ITS-1-5.8S rRNA gene sequence of buckwheat.

These results showed that buckwheat PCR using the primers could detect, with high sensitivity and specificity, the ITS-1-5.8S rRNA gene sequences of the general plants belonging to the genus *Fagopyrum.* We decided to use the present primers in PCR that quantified the copy number of the ITS-1-5.8S rRNA gene sequence of buckwheat (hereinafter, referred to as a quantitative PCR method for a buckwheat sequence).

<u>D. PCR that detects a portion of ITS-1 sequence of statice (for correction)</u>

Next, the detection of, by PCR, a standard plant sample used in correction was investigated.

In the present Example, statice, a spermatophyte not described in an upland weed list by The Weed Science Society of Japan, whose seed was easily available was used as the standard plant sample.

(1) Primers for detecting statice:

Based on the DNA sequence (AJ222860) of statice registered in GenBank, primers having the following sequences for PCR that detected a portion of the ITS-1 sequence of statice (hereinafter, referred to as statice PCR) were designed to synthesize oligo DNA primers (manufactured by QIAGEN, OPC-purified oligonucleotides):

5'-TTG GAC GTG TAT CCC TTG TGG TTC-3' (SEQ ID NO: 57);

and

5'-CAC GAA GGT GAA AGT TGC GTT CAT-3' (SEQ ID NO: 58).

(2) Statice PCR:

Statice PCR was conducted basically in the same way as the above Example 1.C.(3) except that the above-described primers were used at a final concentration of 0.2 μM each. The results are shown in Figs. 3 and 4.

The extracted plant DNA was confirmed to have a purity level capable of PCR amplification by obtaining a PCR amplification product with primers for amplifying a portion of plant chloroplast DNA (data not shown).

(3) Specificity of statice PCR:

As a result of statice PCR, a PCR amplification product having a size of approximately 101 bp expected from the target ITS-1 sequence of statice was obtained from 50 ng of the DNA of the statice seed, as shown in Fig. 3. In addition, a PCR amplification product having a target size and a non-specific PCR amplification product were not obtained from 50 ng each of the DNAs of the Shirahana buckwheat seed, Dattan buckwheat seed, wheat seed, peanut seed, soybean seed, maize seed, mustard seed, white pepper, rice, and black bindweed leaf, as shown in Fig. 3. Similarly, it was also confirmed that a PCR amplification product was not obtained from salmon sperm DNA (data not shown).

Thus, the primers for detecting statice DNA are presumed to have specificity to statice DNA.

(4) Evaluation of food ingredients for presence or absence of contamination with statice:

Next, confirmation of whether statice was suitable as the standard plant sample was conducted. Namely, statice PCR was conducted to confirm that statice did not contaminate a food or a food ingredient.

As a result of statice PCR, a PCR amplification product having a target size and a non-specific PCR amplification product were not obtained from 50 ng each of the DNAs of the seeds of 5 types of wheat, 5 types of corn grits, and 3 types of mustard, as shown in Fig. 4.

(5) Evaluation of statice for presence or absence of contamination with buckwheat:

A quantitative PCR method for a buckwheat sequence established as described below was conducted to confirm whether or not buckwheat contaminated the sample of the statice seed. As a result of the quantitative PCR method for the buckwheat sequence, it was confirmed that the fluorescent signal indicating amplification was not found from the DNA of the statice seed, and that contamination was not observed (data not shown).

(6) Nucleotide sequence analysis of statice PCR amplification product:

The nucleotide sequence of the statice DNA-derived PCR amplification product thus obtained was analyzed by double-strand direct sequencing using primers of SEQ ID NOs: 57 and 58. The obtained nucleotide sequence was compared with the nucleotide sequence (AJ222860) of statice, *Limonium simiatum,* registered in GenBank to confirm that the nucleotide sequence of the statice DNA-derived PCR amplification product matched 100% to the target site of the nucleotide sequence (AJ222860) of statice (*Limonium sinuatum*) registered in GenBank. It could be confirmed that the statice PCR amplified and detected a portion of the target ITS-1 sequence of statice.

These results suggested that mutual contamination did not take place between statice and food ingredients, and that the statice was suitable as the standard plant sample for correction. We thus decided to use the primers of SEQ ID NOs: 57 and 58 in PCR that quantified the copy number of the ITS-1 sequence of statice (hereinafter, referred to as a quantitative PCR method for a statice sequence).

<u>E. Construction of plasmid for standard curves used in quantitative analysis</u>

(1) Ligation PCR for target DNA sequences of buckwheat PCR and statice PCR and nucleotide sequence analysis of amplification product from ligation PCR:

The target amplification product of buckwheat and the target amplification product of statice were ligated by a PCR method and introduced into a TA cloning vector. The TA cloning vector was introduced into *E. coli* and amplified, thereby constructing a plasmid for standard curves for quantitatively analyzing the copy numbers of buckwheat and statice.

At first, oligo DNA primers (manufactured by QIAGEN, OPC-purified oligonucleotides) having sequences below were synthesized and used as primers. These primers contain the primer sites for buckwheat and statice used in the above-described buckwheat PCR and statice PCR.

5'-TCT AGA CGC CAA GGA CCA CGA ACA GAA G-3' (SEQ ID NO: 60)

5'-CAA AAG CTT CGT TGC CGA GAG TCG TTC TGT TT-3' (SEQ ID NO: 61)

5'-ACG AAG CTT TTG GAC GTG TAT CCC TTG TGG TTC-3' (SEQ ID NO: 62)

5'-GGA TCC CAC GAA GGT GAA AGT TGC GTT CAT-3' (SEQ ID NO: 63).

A ligation plasmid was constructed using HotStarTaq Master Mix Kit manufactured by QIAGEN with reference to the method by Jayaraman K. et al. (1992. A PCR-Mediated Gene Synthesis Strategy Involving the Assembly of Oligonucleotides Representing Only One of the Strands, BioTechniques 12: 392-398) according to procedures below.

To 25 μl of 2 × HotStartTaq Master Mix (HotStar Taq DNA Polymerase, PCR buffer containing 3mM $MgCl_2$, and 400 μM each dNTP), dNTP (500 μM at a final concentration) was added, primers of SEQ ID NOs: 60 and 63 (1.0 μM each at a final concentration) as outer primers, primers of SEQ ID NOs: 61 and 62 (25 nM each at a final concentration) as bridging primers were added. As template DNAs, the PCR amplification product with the target DNA sequence of buckwheat PCR obtained in Example 1.C.(4) and the PCR amplification product with the target DNA sequence of statice PCR obtained in Example 1.D.(3) were added. The final volume was adjusted with sterilized ultrapure water to 50 μl to make a reaction solution, which was in turn placed in a 0.2-ml microtube and reacted using a thermal cycler PTC-200 DNA Engine manufactured by MJ Research according to the following PCR steps: enzyme activation at 95°C for 15 minutes; 15 cycles of denaturation at 95°C for 1 minute, annealing at 40°C for 1 minute, and extension 72°C for 1 minute; and 30 cycles of denaturation at 95°C for 1 minute, annealing at 66°C for 1 minute, and extension 72°C for 1 minute. The resulting PCR reaction solution was subjected to ethidium bromide-containing 2% agarose gel electrophoresis and analyzed with a fluorescent image analyzer FluorImager 595 manufactured by Amersham Biosciences. The nucleotide sequence of the resulting PCR amplification product was analyzed by double-strand direct sequencing using primers of SEQ ID NOs: 60 and 63.

As a result of ligation PCR, a PCR amplification product having an expected size of approximately 200 bp was obtained (data not shown). As a result of nucleotide sequence analysis, it was confirmed that this PCR amplification product contained the target DNA sequences of buckwheat PCR and statice PCR (data not shown).

(2) Insertion of ligation PCR amplification product into plasmid and nucleotide sequence analysis of inserted DNA fragment:

Using pGEM-T Easy Vector System (manufactured by Promega), the PCR amplification product thus obtained was TA-cloned into pGEM-T Easy Vector, with which *E. coli* (*E. coli* JM109 (DH5α)) was then transformed. A transformant, having the approximately 220-bp inserted fragment that could be confirmed to contain the target DNA sequences of buckwheat PCR and statice PCR by colony PCR and nucleotide sequence analysis, was subjected to liquid culture in a LB medium. QIAGEN Hi Speed Plasmid Midi Kit manufactured by QIAGEN was used to extract and purify the plasmid from the resulting culture. The nucleotide sequence of the DNA fragment inserted into the purified plasmid was analyzed by double-strand sequencing using primers for the sequence on the plasmid. As a result, it was confirmed that the nucleotide sequence of the DNA fragment inserted into the plasmid of the transformant contained the target DNA sequences of buckwheat PCR and statice PCR, as intended (data not shown).

(3) Preparation of dilution series of plasmid for standard curves:

The number (copy number) of the plasmid molecules was calculated based on the plasmid length and the absorbance (Abs. 260 nm) of the above-described plasmid extracted and purified. The plasmid was diluted with 5 ng/μl salmon sperm DNA (manufactured by Wako Pure Chemical Industries, fibrous sodium deoxyribonucleate from salmon testis dissolved in sterilized ultrapure water) to prepare a dilution series of the plasmid for standard curves at $10^9$ to $10^1$ copies/2.5 μl. We decided to use this dilution series in the generation of standard curves for the quantitative PCR methods for buckwheat and statice sequences.

F. PCR that quantifies copy number of buckwheat sequence

(1) TaqMan MGB probe for detecting buckwheat sequence:

A TaqMan MGB probe (manufactured by Applied Biosystems Japan, reporter dye FAM) having a sequence below was synthesized and used as a probe for detecting a buckwheat sequence. A sequence universal to 21 sequences registered in GenBank as the ITS-1-5.8S rRNA gene sequences of plants belonging to the genus *Fagopyrum* was employed as the probe sequence.

5'-CGG GAC GCG CTT C-3' (SEQ ID NO: 64)

(2) Quantitative PCR method for buckwheat sequence:

A Quantitative PCR method for a buckwheat sequence was conducted using QuantiTect Probe PCR Kit manufactured by QIAGEN according to procedures below.

Primers of SEQ ID NOs: 14 and 15 (0.2 μM each at a final concentration), the TaqMan MGB probe of SEQ ID NO: 64 (0.2 μM at a final concentration), and template DNA were added to 12.5 μl of 2 × QuantiTect Probe PCR Master Mix. The final volume was adjusted with sterilized ultrapure water to 25 μl to make a solution, which was in turn dispensed into a 96-well PCR plate. For standard curves, a solution supplemented with the dilution series of the plasmid DNA for standard curves instead of the template DNA was dispensed. The 96-well PCR plate into which each of the solutions was dispensed was loaded in a real-time PCR device Sequence Detection System 7700 manufactured by Applied Biosystems, in which the solution was reacted according to the following PCR steps: at 50°C for 2 minutes; 95°C for 15 minutes; and 45 cycles of denaturation at 95°C for 1 minute, annealing at 66°C for 2 minutes, and extension at 72°C for 1 minute. Every reaction was conducted with the same samples in duplicate (in 2 wells). After the completion of reaction, fluorescence data taken during the extension step was analyzed. A baseline was first set to cycles 0 to 1 and then appropriately set to within a range before a cycle where the increase of fluorescence was confirmed to begin. A threshold line was set according to the method described in Kuribara H et al., 2002, Novel Reference Molecules for Quantitation of Genetically Modified Maize and Soybean, Journal of AOAC International 85: 1077-1089. The results are shown in Figs. 5A, 5B, 6, 7, and 8.

The extracted plant DNA was confirmed to have a purity level capable of PCR amplification by success of obtaining a PCR amplification product with primers for amplifying a portion of plant chloroplast DNA (data not shown).

(3) Specificity of quantitative PCR method for buckwheat sequence:

As a result of the quantitative PCR method for the buckwheat sequence, a fluorescent signal indicating amplification was found from the DNA from the Shirahana buckwheat seed, as shown in Figs. 5A and 5B. On the other hand, a fluorescent signal indicating amplification was not observed in 50 ng each of the DNAs from the wheat leaf, peanut seed, soybean leaf, maize leaf, mustard leaf, white mustard, rice, and statice seed. Similarly, a fluorescent signal indicating amplification was not observed in salmon sperm DNA (data not shown). Although a weak amplification signal was observed in 50 ng of the DNA of the black bindweed leaf as shown in Fig. 6, which occurred at a threshold cycle (Ct value) later than that of 10 copies for the standard curve and did not reach the threshold line.

This specificity corresponds to a specificity level at which, when PCR is conducted with 50 ng of DNA extracted from a certain sample supplemented with statice as a template, the sample is not quantified as a false positive even if the sample was black bindweed (related species of buckwheat), one species of weeds that are 100% inedible.

(4) Quantitative property and sensitivity of quantitative PCR method for buckwheat sequence:

As a result of the quantitative PCR method for the buckwheat sequence, a quantitative property and sensitivity where a standard curve having a correlation coefficient of 0.999 and a slope of -3.504 could be drawn with $10^8$ to $10^1$ copies of the plasmid for standard curves could be confirmed, as shown in Figs. 7 and 8. Sensitivity that attained a fluorescent signal indicating amplification could also be found from 50 fg of the Shirahana buckwheat DNA. In addition, when the Ct value of 5 ng to 50 fg of the Shirahana buckwheat DNA was plotted, a quantitative property that could draw a correlated linear curve could also be confirmed in this range (data not shown).

These results demonstrated that the quantitative PCR method for the buckwheat sequence using the primers of SEQ ID NOs: 14 and 15 together with the probe of SEQ ID NO: 64 could detect, with high sensitivity and specificity, the ITS-1-5.8S rRNA gene sequences of the general plants belonging to the genus *Fagopyrum* and quantify their copy numbers. We decided to use the present quantitative PCR method for the buckwheat sequence in combination with a quantitative PCR method for a statice sequence for correction shown below in the measurement of the amount of contaminating buckwheat.

G. PCR that quantifies copy number of statice sequence

(1) TaqMan MGB probe for detecting statice sequence:

A TaqMan MGB probe (manufactured by Applied Biosystems Japan, reporter dye FAM) having a sequence below was synthesized and used as a probe for detecting a statice sequence.

5'-TGT GCG ACG CGG AAT G-3' (SEQ ID NO: 59)

(2) Quantitative PCR method for statice sequence and analysis:

A quantitative PCR method for a statice sequence was conducted basically in the same way as Example 1.F. (2) except that primers of SEQ ID NOs: 57 and 58 were used at a final concentration of 0.2 μM each and the TaqMan MGB probe of SEQ ID NO: 59 was used at a final concentration of 0.2 μM. The results are shown in Figs. 9, 10, and 11.

(3) Specificity of quantitative PCR method for statice sequence:

As a result of the quantitative PCR method for the statice sequence, a fluorescent signal indicating amplification was found from the DNA from the statice seed, as shown in Fig. 9. On the other hand, a fluorescent signal indicating amplification was not observed in 50 ng each of the DNAs from the Shirahana buckwheat seed, Dattan buckwheat seed, wheat seed, peanut seed, soybean seed, maize seed, mustard seed, white mustard, rice, and black bindweed leaf. Similarly, a fluorescent signal indicating amplification was not observed in salmon sperm DNA (data not shown).

(4) Quantitative property of quantitative PCR method for statice sequence:

As a result of the quantitative PCR method for the statice sequence, a quantitative property that could draw a standard curve having a correlation coefficient of 0.999 and a slope of -3.386 with $10^8$ to $10^1$ copies of the plasmid for standard curves could be confirmed, as shown in Figs. 10 and 11.

These results demonstrated that the quantitative PCR method for the statice sequence using the primers of SEQ ID NOs: 57 and 58 together with the probe of SEQ ID NO: 59 could specifically detect the ITS-1 sequence of statice and quantify its copy number. We decided to use the present quantitative PCR method for the statice sequence for correction in combination with the quantitative PCR method for the buckwheat sequence shown in Example 1.F. in the measurement of the amount of contaminating buckwheat.

Example 2

A. Statice used as standard, a variety of buckwheat flour samples, and buckwheat, rice, and wheat used in preparation of artificially contaminated sample

(1) Statice:

Excellent Light Blue for a cut flower (single lot) sold by Sakata Seed Corporation was used.

(2) Buckwheat:

The buckwheat flour of Shirahana buckwheat (common buckwheat; *Fagopyrum esculentum,* diploid), the buckwheat flour of Dattan buckwheat (*F. tataricum,* diploid), the buckwheat flour of Takane Ruby (*F. esculentum,* diploid), and the buckwheat flour of Great Ruby (*F. esculentum,* tetraploid) sold by Takano Co., Ltd. were used. Shirahana buckwheat flour was used in the preparation of an artificially contaminated sample.

(3) Wheat:

Commercially-available Norin 61 was used.

(4) Rice:

Commercially-available chemical-free Akita Komachi brown rice was used.

B. Pulverization and DNA extraction of statice used as standard and rice and wheat used in preparation of artificially contaminated sample

(1) Pulverization:

Pulverization was performed with Ultra Centrifugal Mill ZM1 (manufactured by Retsch) equipped with a rotor (made of stainless steel, 24-edged) and a screen (made of stainless steel, 0.20 mm).

(2) Washing of mill

The parts of the mill such as a sample holder, a sample lid, a rotor, a screen, fasteners, and a jig were washed with water, immersed in 10% bleaching solution, washed with water, and dried, before and after use for the pulverization of the sample. The main body of the mill was washed with an air gun and wiped, and then used.

(3) Confirmation of absence of contamination of mill with buckwheat and statice:

Before the pulverization of the sample in large amounts, a portion thereof or commercially-available freeze-dried maize with cornhusk not contaminated with buckwheat and statice was pulverized. DNA was then extracted therefrom to confirm the present or absence of a fluorescent signal indicating amplification from 50 ng of the template DNA by the quantitative PCR methods for the buckwheat sequence and the statice sequence shown in Example 1.F and Example 1.G. When no fluorescent signal was observed, the mill was assessed as being not contaminated, and the work proceeded to do the pulverization of the sample in large amounts illustrated below. When a fluorescent signal was observed, the mill was assessed as being contaminated. In this case, the mill was washed again, and brown rice (1 kg) already confirmed to have no contamination with buckwheat and statice was pulverized in this mill. After the washing of the mill and the replacement of its screen with a new one, the commercially-available freeze-dried maize with cornhusk not contaminated with buckwheat and statice was pulverized again, and the presence or absence of a fluorescent signal was confirmed in the same way as above. After the mill could be assessed as being not contaminated with buckwheat and statice, the work proceeded to do the pulverization of the sample in large amounts illustrated below.

(4) Pulverization of statice in large amounts and confirmation of absence of contamination of pulverized powder with buckwheat:

In the mill that was confirmed to have no contamination with buckwheat, approximately 1 kg of statice was pulverized. Ten 2-g aliquots were sampled from the pulverized powder, and DNA was extracted therefrom with DNeasy Plant Maxi Kit by the method described in Example 1.B.(2) to confirm the absence of a fluorescent signal indicating amplification from 50 ng of the template DNA by the quantitative PCR method for the buckwheat sequence (data not shown). The powder of statice not contaminated with buckwheat was secured by these procedures.

(5) Pulverization of rice and wheat in large amounts and confirmation of absence of contamination of pulverized powders with buckwheat and statice:

In the mill that was confirmed to have no contamination with buckwheat and statice, approximately 500 g of rice was pulverized. Five 2-g aliquots were sampled from the pulverized powder, and DNA was extracted therefrom with DNeasy Plant Maxi Kit by the method described in Example 1.B.(2) to confirm the absence of a fluorescent signal indicating amplification from 50 ng of the template DNA by the quantitative PCR methods for the buckwheat sequence and the statice sequence (data not shown). The same procedures were conducted for wheat. The pulverized powders of rice and wheat not contaminated with buckwheat and statice were obtained by these procedures.

C. Preparation of artificially contaminated sample

(1) Artificially contaminated sample of rice pulverized powder containing buckwheat flour:

Six anti-static OP bags (manufactured by Fukusuke Kogyo, PZ type No. 6 (special anti-statice treatment) reclosable with a zipper and three sides sealed), in which 45.00 g of the rice pulverized powder was weighed and placed, were prepared and numbered 1 through 6. In the bag No. 1, 5.00 g of buckwheat flour was weighed and placed. The contents of the bag were manually mixed for 15 minutes with the top of the bag closed, to obtain the rice pulverized powder containing 10% buckwheat flour. Subsequently, 5.00 g of this powder of rice containing 10% (100, 000 ppm) buckwheat flour was weighed and placed in the bag No.2. The contents of the bag were manually mixed for 15 minutes with its mouth closed, to obtain the powder of rice containing 1% (10,000 ppm) buckwheat flour. These dilution and mixing procedures were repeated to prepare the rice pulverized powders containing 100,000 to 1 ppm of buckwheat flour.

(2) Artificially contaminated sample of wheat pulverized powder containing buckwheat flour:

The wheat pulverized powders containing 100,000 to 1 ppm of buckwheat flour were prepared in the same way as above.

(3) Artificially contaminated sample of pulverized powder of rice and wheat containing buckwheat flour:

In an anti-static OP bag (manufactured by Fukusuke Kogyo, PZ type No. 5 (special anti-statice treatment) reclosable with a zipper and three sides sealed), 12.5 g of the rice pulverized powder containing 10 ppm of buckwheat flour and 12.5 g of the wheat pulverized powder containing 10 ppm of buckwheat flour were weighed and placed. The contents of the bag were manually mixed for 15 minutes with top of the bag closed, to obtain the pulverized powder of rice and wheat containing 10 ppm of buckwheat flour

D. Determination of artificially contaminated sample-sampling scale upon DNA extraction

(1) Particle size distribution measurement of Shirahana buckwheat flour:

For determining the particle size of buckwheat flour with the assumption that the buckwheat flour was a globular, the particle size distribution measurement (laser diffraction/scattering method, dry process, under the condition of a pressure of 0.5 $kg/cm^2$) of Shirahana buckwheat flour was conducted. The measurement was outsourced to Seishin Enterprise Co., Ltd., Powder Technology Centre. As a result, the particle size of the Shirahana buckwheat flour in terms of a particle size (median size) (x50) was 80.941 μm.

(2) Bulk density measurement of Shirahana buckwheat flour:

For determining the density (density including inter- and intra-particle voids and pores) of buckwheat flour, the bulk density measurement (Mercury (Hg) method: a method where buckwheat flour is placed in a cell having a fixed volume, which is then filled with mercury) of Shirahana buckwheat flour was conducted. The measurement was outsourced to Seishin Enterprise Co., Ltd., Powder Technology Centre. As a result, the bulk density of the Shirahana buckwheat flour (by the Hg method) was 1.181 $g/cm^3$.

Volume occupied by buckwheat flour = (volume of cell) - (volume of mercury added)

Bulk density of buckwheat flour (Hg method) =

(volume of buckwheat flour added) / (volume occupied by buckwheat flour)

(3) Trial calculation of particle number of buckwheat flour in artificially contaminated sample and determination of sampling scale:

Weight per particle of buckwheat flour was calculated from the measured values (the particle size of 80.941 μm and the density of 1.181 $g/cm^3$) of the Shirahana buckwheat flour to make a trial calculation of the particle number of the buckwheat flour in the artificially contaminated samples of varying buckwheat flour concentrations. The results are shown in Table 2. This result revealed that, when a sample for DNA extraction was sampled from the artificially contaminated sample containing 10 ppm of contaminating buckwheat of interest in quantification, 4 g or more of the sample for DNA extraction was required for placing at least approximately 100 particles of buckwheat flour in the sample that had been sampled. We decided to sample a 5-g aliquot for DNA extraction.

[Table 2] Particle number of Shirahana buckwheat flour in artificially contaminated sample

| Concentration of Shirahana buckwheat flour in artificially contaminated sample (ppm:μg/g) | Particle number of Shirahana buckwheat flour in sampling of Ng of artificially contaminated sample (calculated at one particle of buckwheat flour = 0.3277 μg) | | |
|---|---|---|---|
| N(g) = | 2 | 4 | 5 |
| 1,000,000 ppm | 3,051,167 | 12,204,669 | 15,255,836 |
| 100,000 ppm | 305,117 | 1,220,467 | 1,525,584 |
| 10,000 ppm | 30,512 | 122,047 | 152,558 |
| 1,000 ppm | 3,051 | 12,205 | 15,256 |
| 100 ppm | 305 | 1,220 | 1,526 |
| 10 ppm | 31 | 122 | 153 |
| 1 ppm | 3 | 12 | 15 |
| 100 particles or more | | | |

E. DNA extraction from 100% buckwheat flour + statice standard, and artificially contaminated sample + statice standard

(1) Variety of buckwheat flour samples:

Six samples were sampled from Shirahana buckwheat flour and three samples were sampled from each of Takane Ruby buckwheat flour, Great Ruby buckwheat flour, and Dattan buckwheat flour. These samples were used in DNA extraction.

(2) Artificially contaminated sample:

Three samples were sampled from each of the wheat pulverized powder containing 100 ppm of Shirahana buckwheat flour, the wheat pulverized powder containing 10 ppm of Shirahana buckwheat flour, the rice pulverized powder containing 10 ppm of Shirahana buckwheat flour, and the pulverized powder of wheat and rice containing 10 ppm of Shirahana buckwheat flour, and used in DNA extraction.

(3) DNA extraction:

DNA extraction was conducted using Genomic-tip manufactured by QIAGEN with reference to QIAGEN Genomic DNA Handbook and User-Developed Protocol: Isolation of genomic DNA from plants using the QIAGEN Genomic-tip according to procedures below.

In a 50-ml tube, 5 g of the sample and 1 g of the statice pulverized powder were placed and to which 30 ml of Carlson Lysis Buffer (0.1 M Tris-HCl (pH 9.5), 2% CTAB, 1.4 M Polyethylene Glycol #6000, and 20 mM EDTA), 60 μl of RNase A (100 mg/ml), 75 μl of 2-mercaptoethanol, and 600 μl of proteinase K (20 mg/ml) were added. For further enhancing the dispersibility of the sample, three zirconia balls (manufactured by Nikkato, YTZ ball, ϕ7 mm) were added to the mixture and mixed for 10 minutes or more with a shaker (manufactured by Iwaki Sangyo, KM Shaker V-DX) at Speed 100 until lumps were eliminated, followed by incubation at 74°C for 20 minutes. During the incubation, the tube was manually shaken and mixed every five minutes.

Following centrifugation at 3,000 x g for 10 minutes, 4 ml of the resulting supernatant was collected into a 15-ml tube and 5 ml of phenol:chloroform:isoamyl alcohol (25:24:1) was added and well mixed. After this mixture was centrifuged at 3,000 x g for 10 minutes, the resulting supernatant (aqueous layer) was collected into a 15-ml tube and 3.5 ml of chloroform:isoamyl alcohol (24:1) was added and well mixed. After this mixture was centrifuged at 3,000 x g for 10 minutes, the resulting supernatant (aqueous layer) was collected into a 15-ml tube and subjected again to extraction with chloroform:isoamyl alcohol (24:1) and centrifugation to collect a supernatant (aqueous layer). A precipitate collected from a 150-μl aliquot of the supernatant (aqueous layer) by isopropanol precipitation was dissolved in 100 μl of sterilized

ultrapure water and 900 μl of Buffer QBT was added. The resulting solution was applied to Genomic-tip 20/G Column equilibrated with 1 ml of Buffer QBT, to which DNA was then adsorbed. Then, the Column was washed with 4 ml of Buffer QC. Finally, a precipitate collected by DNA elution with 1 ml of Buffer QF and isopropanol precipitation was dissolved in 40 μl of sterilized ultrapure water. A DNA concentration in the resulting solution was measured, and the DNA solution appropriately diluted with sterilized ultrapure water was used as a template DNA sample for PCR.

F. Calculation of "copy number of statice sequence/copy number of buckwheat sequence ratio" in DNA extracted from 100% buckwheat flour supplemented with statice standard

The quantitative PCR methods for the buckwheat sequence and the statice sequence were conducted by the method described in Example 1.F. and Example 1.G. Based on the standard curves, the copy number of the buckwheat sequence and the copy number of the statice sequence of 50 ng of DNA extracted from 100% buckwheat flour supplemented with the statice standard were quantified. Based on the quantitative values, "the copy number of the statice sequence/the copy number of the buckwheat sequence=Lo/Fo ratio" was calculated. The Lo/Fo ratio of each buckwheat flour sample was calculated by simultaneously measuring the same samples in 2 wells and obtaining the average of ratios from two measurements.

As a result of Lo/Fo ratio measurement, the Lo/Fo ratio was 2.36 for the Shirahana buckwheat flour (6 extracted samples each measured in duplicate in two wells), 3.25 for the Takane Ruby buckwheat flour, 2.70 for the Great Ruby buckwheat flour, and 4.75 for the Dattan buckwheat flour (3 extracted samples each measured in duplicate in two wells), as shown in Table 3. We decided that the amount of contaminating buckwheat was determined using the Lo/Fo ratio of the Shirahana buckwheat flour obtained here and "the copy number of the buckwheat sequence/the copy number of the statice sequence =Fs/Ls ratio" of the artificially contaminated sample calculated in Example 2.G. The raw data of a variety of buckwheat flour samples in Lo/Fo ratio measurement is shown in Tables 4A and 4B.

[Table 3] Lo/Fo ratios of variety of buckwheat flour samples

| Sample name | | **Lo/Fo** 1st measurement Measured value 1 | Average | **Lo/Fo** 2st measurement Measured value 1 | Average | **Lo/Fo** Average from two measurements |
|---|---|---|---|---|---|---|
| Shirahana buckwheat flour 100% | **No. 1** | **2.23** | **2.37** | | **2.36** | **2.36** |
| | **No. 2** | **2.38** | | **2.44** | | |
| | **No. 3** | **2.12** | | **2.11** | | |
| | **No. 4** | **2.84** | | **2.70** | | |
| | **No. 5** | **2.12** | | **2.11** | | |
| | **No. 6** | **2.50** | | **2.56** | | |
| Dattan buckwheat flour 100% | **No. 1** | **4.33** | **4.82** | **4.06** | **4.69** | **4.75** |
| | **No. 2** | **5.42** | | **5.27** | | |
| | **No. 3** | **4.70** | | **4.72** | | |
| Takane Ruby buckwheat flour 100% | **No. 1** | **3.40** | **3.20** | **3.66** | **3.30** | **3.25** |
| | **No. 2** | **2.58** | | **2.40** | | |
| | **No. 3** | **3.61** | | **3.85** | | |

Table continued

| Sample name | | Lo/Fo 1st measurement Measured value 1 | Average | Lo/Fo 2st measurement Measured value 1 | Average | Lo/Fo Average from two measurements |
|---|---|---|---|---|---|---|
| Great Ruby buckwheat flour 100% | **No. 1** | **2.39** | **2.67** | **2.38** | **2.72** | **2.70** |
| | **No. 2** | **2.92** | | **2.92** | | |
| | **No. 3** | **2.72** | | **2.87** | | |

[Table 4A]

Raw data of variety of buckwheat flour samples in Lo/Fo ratio measurement (first measurement)

**Raw data of variety of buckwheat flour samples in first measurement**

***Fagopyrum:***

quantitative PCR for copy number of buckwheat sequence

Sample information (buckwheat flour)

| Sample | | Ct | Copy number | Average copy number (Fo copy) |
|---|---|---|---|---|
| Shirahana buckwheat flour 100% | No. 1 | 14.4 | 2.70E+07 | **26,018,826** |
| | | 14.6 | 2.50E+07 | |
| | No. 2 | 14.8 | 2.10E+07 | **20,441,034** |
| | | 14.9 | 2.00E+07 | |
| | No. 3 | 14.3 | 3.00E+07 | **29,482,716** |
| | | 14.4 | 2.90E+07 | |
| | No. 4 | 14.7 | 2.30E+07 | **22,277,192** |
| | | 14.8 | 2.20E+07 | |
| | No. 5 | 14.3 | 3.00E+07 | **29,360,360** |
| | | 14.4 | 2.80E+07 | |
| | No. 6 | 14.6 | 2.50E+07 | **24,691,600** |
| | | 14.6 | 2.40E+07 | |
| Dattan buckwheat flour 100% | No. 1 | 15.2 | 1.60E+07 | **14,956,499** |
| | | 15.4 | 1.40E+07 | |
| | No. 2 | 15.6 | 1.30E+07 | **12,823,798** |
| | | 15.6 | 1.30E+07 | |
| | No. 3 | 15.3 | 1.60E+07 | **14,854,976** |
| | | 15.4 | 1.40E+07 | |
| Takane Ruby buckwheat flour 100% | No. 1 | 15.1 | 1.70E+07 | **17,177,656** |
| | | 15.2 | 1.70E+07 | |
| | No. 2 | 14.4 | 2.80E+07 | **26,409,548** |
| | | 14.6 | 2.50E+07 | |
| | No. 3 | 14.9 | 2.00E+07 | **19,925,876** |
| | | 14.9 | 1.90E+07 | |
| Great Ruby buckwheat flour 100% | No. 1 | 14.3 | 3.00E+07 | **28,209,852** |
| | | 14.5 | 2.70E+07 | |
| | No. 2 | 14.7 | 2.30E+07 | **22,488,190** |
| | | 14.8 | 2.20E+07 | |
| | No. 3 | 14.4 | 2.80E+07 | **26,490,346** |
| | | 14.6 | 2.50E+07 | |

Sample information (plasmid for standard curves)

| Sample | Ct | Copy number | Average copy number (Fo copy) |
|---|---|---|---|
| **1,000 copy** | 29.7 | 1.00E+03 | **1,000** |
| | 30.0 | 1.00E+03 | |
| **10,000 copy** | 26.3 | 1.00E+04 | **10,000** |
| | 26.3 | 1.00E+04 | |
| **100,000 copy** | 22.9 | 1.00E+05 | **100,000** |
| | 22.8 | 1.00E+05 | |
| **1,000,000 copy** | 19.3 | 1.00E+06 | **1,000,000** |
| | 19.3 | 1.00E+06 | |
| **10,000,000 copy** | 15.7 | 1.00E+07 | **10,000,000** |
| | 15.7 | 1.00E+07 | |
| **100,000,000 copy** | 12.7 | 1.00E+08 | **100,000,000** |
| | 12.8 | 1.00E+08 | |
| No template Control | 45.0 | | **0** |
| | 45.0 | | |

Standard curve

Slope: -3.461 Y-intercept: 40.165

Correlation coefficient: 0.999 Threshold line: 0.26

Baseline: (3, 10)

***Limonium:***

quantitative PCR for copy number of statice sequence

Sample information (buckwheat flour)

| Sample | | Ct | Copy number | Average copy number (Lo copy) |
|---|---|---|---|---|
| Shirahana buckwheat flour 100% | No. 1 | 13.8 | 5.70E+07 | **58,115,672** |
| | | 13.7 | 5.90E+07 | |
| | No. 2 | 14.0 | 4.90E+07 | **48,713,304** |
| | | 14.0 | 4.90E+07 | |
| | No. 3 | 13.6 | 6.20E+07 | **62,454,708** |
| | | 13.6 | 6.30E+07 | |
| | No. 4 | 13.7 | 6.10E+07 | **63,166,024** |
| | | 13.6 | 6.50E+07 | |
| | No. 5 | 13.6 | 6.20E+07 | **62,256,136** |
| | | 13.6 | 6.20E+07 | |
| | No. 6 | 13.7 | 6.10E+07 | **61,832,168** |
| | | 13.6 | 6.20E+07 | |
| Dattan buckwheat flour 100% | No. 1 | 13.6 | 6.50E+07 | **64,791,648** |
| | | 13.6 | 6.50E+07 | |
| | No. 2 | 13.5 | 6.90E+07 | **69,477,952** |
| | | 13.5 | 6.90E+07 | |
| | No. 3 | 13.4 | 7.20E+07 | **69,844,016** |
| | | 13.5 | 6.80E+07 | |
| Takane Ruby buckwheat flour 100% | No. 1 | 13.8 | 5.60E+07 | **58,425,484** |
| | | 13.7 | 6.00E+07 | |
| | No. 2 | 13.5 | 6.70E+07 | **68,158,464** |
| | | 13.5 | 6.90E+07 | |
| | No. 3 | 13.4 | 7.10E+07 | **72,032,008** |
| | | 13.4 | 7.30E+07 | |
| Great Ruby buckwheat flour 100% | No. 1 | 13.5 | 6.70E+07 | **67,316,112** |
| | | 13.5 | 6.80E+07 | |
| | No. 2 | 13.6 | 6.50E+07 | **65,631,320** |
| | | 13.5 | 6.70E+07 | |
| | No. 3 | 13.4 | 7.20E+07 | **71,952,496** |
| | | 13.4 | 7.20E+07 | |

Sample information (plasmid for standard curves)

| Sample | Ct | Copy number | Average copy number (Lo copy) |
|---|---|---|---|
| **1,000 copy** | 29.7 | 1.00E+03 | **1,000** |
| | 30.0 | 1.00E+03 | |
| **10,000 copy** | 26.6 | 1.00E+04 | **10,000** |
| | 26.8 | 1.00E+04 | |
| **100,000 copy** | 23.1 | 1.00E+05 | **100,000** |
| | 23.1 | 1.00E+05 | |
| **1,000,000 copy** | 19.6 | 1.00E+06 | **1,000,000** |
| | 19.6 | 1.00E+06 | |
| **10,000,000 copy** | 16.1 | 1.00E+07 | **10,000,000** |
| | 16.2 | 1.00E+07 | |
| **100,000,000 copy** | 13.1 | 1.00E+08 | **100,000,000** |
| | 13.1 | 1.00E+08 | |
| No template Control | 45.0 | | **0** |
| | 45.0 | | |

Standard curve

Slope: -3.390 Y-intercept: 40.055

Correlation coefficient: 0.999 Threshold line: 0.26

Baseline: (3, 10)

[Table 4A]

Raw data of variety of buckwheat flour samples in Lo/Fo ratio measurement (first measurement)

Lo/Fo ratios of variety of buckwheat flour samples (first measurement)

| Sample | | Lo/Fo ratio of buckwheat flour 100% Measured value | Average |
|---|---|---|---|
| Shirahana buckwheat flour 100% | No. 1 | 2.23 | 2.37 |
| | No. 2 | 2.38 | |
| | No. 3 | 2.12 | |
| | No. 4 | 2.84 | |
| | No. 5 | 2.12 | |
| | No. 6 | 2.50 | |
| Dattan buckwheat flour 100% | No. 1 | 4.33 | 4.82 |
| | No. 2 | 5.42 | |
| | No. 3 | 4.70 | |
| Takane Ruby buckwheat flour 100% | No. 1 | 3.40 | 3.20 |
| | No. 2 | 2.58 | |
| | No. 3 | 3.61 | |
| Great Ruby buckwheat flour 100% | No. 1 | 2.39 | 2.67 |
| | No. 2 | 2.92 | |
| | No. 3 | 2.72 | |

[Table 4B]

Raw data of variety of buckwheat flour samples in Lo/Fo ratio measurement (second measurement)

**Raw data of variety of buckwheat flour samples in second measurement**

***Fagopyrum:***

quantitative PCR for copy number of buckwheat sequence

Sample information (buckwheat flour)

| Sample | | Ct | Copy number | Average copy number (Fo copy) |
|---|---|---|---|---|
| Shirahana buckwheat flour 100% | No. 1 | 14.3 | 2.90E+07 | **26,518,246** |
| | | 14.5 | 2.40E+07 | |
| | No. 2 | 14.7 | 2.10E+07 | **21,164,988** |
| | | 14.7 | 2.10E+07 | |
| | No. 3 | 14.1 | 3.30E+07 | **31,558,050** |
| | | 14.2 | 3.00E+07 | |
| | No. 4 | 14.6 | 2.30E+07 | **23,066,282** |
| | | 14.6 | 2.30E+07 | |
| | No. 5 | 14.1 | 3.20E+07 | **30,485,280** |
| | | 14.2 | 2.90E+07 | |
| | No. 6 | 14.5 | 2.50E+07 | **25,047,642** |
| | | 14.5 | 2.50E+07 | |
| Dattan buckwheat flour 100% | No. 1 | 15.1 | 1.70E+07 | **16,326,365** |
| | | 15.2 | 1.60E+07 | |
| | No. 2 | 15.4 | 1.40E+07 | **13,136,491** |
| | | 15.5 | 1.30E+07 | |
| | No. 3 | 15.0 | 1.70E+07 | **15,677,427** |
| | | 15.3 | 1.40E+07 | |
| Takane Ruby buckwheat flour 100% | No. 1 | 15.0 | 1.70E+07 | **16,998,440** |
| | | 15.1 | 1.70E+07 | |
| | No. 2 | 14.1 | 3.30E+07 | **30,135,004** |
| | | 14.3 | 2.80E+07 | |
| | No. 3 | 14.9 | 1.90E+07 | **18,706,756** |
| | | 15.0 | 1.80E+07 | |
| Great Ruby buckwheat flour 100% | No. 1 | 14.1 | 3.20E+07 | **29,831,912** |
| | | 14.3 | 2.80E+07 | |
| | No. 2 | 14.5 | 2.40E+07 | **23,647,696** |
| | | 14.6 | 2.30E+07 | |
| | No. 3 | 14.4 | 2.60E+07 | **24,742,444** |
| | | 14.6 | 2.30E+07 | |

Sample information (plasmid for standard curves)

| Sample | Ct | Copy number | Average copy number (Fo copy) |
|---|---|---|---|
| **10,000 copy** | 26.2 | 1.00E+04 | **10,000** |
| | 26.3 | 1.00E+04 | |
| **100,000 copy** | 22.8 | 1.00E+05 | **100,000** |
| | 22.6 | 1.00E+05 | |
| **1,000,000 copy** | 19.2 | 1.00E+06 | **1,000,000** |
| | 19.3 | 1.00E+06 | |
| **10,000,000 copy** | 15.7 | 1.00E+07 | **10,000,000** |
| | 15.7 | 1.00E+07 | |
| **100,000,000 copy** | 12.2 | 1.00E+08 | **100,000,000** |
| | 12.3 | 1.00E+08 | |
| **1,000,000,000 copy** | 9.2 | 1.00E+09 | **1,000,000,000** |
| | 9.2 | 1.00E+09 | |
| No template Control | 45.0 | | **0** |
| | 45.0 | | |

Standard curve

Slope: -3.43  Y-intercept: 39.853

Correlation coefficient: 0.999  Threshold line: 0.26

Baseline: (1, 5)

***Limonium:***

quantitative PCR for copy number of statice sequence

Sample information (buckwheat flour)

| Sample | | Ct | Copy number | Average copy number (Lo copy) |
|---|---|---|---|---|
| Shirahana buckwheat flour 100% | No. 1 | 15.9 | 5.90E+07 | **59,483,544** |
| | | 15.9 | 6.00E+07 | |
| | No. 2 | 16.1 | 5.10E+07 | **51,726,840** |
| | | 16.1 | 5.30E+07 | |
| | No. 3 | 15.7 | 6.70E+07 | **66,458,736** |
| | | 15.8 | 6.50E+07 | |
| | No. 4 | 15.9 | 6.00E+07 | **62,320,324** |
| | | 15.8 | 6.40E+07 | |
| | No. 5 | 15.8 | 6.60E+07 | **64,315,224** |
| | | 15.8 | 6.30E+07 | |
| | No. 6 | 15.8 | 6.30E+07 | **64,025,272** |
| | | 15.8 | 6.50E+07 | |
| Dattan buckwheat flour 100% | No. 1 | 15.7 | 6.60E+07 | **66,255,696** |
| | | 15.7 | 6.60E+07 | |
| | No. 2 | 15.6 | 7.10E+07 | **69,291,272** |
| | | 15.7 | 6.80E+07 | |
| | No. 3 | 15.5 | 7.50E+07 | **74,041,168** |
| | | 15.6 | 7.30E+07 | |
| Takane Ruby buckwheat flour 100% | No. 1 | 15.9 | 5.90E+07 | **62,179,320** |
| | | 15.7 | 6.60E+07 | |
| | No. 2 | 15.7 | 7.00E+07 | **72,273,880** |
| | | 15.6 | 7.50E+07 | |
| | No. 3 | 15.7 | 7.00E+07 | **72,066,528** |
| | | 15.6 | 7.40E+07 | |
| Great Ruby buckwheat flour 100% | No. 1 | 15.6 | 7.30E+07 | **71,145,008** |
| | | 15.7 | 6.90E+07 | |
| | No. 2 | 15.7 | 6.80E+07 | **68,944,320** |
| | | 15.7 | 7.00E+07 | |
| | No. 3 | 15.6 | 7.10E+07 | **71,057,568** |
| | | 15.6 | 7.10E+07 | |

Sample information (plasmid for standard curves)

| Sample | Ct | Copy number | Average copy number (Lo copy) |
|---|---|---|---|
| **10,000 copy** | 28.6 | 1.00E+04 | **10,000** |
| | 28.8 | 1.00E+04 | |
| **100,000 copy** | 25.4 | 1.00E+05 | **100,000** |
| | 25.6 | 1.00E+05 | |
| **1,000,000 copy** | 21.7 | 1.00E+06 | **1,000,000** |
| | 21.8 | 1.00E+06 | |
| **10,000,000 copy** | 18.4 | 1.00E+07 | **10,000,000** |
| | 18.4 | 1.00E+07 | |
| **100,000,000 copy** | 15.1 | 1.00E+08 | **100,000,000** |
| | 15.2 | 1.00E+08 | |
| **1,000,000,000 copy** | 11.8 | 1.00E+09 | **1,000,000,000** |
| | 11.8 | 1.00E+09 | |
| No template Control | 45.0 | | **0** |
| | 45.0 | | |

Standard curve

Slope: -3.398  Y-intercept: 42.303

Correlation coefficient: 0.999  Threshold line: 1.02

Baseline: (1, 5)

[Table 4B]

Raw data of variety of buckwheat flour samples in Lo/Fo ratio measurement (second measurement)

Lo/Fo ratios of variety of buckwheat flour samples (second measurement)

| Sample | Lo/Fo ratio of buckwheat flour 100% Measured value | | Average |
|---|---|---|---|
| Shirahana buckwheat flour 100% | No. 1 | 2.24 | 2.36 |
| | No. 2 | 2.44 | |
| | No. 3 | 2.11 | |
| | No. 4 | 2.70 | |
| | No. 5 | 2.11 | |
| | No. 6 | 2.56 | |
| Dattan buckwheat flour 100% | No. 1 | 4.06 | 4.69 |
| | No. 2 | 5.27 | |
| | No. 3 | 4.72 | |
| Takane Ruby buckwheat flour 100% | No. 1 | 3.66 | 3.30 |
| | No. 2 | 2.40 | |
| | No. 3 | 3.85 | |
| Great Ruby buckwheat flour 100% | No. 1 | 2.38 | 2.72 |
| | No. 2 | 2.92 | |
| | No. 3 | 2.87 | |

The measurement value of the Dattan buckwheat flour deviated most from the measurement value of the Shirahana buckwheat flour and however, was only about twice the measurement value of the Shirahana buckwheat flour. Thus, the present method is considered to have sufficient precision as a quantifying method by PCR.

G. Calculation of "copy number of buckwheat sequence/copy number of statice sequence ratio" in DNA extracted from artificially contaminated sample supplemented with statice standard and calculation of amount of buckwheat contaminating artificially contaminated sample

The quantitative PCR methods for the buckwheat sequence and the statice sequence were conducted by the method described in Example 1.F. and Example 1.G. Based on the standard curves, the copy number of the buckwheat sequence and the copy number of the statice sequence of 50 ng of DNA extracted from the artificially contaminated sample supplemented with the statice standard were quantified. Based on the quantitative values, "the copy number of the buckwheat sequence/the copy number of the statice sequence = Fs/Ls ratio" was calculated. The Fs/Ls ratio of the artificially contaminated sample was calculated by extracting 3 samples from the same sample, each of which was measured in 2 wells. The amount ($\mu$g) of buckwheat contaminating the artificially contaminated sample (1 g) was determined using the Fs/Ls ratio calculated here and the Lo/Fo ratio calculated in Example 2.F according to an equation

below.

$$\text{Amount of contaminating buckwheat (ppm } (\mu g/g)) = Fs/Ls \times Lo/Fo \times 1{,}000{,}000$$

As a result of Fs/Ls ratio measurement and the calculation of the amount of contaminating buckwheat, a reasonable value could be obtained in both of two measurements for the wheat pulverized powder containing 100 ppm of Shirahana buckwheat flour, the wheat pulverized powder containing 10 ppm of Shirahana buckwheat flour, the rice pulverized powder containing 10 ppm of Shirahana buckwheat flour, and the pulverized powder of wheat and rice containing 10 ppm of Shirahana buckwheat flour, as shown in Table 5. The raw data of a variety of artificially contaminated sample in Fs/Ls ratio measurement is shown in Tables 6A and 6B.

[Table 5] Summary of measurement result for amount of buckwheat contaminating in artificially contaminated sample (PCR) Measurement result for amount of buckwheat contaminating in artificially contaminated sample

| Artificially contaminated sample name | | Buckwheat flour concentration (ppm : μg/g) | |
|---|---|---|---|
| | | 1st measurement | 2nd measurement |
| Wheat containing 100 ppm buckwheat | No. 1 | 97.9 | 83.0 |
| | No. 2 | 84.5 | 75.5 |
| | No. 3 | 89.6 | 81.6 |
| Wheat containing 10 ppm buckwheat | No.1 | 6.4 | 4.6 |
| | No. 2 | 14.4 | 10.8 |
| | No. 3 | 8.9 | 7.7 |
| Rice containing 100 ppm buckwheat | No. 1 | 9.0 (Reference value) | 7.5 |
| | No. 2 | 7.5 | 5.1 |
| | No. 3 | 5.5 (Reference value) | 4.7 |
| Rice and wheat containing 10 ppm buckwheat | No. 1 | 9.2 | 6.2 |
| | No. 2 | 7.0 | 4.9 |
| | No. 3 | 9.0 | 8.2 |

* Three samples were extracted from each artificially contaminated sample and each measurement was performed with n=2.

* 50 ng of DNA extracted from 5 g of artificially contaminated sample supplemented with 1 g of Limonium (statice) standard was subjected to PCR.

* Lo/Fo value=2.36 of Shirahana buckwheat was used in calculation of buckwheat flour concentration (ppm) in artificially contaminated sample.

* No. 1 and No. 3 samples of rice containing 10 ppm buckwheat were indicated by reference values because they fell outside standard curve range in quantification of copy number of statice sequence.

[Table 6A]

Raw data of variety of artificially contaminated samples in measurement of amount of contaminating buckwheat (first measurement)

**Raw data of artificially contaminated samples in first measurement**

***Fagopyrum:***

quantitative PCR for copy number of buckwheat sequence

Sample information (artificially contaminated sample)

| Sample | | Ct | Copy number | Average copy number (Fs copy) |
|---|---|---|---|---|
| Wheat containing 100 ppm buckwheat | No. 1 | 29.4 | 1.40E+03 | **1,393** |
| | | 29.4 | 1.40E+03 | |
| | No. 2 | 29.7 | 1.10E+03 | **1,162** |
| | | 29.6 | 1.20E+03 | |
| | No. 3 | 29.6 | 1.20E+03 | **1,280** |
| | | 29.5 | 1.30E+03 | |
| Wheat containing 10 ppm buckwheat | No. 1 | 33.7 | 7.90E+01 | **82** |
| | | 33.6 | 8.50E+01 | |
| | No. 2 | 32.3 | 2.10E+02 | **200** |
| | | 32.4 | 1.90E+02 | |
| | No. 3 | 33.1 | 1.20E+02 | **121** |
| | | 33.1 | 1.20E+02 | |
| Rice containing 10 ppm buckwheat | No. 1 | 31.1 | 4.40E+02 | **445** |
| | | 31.1 | 4.50E+02 | |
| | No. 2 | 31.7 | 2.90E+02 | **300** |
| | | 31.7 | 3.00E+02 | |
| | No. 3 | 32.0 | 2.50E+02 | **260** |
| | | 31.9 | 2.70E+02 | |
| Rice + Wheat containing 10 ppm buckwheat | No. 1 | 32.1 | 2.40E+02 | **214** |
| | | 32.4 | 1.90E+02 | |
| | No. 2 | 33.0 | 1.30E+02 | **143** |
| | | 32.7 | 1.60E+02 | |
| | No. 3 | 32.3 | 2.10E+02 | **210** |
| | | 32.3 | 2.10E+02 | |

***Limonium:***

quantitative PCR for copy number of statice sequence

Sample information (artificially contaminated sample)

| Sample | | Ct | Copy number | Average copy number (Ls copy) |
|---|---|---|---|---|
| Wheat containing 100 ppm buckwheat | No. 1 | 14.6 | 3.40E+07 | **33,597,292** |
| | | 14.6 | 3.30E+07 | |
| | No. 2 | 14.7 | 3.20E+07 | **32,452,070** |
| | | 14.6 | 3.30E+07 | |
| | No. 3 | 14.6 | 3.40E+07 | **33,721,376** |
| | | 14.6 | 3.30E+07 | |
| Wheat containing 10 ppm buckwheat | No. 1 | 14.7 | 3.00E+07 | **30,161,998** |
| | | 14.8 | 3.00E+07 | |
| | No. 2 | 14.6 | 3.30E+07 | **32,846,720** |
| | | 14.6 | 3.20E+07 | |
| | No. 3 | 14.6 | 3.20E+07 | **31,901,436** |
| | | 14.7 | 3.10E+07 | |
| Rice containing 10 ppm buckwheat | No. 1 | 12.8 | 1.10E+08 | **116,638,192** |
| | | 12.7 | 1.20E+08 | |
| | No. 2 | 13.1 | 9.40E+07 | **94,101,296** |
| | | 13.1 | 9.40E+07 | |
| | No. 3 | 12.8 | 1.10E+08 | **112,316,848** |
| | | 12.8 | 1.10E+08 | |
| Rice + Wheat containing 10 ppm buckwheat | No. 1 | 13.9 | 5.50E+07 | **54,842,760** |
| | | 13.9 | 5.50E+07 | |
| | No. 2 | 14.1 | 4.70E+07 | **48,308,684** |
| | | 14.0 | 4.90E+07 | |
| | No. 3 | 13.8 | 5.70E+07 | **55,342,960** |
| | | 13.9 | 5.40E+07 | |

Sample information (plasmid for standard curves)

| Sample | Ct | Copy number | Average copy number (Fo copy) |
|---|---|---|---|
| **10 copy** | 37.2 | 1.00E+01 | **10** |
| | 37.3 | 1.00E+01 | |
| **100 copy** | 33.1 | 1.00E+02 | **100** |
| | 33.4 | 1.00E+02 | |
| **1,000 copy** | 29.7 | 1.00E+03 | **1,000** |
| | 29.7 | 1.00E+03 | |
| **10,000 copy** | 26.3 | 1.00E+04 | **10,000** |
| | 26.3 | 1.00E+04 | |
| **100,000 copy** | 22.9 | 1.00E+05 | **100,000** |
| | 22.8 | 1.00E+05 | |
| **1,000,000 copy** | 19.2 | 1.00E+06 | **1,000,000** |
| | 19.3 | 1.00E+06 | |
| **10,000,000 copy** | 15.7 | 1.00E+07 | **10,000,000** |
| | 15.7 | 1.00E+07 | |
| **100,000,000 copy** | 12.7 | 1.00E+08 | **100,000,000** |
| | 12.7 | 1.00E+08 | |
| No template Control | 45.0 | | **0** |
| | 45.0 | | |

Standard curve

Slope: -3.504 Y-intercept: 40.394

Correlation coefficient: 0.999 Threshold line: 0.26

Baseline: (3, 10)

Sample information (plasmid for standard curves)

| Sample | Ct | Copy number | Average copy number (Lo copy) |
|---|---|---|---|
| **10 copy** | 36.7 | 1.00E+01 | **10** |
| | 36.6 | 1.00E+01 | |
| **100 copy** | 33.2 | 1.00E+02 | **100** |
| | 33.4 | 1.00E+02 | |
| **1,000 copy** | 29.8 | 1.00E+03 | **1,000** |
| | 29.8 | 1.00E+03 | |
| **10,000 copy** | 26.7 | 1.00E+04 | **10,000** |
| | 27.1 | 1.00E+04 | |
| **100,000 copy** | 23.1 | 1.00E+05 | **100,000** |
| | 23.0 | 1.00E+05 | |
| **1,000,000 copy** | 19.6 | 1.00E+06 | **1,000,000** |
| | 19.8 | 1.00E+06 | |
| **10,000,000 copy** | 16.2 | 1.00E+07 | **10,000,000** |
| | 16.2 | 1.00E+07 | |
| **100,000,000 copy** | 13.1 | 1.00E+08 | **100,000,000** |
| | 13.2 | 1.00E+08 | |
| No template Control | 45.0 | | **0** |
| | 45.0 | | |

Standard curve

Slope: -3.382 Y-intercept: 40.043

Correlation coefficient: 0.999 Threshold line: 0.26

Baseline: (3, 10)

[Table 6A]

Raw data of variety of artificially contaminated samples in measurement of amount of contaminating buckwheat (first measurement)

| Result of calculating amount of contaminating buckwheat | | | Amount of contaminating buckwheat (ppm: μg/g) |
|---|---|---|---|
| Sample | | Fs/Ls | Fo/Lo = 2.36 |
| Wheat containing 100 ppm buckwheat | No. 1 | 4.1E-05 | **97.9 ppm** |
| | No. 2 | 3.6E-05 | **84.5 ppm** |
| | No. 3 | 3.8E-05 | **89.6 ppm** |
| Wheat containing 10 ppm buckwheat | No. 1 | 2.7E-06 | **6.4 ppm** |
| | No. 2 | 6.1E-06 | **14.4 ppm** |
| | No. 3 | 3.8E-06 | **8.9 ppm** |
| Rice containing 10 ppm buckwheat | No. 1 | 3.8E-06 | **9.0 ppm** (Reference) |
| | No. 2 | 3.2E-06 | **7.5 ppm** |
| | No. 3 | 2.3E-06 | **5.5 ppm** (Reference) |
| Rice + Wheat containing 10 ppm buckwheat | No. 1 | 3.9E-06 | **9.2 ppm** |
| | No. 2 | 3.0E-06 | **7.0 ppm** |
| | No. 3 | 3.8E-06 | **9.0 ppm** |

*Artificially contaminated samples of rice containing 10 ppm buckwheat were indicated by reference values because copy number of statice sequence exceeded standard curve range.

[Table 6B] Raw data of variety of artificially contaminated samples in measurement of amount of contaminating buckwheat (second measurement)

**Raw data of artificially contaminated samples in second measurement**

***Fagopyrum:***

quantitative PCR for copy number of buckwheat sequence

Sample information (artificially contaminated sample)

| Sample | | Ct | Copy number | Average copy number (Fs copy) |
|---|---|---|---|---|
| Wheat containing 100 ppm buckwheat | No. 1 | 30.8 | 1.20E+03 | 1,180 |
| | | 30.8 | 1.20E+03 | |
| | No. 2 | 31.1 | 9.40E+02 | 1,018 |
| | | 30.9 | 1.10E+03 | |
| | No. 3 | 30.9 | 1.10E+03 | 1,143 |
| | | 30.8 | 1.20E+03 | |
| Wheat containing 10 ppm buckwheat | No. 1 | 35.2 | 6.20E+01 | 58 |
| | | 35.4 | 5.40E+01 | |
| | No. 2 | 34.1 | 1.30E+02 | 148 |
| | | 33.7 | 1.70E+02 | |
| | No. 3 | 34.6 | 9.20E+01 | 108 |
| | | 34.2 | 1.20E+02 | |
| Rice containing 10 ppm buckwheat | No. 1 | 32.4 | 4.00E+02 | 423 |
| | | 32.3 | 4.40E+02 | |
| | No. 2 | 33.1 | 2.50E+02 | 231 |
| | | 33.4 | 2.10E+02 | |
| | No. 3 | 33.3 | 2.20E+02 | 249 |
| | | 33.0 | 2.80E+02 | |
| Rice + Wheat containing 10 ppm buckwheat | No. 1 | 33.7 | 1.70E+02 | 147 |
| | | 34.1 | 1.30E+02 | |
| | No. 2 | 34.5 | 9.80E+01 | 109 |
| | | 34.2 | 1.20E+02 | |
| | No. 3 | 33.3 | 2.20E+02 | 209 |
| | | 33.5 | 2.00E+02 | |

Sample information (plasmid for standard curves)

| Sample | Ct | Copy number | Average copy number (Fo copy) |
|---|---|---|---|
| 10 copy | 38.0 | 1.00E+01 | 10 |
| | 38.1 | 1.00E+01 | |
| 100 copy | 35.1 | 1.00E+02 | 100 |
| | 34.2 | 1.00E+02 | |
| 1,000 copy | 31.0 | 1.00E+03 | 1,000 |
| | 31.2 | 1.00E+03 | |
| 10,000 copy | 27.5 | 1.00E+04 | 10,000 |
| | 27.5 | 1.00E+04 | |
| 100,000 copy | 24.0 | 1.00E+05 | 100,000 |
| | 24.0 | 1.00E+05 | |
| 1,000,000 copy | 20.4 | 1.00E+06 | 1,000,000 |
| | 20.4 | 1.00E+06 | |
| 10,000,000 copy | 16.9 | 1.00E+07 | 10,000,000 |
| | 16.9 | 1.00E+07 | |
| 100,000,000 copy | 13.6 | 1.00E+08 | 100,000,000 |
| | 13.7 | 1.00E+08 | |
| 1,000,000,000 copy | 10.5 | 1.00E+09 | 1,000,000,000 |
| | 10.6 | 1.00E+09 | |
| | 45.0 | | 0 |
| | 45.0 | | |

Standard curve

Slope: -3.475 Y-intercept: 41.45

Correlation coefficient: 0.999 Threshold line: 0.51

Baseline: (3, 8)

***Limonium:***

quantitative PCR for copy number of statice sequence

Sample information (artificially contaminated sample)

| Sample | | Ct | Copy number | Average copy number (Ls copy) |
|---|---|---|---|---|
| Wheat containing 100 ppm buckwheat | No. 1 | 16.4 | 3.40E+07 | 33,560,780 |
| | | 16.4 | 3.30E+07 | |
| | No. 2 | 16.5 | 3.10E+07 | 31,837,948 |
| | | 16.4 | 3.20E+07 | |
| | No. 3 | 16.4 | 3.30E+07 | 33,067,796 |
| | | 16.4 | 3.30E+07 | |
| Wheat containing 10 ppm buckwheat | No. 1 | 16.6 | 3.00E+07 | 29,930,496 |
| | | 16.5 | 3.00E+07 | |
| | No. 2 | 16.4 | 3.30E+07 | 32,418,082 |
| | | 16.5 | 3.20E+07 | |
| | No. 3 | 16.4 | 3.30E+07 | 32,839,880 |
| | | 16.4 | 3.30E+07 | |
| Rice containing 10 ppm buckwheat | No. 1 | 14.4 | 1.30E+08 | 133,923,120 |
| | | 14.3 | 1.40E+08 | |
| | No. 2 | 14.7 | 1.10E+08 | 107,428,504 |
| | | 14.7 | 1.10E+08 | |
| | No. 3 | 14.4 | 1.30E+08 | 125,724,392 |
| | | 14.4 | 1.30E+08 | |
| Rice + Wheat containing 10 ppm buckwheat | No. 1 | 15.6 | 5.60E+07 | 56,432,404 |
| | | 15.6 | 5.70E+07 | |
| | No. 2 | 15.7 | 5.20E+07 | 52,445,608 |
| | | 15.7 | 5.30E+07 | |
| | No. 3 | 15.5 | 5.90E+07 | 60,105,248 |
| | | 15.5 | 6.10E+07 | |

Sample information (plasmid for standard curves)

| Sample | Ct | Copy number | Average copy number (Lo copy) |
|---|---|---|---|
| | | | |
| 100 copy | 35.2 | 1.00E+02 | 100 |
| | 35.3 | 1.00E+02 | |
| 1,000 copy | 31.5 | 1.00E+03 | 1,000 |
| | 31.6 | 1.00E+03 | |
| 10,000 copy | 28.4 | 1.00E+04 | 10,000 |
| | 28.6 | 1.00E+04 | |
| 100,000 copy | 24.7 | 1.00E+05 | 100,000 |
| | 24.9 | 1.00E+05 | |
| 1,000,000 copy | 21.5 | 1.00E+06 | 1,000,000 |
| | 21.6 | 1.00E+06 | |
| 10,000,000 copy | 17.9 | 1.00E+07 | 10,000,000 |
| | 17.9 | 1.00E+07 | |
| 100,000,000 copy | 14.7 | 1.00E+08 | 100,000,000 |
| | 14.8 | 1.00E+08 | |
| 1,000,000,000 copy | 11.6 | 1.00E+09 | 1,000,000,000 |
| | 11.6 | 1.00E+09 | |
| | 45.0 | | 0 |
| | 45.0 | | |

Standard curve

Slope: -3.385 Y-intercept: 41.855

Correlation coefficient: 0.999 Threshold line: 0.77

Baseline: (3, 8)

[Table 6B]

Raw data of variety of artificially contaminated samples in measurement of amount of contaminating buckwheat (second measurement)

| Result of calculating amount of contaminating buckwheat<br>Sample | | Fs/Ls | Amount of contaminating buckwheat (ppm: μg/g)<br>Fo/Lo = 2.36 |
|---|---|---|---|
| Wheat containing 100 ppm buckwheat | No. 1 | 3.5E-05 | 83.0 ppm |
| | No. 2 | 3.2E-05 | 75.5 ppm |
| | No. 3 | 3.5E-05 | 81.6 ppm |
| Wheat containing 10 ppm buckwheat | No. 1 | 1.9E-06 | 4.6 ppm |
| | No. 2 | 4.6E-06 | 10.8 ppm |
| | No. 3 | 3.3E-06 | 7.7 ppm |
| Rice containing 10 ppm buckwheat | No. 1 | 3.2E-06 | 7.5 ppm |
| | No. 2 | 2.2E-06 | 5.1 ppm |
| | No. 3 | 2.0E-06 | 4.7 ppm |
| Rice + Wheat containing 10 ppm buckwheat | No. 1 | 2.6E-06 | 6.2 ppm |
| | No. 2 | 2.1E-06 | 4.9 ppm |
| | No. 3 | 3.5E-06 | 8.2 ppm |

Example 3

A. Plant sample used in DNA extraction

(1) Peanut, buckwheat (Shirahana buckwheat), and statice seeds:

The same seeds as Example 1.A.(1) and Example 1.A.(2) were used.

(2) Wheat, soybean, and maize leaves:

The same leaves as Example 1.A.(3) were used.

(3) Adzuki bean, almond, walnut, macadamia nut, and hazelnut seeds, pine nut, sunflower seed, poppy seed, sesame, and apple:

Commercially-available products were used.

(4) Buckwheat (Shirahana buckwheat) and adzuki bean leaves

Leaves germinated from commercially-available seeds were used.

B. DNA extraction

(1) DNA extraction from statice seed

DNA extraction was conducted in the same way as Example 1.B.(2).

(2) DNA extraction from peanut, almond, and hazelnut seeds, poppy seed, and sesame:

DNA extraction was conducted in the same way as Example 1.B.(3).

(3) DNA extraction from buckwheat (Shirahana buckwheat), wheat, soybean, maize, and adzuki bean leaves, and apple seed:

DNA extraction was conducted in the same way as Example 1.B.(4).

(4) DNA extraction from macadamia nut seed:

DNA extraction was conducted using Genomic-tip manufactured by QIAGEN with reference to QIAGEN Genomic DNA Handbook according to procedures below.

In a 50-ml tube, 1 g of a pulverized sample was introduced and 10 ml of Buffer G2, 200 μl of proteinase K (20 mg/ml), and 20 μl of RNase A (100 mg/ml) were added and mixed, followed by incubation at 50°C for 1 hour. The resulting mixture was then centrifuged at approximately 3,000 x g for 10 minutes to obtain its supernatant. The supernatant from which oil contents and powders were removed was further centrifuged at approximately 3,000 x g for 10 minutes to obtain its supernatant. The obtained supernatant was applied to Genomic-tip 20/G Column equilibrated with 1 ml of Buffer QBT, to which DNA was then adsorbed. Then, the Column was washed with 4 ml of Buffer QC. A precipitate collected by elution with 1 ml of Buffer QF preheated to 50°C and isopropanol precipitation was dissolved in 100 μl of sterilized ultrapure water. A DNA concentration in the resulting solution was measured, and the DNA solution appropriately diluted with sterilized ultrapure water was used as a template DNA sample for PCR.

(5) DNA extraction from walnut seed, pine nut, and sunflower seed:

DNA extraction was conducted using DNeasy Plant Maxi Kit manufactured by QIAGEN with reference to DNeasy Plant Maxi Kit Handbook according to procedures below.

In a 15-ml tube, 1 g of a pulverized sample was introduced and 10 ml of Buffer AP1 and 10 μl of RNase A (100 mg/ml) were added and mixed, followed by incubation at 65°C for 60 minutes. The resulting solution was then centrifuged at approximately 3,000 x g for 10 minutes to obtain its supernatant. To this supernatant, 1.5 ml of Buffer AP2 was added. The resulting mixture was left on ice for 10 minutes and centrifuged to obtain its supernatant. The obtained supernatant was applied to QIAshredder Spin Column to obtain a flow-through solution from the Column by centrifugation. To this flow-through solution, 1.5 volumes of Buffer AP3 and 1 volume of ethanol were added and mixed. The resulting mixture was applied to DNeasy Spin Column and centrifuged at approximately 1,500 x g for 1 minute to have DNA adsorbed to the Column. Then, 10 ml of Buffer AW was added to the Column and centrifuged at approximately 1,500 x g for 1 minute, followed by the washing of the Column. Again, 10 ml of Buffer AW was added to the Column and centrifuged at approximately 1,500 x g for 1 minute. Subsequently, the Buffer AW that remained in the Column was completely eliminated by centrifugation at approximately 3,000 x g for 10 minutes. Finally, 1 ml of sterilized ultrapure water preincubated at 65°C was added to the Column and left for 5 minutes. The Column was then centrifuged at approximately 3,000 x g for 5 minutes to elute DNA from the Column. A precipitate collected by isopropanol precipitation was dissolved in 100 μl of sterilized ultrapure water. A DNA concentration in the resulting solution was measured, and the DNA solution appropriately diluted with sterilized ultrapure water was used as a template DNA sample for PCR.

C. PCR that detects a portion of ITS-1 sequence of peanut

(1) Primers for detecting peanut:

Sequences universal to the ITS-1 sequences of the following 11 sequences registered in GenBank of plants belonging to the genus *Arachis* were used as primer sequences. Concerning *Arachis hypogaea* among these plants, a sequence obtained from the analysis of a commercially-available peanut was also used, in place of *Arachis hypogaea* (AF156675) registered in GenBank.

*1: Arachis batizocoi* (AF203553)
*2: Arachis correntina* (AF203554)
*3: Arachis hermannii* (AF203556)
*4: Arachis hoehnei* (AJ320395)
*5: Arachis hypogaea* (AF156675 and sequence obtained from analysis of commercially-available peanut)
6: *Arachis magna* (AF203555)
7: *Arachis major* (AF203552)
8: *Arachis palustris* (AF203557)
9: *Arachis pintoi* (AF203551)
10: *Arachis triseminata* (AF204233)
11: *Arachis villosa* (AF203558)

Then, oligo DNA primers (manufacture by QIAGEN, OPC-purified oligonucleotides) having sequences below were synthesized and used as primers for PCR that detected a portion of the ITS-1 sequence of a peanut (hereinafter, referred to as peanut PCR).

5'-GCG GAA AGC GCC AAG GAA GC-3' (SEQ ID NO:21)

5'-GTC GCC CCG ACC GGA TG-3' (SEQ ID NO:66)

5'-CGT CGC CCC GAC CGG AT-3' (SEQ ID NO:26)

5'-TCG TCG CCC CGA CCG GAT G-3' (SEQ ID NO:65)

(2) Specificity of primers for detecting peanut (PCR simulation):

A PCR simulation software Amplify 1.0 (Bill Engels) was used to confirm whether a result of the simulation showed that a PCR amplification product was obtained with the primers for detecting a peanut, based on 11 sequences of plants belonging to the genus *Arachis,* 8 sequences of likely-to-be-allergenic plants other than a peanut (buckwheat, wheat, soybean, walnut, matsutake mushroom, peach, apple, and orange), 8 sequences of plants frequently used as food ingredients (maize, rice, pepper, mustard, carrot, shiitake mushroom, Chinese cabbage, and turnip), 6 sequences of plants of the family *Leguminosae* (kidney bean, lima bean, lentil, chickpea, mung bean, and adzuki bean), 69 sequences of related plant species of a peanut, and statice. The related plant species of a peanut used herein refer to plants other than the genus *Arachis,* which attained Score 60 bits or more when the ITS-1 sequence portion in the nucleotide sequence (AF156675) of a peanut, *Arachis hypogaea,* registered in GenBank was subjected to BLAST homology search. This time, the sequence of a species attaining the highest score in a genus to which each of the plants belonged was selected as a representative sequence of the genus. The PCR simulation was conducted for the ITS-1-5.8S rRNA gene-ITS-2 sequence region of that sequence. The GenBank Accession Number of the sequence used in the simulation and a result of the simulation in the case of using the combination of the primers of SEQ ID NOs: 21 and 65 are shown as a representative in Tables 7A to 7E. Abbreviated letters and symbols in Tables 7A to 7E are as shown below:

Filled-in asterisk: those expected to yield a PCR amplification product having a size around a target size (±10 bp)
W value: Possibility of yielding a PCR amplification product
High possibility ... W6>W5>W4>W3>W2 ... Low possibility
Numeric (bp): the size (bp) of a PCR amplification product

A value where 2 was subtracted from a value obtained in the amplification -: those expected to yield no PCR amplification product

[Table 7A]

| Primers for detecting peanut (SEQ ID NOs: 21 and 65): amplification product | | | | | | | |
|---|---|---|---|---|---|---|---|
| Scientific name (Common name) | | GenBank Accession No. | W6 | W5 | W4 | W3 | W2 |
| Genus Arachis | ★*Arachis batizocoi* | AF203553 | 76bp | - | - | - | - |
| | ★*Arachis correntina* | AF203554 | 76bp | - | - | - | - |
| | ★*Arachis hermannii* | AF203556 | 76bp | - | - | - | - |
| | ★*Arachis hoehnei* | AJ320395 | 76bp | - | - | - | - |
| | ★*Arachis hypogaea* (Commercially-available peanut) | - | 76bp | - | - | - | - |
| | ★*Arachis magna* | AF203555 | 76bp | - | - | - | - |
| | ★*Arachis major* | AF203552 | 76bp | - | - | - | - |
| | ★*Arachis palustris* | AF203557 | 76bp | - | - | - | - |
| | ★*Arachis pintoi* | AF203551 | 76bp | - | - | - | - |
| | ★*Arachis triseminata* | AF204233 | 76bp | - | - | - | - |
| | ★*Arachis villosa* | AF203558 | 76bp | - | - | - | - |
| Related plant species of peanut | *Stylosanthes acuminata* | AJ320282 | - | - | - | - | - |
| | *Stylosanthes angustifolia* | AJ320284 | - | - | - | - | - |
| | *Stylosanthes aurea* | AJ320285 | - | - | - | - | - |
| | *Stylosanthes biflora* | AJ320289 | - | - | - | - | - |
| | *Stylosanthes bracteata* | AJ320346 | - | - | - | - | - |
| | *Stylosanthes calcicola* | AJ320348 | - | - | - | - | - |
| | *Stylosanthes campestris* | AJ320291 | - | - | - | - | - |
| | *Stylosanthes capitata* | AJ320350 | - | - | - | - | - |
| | *Stylosanthes cayennensis* | AJ320292 | - | - | - | - | - |
| | *Stylosanthes erecta* | AJ320352 | - | - | - | - | - |
| | *Stylosanthes fruticosa* | AJ320356 | - | - | - | - | - |
| | *Stylosanthes gracilis* | AJ320296 | - | - | - | - | - |
| | *Stylosanthes grandifolia* | AJ320299 | - | - | - | - | - |
| | *Stylosanthes guianensis subsp. dissitiflora* | AJ320301 | - | - | - | - | - |
| | *Stylosanthes hamata* | AJ320365 | - | - | - | - | - |
| | *Stylosanthes hippocampoides* | AJ320317 | - | - | - | - | - |
| | *Stylosanthes hispida* | AJ320328 | - | - | - | - | - |
| | *Stylosanthes humilis* | AJ320323 | - | - | - | - | - |
| | *Stylosanthes ingrata* | AJ320329 | - | - | - | - | - |
| | *Stylosanthes leiocarpa* | AJ320332 | - | - | - | - | - |

[Table 7B]

| Primers for detecting peanut (SEQ ID NOs: 21 and 65): amplification product | | | | | | | |
|---|---|---|---|---|---|---|---|
| Scientific name (Common name) | | GenBank Accession No. | W6 | W5 | W4 | W3 | W2 |
| Related plant species of peanut | *Stylosanthes linearifolia* | AJ320367 | - | - | - | - | - |
| | *Stylosanthes macrocarpa* | AJ320369 | - | - | - | - | - |
| | *Stylosanthes macrocephala* | AJ320371 | - | - | - | - | - |
| | *Stylosanthes macrosoma* | AJ320333 | - | - | - | - | - |
| | *Stylosanthes mexicana* | AJ320374 | - | - | - | - | - |
| | *Stylosanthes montevidensis* | AJ320336 | - | - | - | - | - |
| | *Stylosanthes pilosa* | AJ320377 | - | - | - | - | - |
| | *Stylosanthes scabra* | AJ320382 | - | - | - | - | - |
| | *Stylosanthes seabrana* | AJ320384 | - | - | - | - | - |
| | *Stylosanthes sericeiceps* | AJ320386 | - | - | - | - | - |
| | *Stylosanthes subsericea* | AJ320387 | - | - | - | - | - |
| | *Stylosanthes sundaica* | AJ320389 | - | - | - | - | - |
| | *Stylosanthes sympodialis* | AJ320391 | - | - | - | - | - |
| | *Stylosanthes tomentosa* | AJ320337 | - | - | - | - | - |
| | *Stylosanthes tuberculata* | AJ320392 | - | - | - | - | - |
| | *Stylosanthes viscose* | AJ320340 | - | - | - | - | - |
| | *Ormocarpum bernierianum* | AF189036 | - | - | - | - | - |
| | *Ormocarpum coeruleum* | AF189037 | - | - | - | - | - |
| | *Ormocarpum drakei* | AF189039 | - | - | - | - | - |
| | *Ormocarpum flavum* | AF189041 | - | - | - | - | - |
| | *Ormocarpum keniense* | AF068155 | - | - | - | - | - |
| | *Ormocarpum kirkii* | AF068152 | - | - | - | - | - |
| | *Ormocarpum klainei* | AF189044 | - | - | - | - | - |
| | *Ormocarpum megalophyllum* | AF068154 | - | - | - | - | - |
| | *Ormocarpum muricatum* | AF068156 | - | - | - | - | - |
| | *Ormocarpum orientale* | AF068159 | - | - | - | - | - |
| | *Ormocarpum pubescens* | AF189045 | - | - | - | - | - |
| | *Ormocarpum rectangulare* | AF189046 | - | - | - | - | - |
| | *Ormocarpum schliebenii* | AF189047 | - | - | - | - | - |
| | *Ormocarpum sennoides* | AF068153 | - | - | - | - | - |
| | *Ormocarpum somalense* | AF189048 | - | - | - | - | - |
| | *Ormocarpum trachycarpum* | AF189049 | - | - | - | - | - |

[Table 7C]

| Primers for detecting peanut (SEQ ID NOs: 21 and 65): amplification product | | | | | | | |
|---|---|---|---|---|---|---|---|
| Scientific name (Common name) | | GenBank Accession No. | W6 | W5 | W4 | W3 | W2 |
| Related plant species of peanut | *Ormocarpum trichocarpum* | AF068158 | - | - | - | - | - |
| | *Ormocarpum verrucosum* | AF189050 | - | - | - | - | - |
| | *Chapmannia floridana* | AF203543 | - | - | - | - | - |
| | *Chapmannia prismatica* | AJ320400 | - | - | - | - | - |
| | *Chapmannia somalensis* | AF203544 | - | - | - | - | - |
| | *Ormocarpopsis aspera* | AF068148 | - | - | - | - | - |
| | *Ormocarpopsis calcicola* | AF068145 | - | - | - | - | - |
| | *Ormocarpopsis itremoensis* | AF068149 | - | - | - | - | - |
| | *Ormocarpopsis mandrarensis* | AF068147 | - | - | - | - | - |
| | *Ormocarpopsis parvifolia* | AF068144 | - | - | - | - | - |
| | *Ormocarpopsis tulearensis* | AF068146 | - | - | - | - | - |
| | *Diphysa humilis* | AF068162 | - | - | - | - | - |
| | *Diphysa macrophylla* | AF189029 | - | - | - | - | - |
| | *Diphysa suberosa* | AF189034 | - | - | - | - | - |
| | *Spigelia coelostylioides* | AF177992 | - | - | - | - | - |
| | *Spigelia hedyotidea* | AF178005 | - | - | - | - | - |
| | *Spigelia marilandica* | AF177991 | - | - | - | - | - |
| Edible plants of family Leguminosae | *Phaseolus vulgaris* (Kidney bean) | AF069128, AF115161 to AF115163, AF115169 | - | - | - | - | - |
| | *Cicer arietinum* (Chickpea) | AJ237698 | - | - | - | - | - |
| | *Lens culinaris subsp. culinaris* (Lentil) | AF228065, AF228066, AJ404739 | - | - | - | - | - |
| | *Phaseolus lunatus* (Lima bean) | AF069129, AF115171, AF115175 | - | - | - | - | - |
| | *Vigna angularis var. nipponensis* (Adzuki bean) | AB059747 | - | - | - | - | - |
| | *Vigna radiate* (Mung bean) | X14337, AB059848 | - | - | - | - | - |

[Table 7D]

| Primers for detecting peanut (SEQ ID NOs: 21 and 65): amplification product | | | | | | | |
|---|---|---|---|---|---|---|---|
| Scientific name (Common name) | | GenBank Accession No. | W6 | W5 | W4 | W3 | W2 |
| Allergenic specific ingredient | *Fagopyrum_esculentum* (Shirahana buckwheat) | AB000330, AB000331 | - | - | - | - | - |
| | *Triticum aestivum* (Wheat) | Z11761, AJ301799 | - | - | - | - | - |
| | *Glycine max* (Soybean) | AJ011337, U60551 | - | - | - | - | - |
| | *Juglans regia* (Walnut) | AF303809, AF179581 | - | - | - | - | - |
| | *Tricholoma matsutake* (Matsutake mushroom) | U62964, AF385751 | - | - | - | - | - |
| | *Prunus persica* (Peach) | AF31874, AF143535 , AF179562 , AF185621 | - | - | - | - | - |
| | *Malus x domestica* (Apple) | AF186477 | - | - | 423bp, 466bp, 238bp | - | - |
| | *Malus x domestica* (Apple) | AF186478 | - | - | 238bp, 155bp | - | - |
| | *Malus x domestica* (Apple) | AF186479 | - | - | 238bp, 112bp, 155bp | - | - |
| | *Citrus sp.* (Valencia Orange) | E08821 | - | - | - | - | - |

[Table 7E]

| Primers for detecting peanut (SEQ ID NOs: 21 and 65): amplification product | | | | | | | |
|---|---|---|---|---|---|---|---|
| Scientific name (Common name) | | GenBank Accession No. | W6 | W5 | W4 | W3 | W2 |
| Principal food ingredient | *Zea mays* (Maize) | U46600 to U46648 | - | - | - | - | - |
| | *Oryza sativa* (Rice) | AF169230 | - | - | - | - | - |
| | *Piper nigrum* (Pepper) | AF275197, AF275198 | - | - | - | - | - |
| | *Sinapis alba* (White mustard) | X15915, AF128106 | - | - | - | - | - |

| | | | | | | | |
|---|---|---|---|---|---|---|---|
| | *Brassica nigra* (Black mustard) | AF128102, AF128103 | - | - | - | - | - |
| | *Brassica juncea* (Chinese mustard) | AF128093 | - | - | - | - | - |
| | *Brassica rapa subsp. rapa* (Turnip) | AF128097 | - | - | - | - | - |
| | *Brassica chinensis* (Chinese cabbage) | AF128098 | - | - | - | - | - |
| | *Lentinula edodes* (Shiitake mushroom) | AF079572 | - | - | - | - | - |
| | *Daucus carota* (Carrot) | X17534 | - | - | - | - | - |
| Standard | *Limonium sinuatum* (Statice) | AJ222860 | - | - | - | - | - |

As shown in Tables 7A to 7C, it was expected from the result of the simulation that a PCR amplification product having a target size of 76 bp was obtained from the 11 sequences of plants belonging to the genus *Arachis* when the combination of the primers of SEQ ID NOs: 21 and 65 was used. In addition, it was expected that a PCR amplification product having the target size and a non-specific PCR amplification product were not obtained from the 7 sequences of likely-to-be-allergenic plants other than a peanut (buckwheat, wheat, soybean, walnut, matsutake mushroom, peach, and orange), the 8 sequences of plants frequently used as food ingredients (maize, rice, pepper, mustard, carrot, shiitake mushroom, Chinese cabbage, and turnip), the 6 sequences of plants of the family *Leguminosae* (kidney bean, lima bean, lentil, chickpea, mung bean, and adzuki bean), the 69 sequences of related plant species of a peanut, and the statice. Because there was expected the possibility that a non-specific PCR amplification product having a different size from the target size but having a weak signal was obtained from the apple, we decided to subject the apple to additional confirmation by actual PCR. The PCR simulation gave results from which a PCR amplification product having the target size was also expected to be obtained from the sequences of plants belonging to the genus *Arachis* in both cases where the combination of the primers having the sequences shown in SEQ ID NOs: 21 and 66 was used and where the combination of the primers of SEQ ID NOs: 21 and 26 was used.

(3) Peanut PCR:

Peanut PCR was conducted using HotStarTaq Master Mix Kit manufactured by QIAGEN according to procedures below.

Primers (0.2 μM each at a final concentration) and template DNA were added to 12.5 μl of 2 × HotStartTaq Master Mix (HotStar Taq DNA Polymerase, PCR buffer with 3mM $MgCl_2$, and 400μM each dNTP), whose final volume was adjusted with sterilized ultrapure water to 25 μl to make a reaction solution. This was in turn introduced in a 0.2-ml microtube and reacted using a thermal cycler GeneAmp PCR System 9600 manufactured by Applied Biosystems according to the following PCR steps: enzyme activation at 95°C for 15 minutes; 45 cycles of denaturation at 95°C for 30 seconds, and annealing and extension at 68°C for 30 seconds; and final extension at 72°C for 4 minutes. The resulting PCR reaction solution was subjected to ethidium bromide-containing 2% agarose gel electrophoresis and analyzed with a fluorescent image analyzer FluorImager 595 manufactured by Amersham Biosciences. The results in the case of using the combination of the primers SEQ ID NOs: 21 and 65 are shown as a representative in Fig. 12. Abbreviated letters and symbols in Fig. 12 are as shown below:

M: 100-bp DNA Ladder Marker
(-): No addition of template DNA
Numeric: Amount of template DNA added
Arrow: Target band (approximately 76 bp) of PCR amplification product

The extracted plant DNA was confirmed to have a purity level capable of PCR amplification by obtaining a PCR amplification product with primers for amplifying a portion of plant chloroplast DNA or a Rubisco gene sequence (data not shown).

(4) Sensitivity and specificity of peanut PCR:

As a result of peanut PCR, a PCR amplification product having a size of approximately 76 bp expected from the target ITS-1 sequence of a peanut was obtained from 500 fg of the peanut DNA, as shown in Fig. 12. Sensitivity that allows the detection of 500 fg of the peanut DNA corresponds to a sensitivity level at which, when PCR is conducted with 50 ng of DNA extracted from a certain sample as a template, 10 ppm of buckwheat DNA contained in the sample DNA can be detected.

As a result of peanut PCR, a PCR amplification product having the target size and a non-specific PCR amplification product were not obtained from 50 ng each of the DNAs of the apple seed, wheat leaf, buckwheat leaf, adzuki bean leaf, soybean leaf, maize leaf, and statice seed, as shown in Fig. 12. Although the PCR simulation had expected the possibility that a non-specific PCR amplification product having a different size from the target size but having a weak signal was obtained from the apple, it could be confirmed that this problem did not arise. Similarly, it was also confirmed that a PCR amplification product was not obtained from the DNAs of the almond seed, hazelnut seed, macadamia nut seed, walnut seed, poppy seed, pine nut, sunflower seed, sesame, and salmon sperm (data not shown). In addition, from the obtained result, the peanut PCR was found to have similar sensitivity and specificity in both cases where the combination of the primers of SEQ ID NOs: 21 and 66 was used and where the combination of the primers of SEQ ID NOs: 21 and 26 was used (data not shown).

(5) Nucleotide sequence analysis of peanut PCR amplification product:

The nucleotide sequence of the peanut DNA-derived PCR amplification product obtained using the combination of the primers of SEQ ID NOs: 21 and 65 was analyzed by double-strand direct sequencing using primers of SEQ ID NOs: 21 and 65. The obtained nucleotide sequence was compared with the nucleotide sequence of a commercially-available peanut, *Arachis hypogaea,* to confirm that the nucleotide sequence of the peanut DNA-derived PCR amplification product matched 100% to the target site of the nucleotide sequence of the commercially-available peanut *(Arachis hypogaea)* (data not shown). This demonstrated that PCR using the primers amplified and detected a portion of the ITS-1 sequence of a peanut. In addition, from the obtained result, the PCR was found to amplify and detect a portion of the ITS-1 sequence of a peanut in both cases where the combination of the primers of SEQ ID NOs: 21 and 66 was used and where the combination of the primers of SEQ ID NOs: 21 and 26 was used (data not shown).

These results showed that peanut PCR using the primers can detect, with high sensitivity and specificity, the ITS-1 sequences of the general plants belonging to the genus *Arachis.* We decided to use these primers in PCR that quantified the copy number of the ITS-1 sequence of a peanut (hereinafter, referred to as a quantitative PCR method for a peanut sequence).

D. PCR that quantifies copy number of peanut sequence

(1) TaqMan MGB probe for detecting peanut sequence:

A TaqMan MGB probe (manufactured by Applied Biosystems Japan, reporter dye FAM) having a sequence below was synthesized and used as a probe for detecting a peanut sequence. A sequence universal to 11 sequences registered in GenBank as the ITS-1 sequences of plants belonging to the genus *Arachis* and the sequence obtained from the analysis of the commercially-available peanut was employed as the probe sequence.

5'-TGC TCT CCC CGC CGG C-3' (SEQ ID NO: 34)

(2) Quantitative PCR method for peanut sequence:

A Quantitative PCR method for a peanut sequence was conducted using QuantiTect Probe PCR Kit manufactured by QIAGEN according to procedures below.

Primers (0.2 μM each at a final concentration), the TaqMan MGB probe of SEQ ID NO: 34 (0.1 μM at a final concentration), and template DNA were added to 12.5 μl of 2 × QuantiTect Probe PCR Master Mix. The final volume was adjusted with sterilized ultrapure water to 25 μl to make a solution, which was in turn dispensed into a 96-well PCR plate. The 96-well PCR plate into which the solution was dispensed was loaded in a real-time PCR device Sequence Detection System 7700 manufactured by Applied Biosystems, in which the solution was reacted according to the following PCR steps: at 95°C for 15 minutes; 45 cycles of denaturation at 95°C for 30 seconds, and annealing and extension at 68°C for 30 seconds; and final extension at 72°C for 4 minutes. Every reaction was conducted with the same samples in duplicate (in 2 wells). After the completion of reaction, fluorescence data taken during the extension step was analyzed. A baseline was first set to cycles 0 to 1 and then appropriately set to within a range before a cycle where the increase of fluorescence was confirmed to begin. A threshold line was set according to the method described in Kuribara H et al., 2002, Novel Reference Molecules for Quantitation of Genetically Modified Maize and Soybean, Journal of AOAC International 85: 1077-1089. The results in the case of using the combination of the primers of SEQ ID NOs: 21 and 65 are shown as a representative in Figs.13, 14, and 15.

The extracted plant DNA was confirmed to have a purity level capable of PCR amplification by obtaining a PCR amplification product with primers for amplifying a portion of plant chloroplast DNA or Rubisco gene sequence (data not shown).

(3) Specificity of quantitative PCR method for peanut sequence:

As a result of the quantitative PCR method for the peanut sequence, a fluorescent signal indicating amplification was found from the DNA of the peanut seed, as shown in Fig. 13. On the other hand, a fluorescent signal indicating amplification was not observed in 50 ng each from the DNAs of the apple seed, wheat leaf, buckwheat leaf, adzuki bean leaf, soybean leaf, maize leaf, and statice seed. Similarly, a fluorescent signal indicating amplification was not observed in the DNAs of the almond seed, hazelnut seed, macadamia nut seed, walnut seed, poppy seed, pine nut, sunflower seed, sesame, apple, and salmon sperm (data not shown). In addition, from the obtained result, the quantitative PCR method was found to have similar specificity in both cases where the combination of the primers of SEQ ID NOs: 21 and 66 was used and where the combination of the primers of SEQ ID NOs: 21 and 26 was used (data not shown).

(4) Quantitative property and sensitivity of quantitative PCR method for peanut sequence:

As a result of the quantitative PCR method for the peanut sequence, a quantitative property and sensitivity where a standard curve having a correlation coefficient of 0.996 and a slope of-3.911 could be drawn with the peanut DNA in an amount ranging from 50 ng to 500 fg could be confirmed, as shown in Figs. 14 and 15. Sensitivity that attained a fluorescent signal indicating amplification could also be found in 50 fg of the peanut DNA. In addition, from the obtained result, the quantitative PCR method was found to have similar quantitative property and sensitivity in both cases where the combination of the primers of SEQ ID NOs: 21 and 66 was used and where the combination of the primers of SEQ ID NOs: 21 and 26 was used (data not shown).

These results demonstrated that the quantitative PCR methods for the peanut sequence using the primers of SEQ ID NOs: 21 and 65 together with the probe of SEQ ID NO: 34, the quantitative PCR methods for the peanut sequence using the primers of SEQ ID NOs: 21 and 66 together with the probe of SEQ ID NO: 34, and the quantitative PCR methods for the peanut sequence using the primers of SEQ ID NOs: 21 and 26 together with the probe of SEQ ID NO: 34 could detect, with high sensitivity and specificity, the ITS-1 sequences of the general plants belonging to the genus *Arachis* and quantify the copy number of the peanut sequence as long as the plasmid for standard curves containing the target sequence of a peanut and the standard curves were generated. The present quantitative PCR method for the peanut sequence can be used in combination with the quantitative PCR method for the statice sequence for correction in the measurement of the amount of a contaminating peanut.

Example 4: Confirmation of quantitative property of quantitative PCR method in processed sample:

Dough (having a diameter of 6 cm and a thickness of 1 mm) prepared by adding 35 g of water and 0.8 g of a salt to 80 g of wheat containing 100 ppm (hereinafter, W/W) of buckwheat was subjected to any of the following four heat treatments: (1) baking (160°C, 10 min), (2) frying (185°C, 5 sec), (3) steaming (100°C, 10 min), and boiling (100°C, 10 min), and used as a processed product model that was cooked. They were then mixed with a statice standard sample, followed by DNA extraction in the same way as above. Buckwheat contained in the heated sample was quantified using a primer set consisting of oligonucleotide having a sequence shown in SEQ ID NO: 14 and oligonucleotide having a sequence shown in SEQ ID NO: 15, in combination with a probe having a sequence shown in SEQ ID NO: 64. Based on the measured quantitative value of buckwheat in the processed product, a buckwheat concentration in the wheat used was determined, when water contents were taken into consideration. As a result, the buckwheat concentration was 145 ppm for (1) the baked product, 56 ppm for (2) the fried product, 198 ppm for (3) the steamed product, and 143 ppm for (4) the boiled product, and a sufficient quantitative property was shown. Thus, it was suggested that quantification by the method of the present invention could be performed in all of the most general heat treatments, baking, frying, steaming, and boiling, in food processing. Therefore, it is considered that the method of the present invention can mainttain this quantitative property for the processed food by processing other than the above-described processing. Thus, the method of the present invention is applicable to a wide range of processed foods.

All publications, patents, and patent applications cited herein are incorporated herein by reference in their entirety.

Industrial Applicability

A PCR method of the present invention that quantifies a plant belonging to a specific plant genus that contaminates a food or a food ingredient can detect and quantify the presence of a very small amount of the plant belonging to the specific plant genus in the food or the food ingredient and as such, is especially effective in the detection of the presence or absence of a plant belonging to an allergenic plant genus such as the genus *Fagopyrum,* the genus *Arachis,* the genus *Triticum,* and the genus *Glycine,* and in the quantification of the plant. The PCR method of the present invention is a method in which correction for influences such as the DNA extraction efficiency of each sample to be examined and the inhibition of PCR reaction is conducted not by externally adding purified DNA as a standard to conduct correction for influences such as the inhibition of PCR reaction in a reaction solution but by simultaneously extracting DNA derived form a specific plant genus to be detected and DNA derived from a standard plant from a sample externally supplemented with a standard plant sample other than purified DNA to conduct a quantitative PCR method. This method allows highly reliable quantification because of being capable of measurement under a condition where influences such as DNA extraction efficiency and the inhibition of PCR reaction are uniform between the standard plant sample and the sample derived from the specific plant genus to be detected. The method of the present invention has an advantage that the method is capable of correction for influences such as DNA extraction efficiency and the inhibition of PCR reaction and even for difference in DNA content among samples to be examined. This method also allows the proper quantitative detection of a plant belonging to a specific plant genus in a DNA-free food ingredient such as salts or a food containing the ingredient.

Thus, the present invention is useful for quantitatively detecting a plant belonging to an allergenic specific plant genus that contaminates a food or a food ingredient. In addition, quantitative analysis by the PCR method can reliably exclude a false positive, if any, by subjecting its PCR amplification product to DNA sequence analysis, and as such, can be said to have excellent industrial applicability.

The quantitative PCR method of the present invention can have a dynamic range wider than those of ELISA methods and can achieve sufficiently high specificity and sensitivity for quantitatively detecting a specific ingredient contaminating a food or a food ingredient. Moreover, the method used in combination with synthesized materials (primer and probe) can attain the high reproducibility and reliability of a measurement result.

## Claims

1. A method of quantifying a plant belonging to a specific plant genus in a food or a food ingredient by a PCR method, comprising:

preparing a sample for correction where a sample derived from the specific plant genus to be detected and a standard plant sample are mixed in a predetermined ratio, and extracting genomic DNA from the sample for correction;

preparing a test sample where a known amount of the standard plant sample is added to the food or the food ingredient to be examined, and extracting genomic DNA from the test sample;

practicing a quantitative PCR using a primer set for detecting the sample derived from the specific plant genus to be detected and a primer set for detecting the standard plant sample with the genomic DNA extracted from each of the sample for correction and the test sample as a template;

determining, as a standard value for correction, a value of the copy number of the DNA derived from the standard plant/the copy number of the DNA derived from the specific plant genus for the sample for correction by the quantitative PCR method; and

determining a value of the copy number of the DNA derived from the specific plant genus/the copy number of the DNA derived from the standard plant for the test sample by the quantitative PCR method, and correcting the value with the standard value for correction to calculate the amount of the plant belonging to the specific plant genus contained in the food or the food ingredient.

**2.** The method according to claim 1, wherein the quantitative PCR method is a real-time PCR method.

**3.** The method according to claim 2, **characterized in that** the real-time PCR method quantifies DNA based on the amount of emitted light by use of a probe with a fluorescent dye at the 5' end and a quencher at the 3' end that hybridizes to an internal region of a genomic DNA site, which is hybridized with each oligonucleotide of a PCR primer set, wherein light emitted from the fluorescent dye at the 5' end of the probe is suppressed by the quencher at the 3' end, while during Taq polymerase-catalyzed DNA extension from the primer in PCR reaction, the probe is degraded by the 5'→3' exonuclease activity of the Taq polymerase to dissociate the fluorescent dye and the quencher, then causing light emission.

**4.** The method according to any one of claims 1 to 3, wherein the standard plant belongs to a plant species other than upland weeds and food crops.

**5.** The method according to claim 4, wherein the standard plant is statice.

**6.** The method according to any one of claims 1 to 5, wherein the specific plant genus to be detected is the genus *Fagopyrum, Arachis, Triticum,* or *Glycine.*

**7.** The method according to claim 2 or 3, wherein the standard plant is statice, a primer set for detecting the statice is a set consisting of oligonucleotide having a sequence shown in SEQ ID NO: 57 and oligonucleotide having a sequence shown in SEQ ID NO: 58, and a probe for detecting the statice is oligonucleotide having a sequence shown in SEQ ID NO: 59.

**8.** The method according to claim 2 or 3, wherein the specific plant genus to be detected is the genus *Fagopyrum,* a primer set for detecting the genus *Fagopyrum* is a set consisting of oligonucleotide having a sequence shown in SEQ ID NO: 14 and oligonucleotide having a sequence shown in SEQ ID NO: 15, and a probe for detecting the genus *Fagopyrum* is oligonucleotide having a sequence shown in SEQ ID NO: 64.

**9.** The method according to claim 2 or 3, wherein the specific plant genus to be detected is the genus *Arachis,* a primer set for detecting the genus *Arachis* is a primer set consisting of oligonucleotide having a sequence shown in SEQ ID NO: 21 and oligonucleotide having a sequence shown in SEQ ID NO: 26, 65, or 66, and a probe for detecting the genus *Arachis* is oligonucleotide having a sequence shown in SEQ ID NO: 34.

**10.** A primer set for detecting statice consisting of oligonucleotide having a sequence shown in SEQ ID NO: 57 and oligonucleotide having a sequence shown in SEQ ID NO: 58.

**11.** A primer set for detecting the genus *Fagopyrum* consisting of oligonucleotide having a sequence shown in SEQ ID NO: 14 and oligonucleotide having a sequence shown in SEQ ID NO: 15.

**12.** A primer set for detecting the genus *Arachis* consisting of oligonucleotide having a sequence shown in SEQ ID NO:

21 and oligonucleotide having a sequence shown in SEQ ID NO: 26, 65, or 66.

**13.** A kit for use in a method of detecting a plant belonging to a specific plant genus in a food or a food ingredient, comprising a primer set for detecting a standard plant sample.

**14.** The kit according to claim 13, further comprising a probe for detecting the standard plant sample.

**15.** The kit according to claim 13 or 14, wherein the standard plant is statice, and a primer set for detecting the statice is a set consisting of oligonucleotide having a sequence shown in SEQ ID NO: 57 and oligonucleotide having a sequence shown in SEQ ID NO: 58.

**16.** The kit according to claim 15, further comprising a probe for detecting the statice having a sequence shown in SEQ ID NO: 59.

**17.** The kit according to any one of claims 13 to 16, further comprising a primer set for detecting the specific plant genus to be detected.

**18.** The kit according to any one of claims 13 to 16, wherein the specific plant genus to be detected is the genus *Fagopyrum,* and a primer set for detecting the genus *Fagopyrum* is a set consisting of oligonucleotide having a sequence shown in SEQ ID NO: 14 and oligonucleotide having a sequence shown in SEQ ID NO: 15.

**19.** The kit according to claim 18, further comprising a probe for detecting the genus *Fagopyrum* having a sequence shown in SEQ ID NO: 64.

**20.** The kit according to any one of claims 13 to 16, wherein the specific plant genus to be detected is the genus *Arachis,* and a primer set for detecting the genus *Arachis* is a set consisting of oligonucleotide having a sequence shown in SEQ ID NO: 21 and oligonucleotide having a sequence shown in SEQ ID NO: 26, 65, or 66.

**21.** The kit according to claim 20, further comprising a probe for detecting the genus *Arachis* having a sequence shown in SEQ ID NO: 34.

**22.** The kit according to claim 15, further comprising a statice sample as the standard plant sample.

**23.** The kit according to claim 13, wherein the standard plant is statice and the specific plant genus to be detected is the genus *Fagopyrum,* the kit further comprising a plasmid for standard curves for the statice and the genus *Fagopyrum* that comprises DNA having an amplification target sequence of the statice and DNA having an amplification target sequence of the genus *Fagopyrum* with the DNAs ligated together.

**24.** The kit according to claim 13, wherein the standard plant is statice and the specific plant genus to be detected is the genus *Arachis,* the kit further comprising a plasmid for standard curves for the statice and the genus *Arachis* that comprises DNA having an amplification target sequence of the statice and DNA having an amplification target sequence of the genus *Arachis* with the DNAs ligated together.

**25.** A kit for use in a method of detecting a plant belonging to the genus *Fagopyrum* in a food or a food ingredient, comprising a primer set for detecting the genus *Fagopyrum* consisting of oligonucleotide having a sequence shown in SEQ ID NO: 14 and oligonucleotide having a sequence shown in SEQ ID NO: 15.

**26.** The kit according to claim 25, further comprising a probe for detecting the genus *Fagopyrum* having a sequence shown in SEQ ID NO: 64.

**27.** A kit for use in a method of detecting a plant belonging to the genus *Arachis* in a food or a food ingredient, comprising a primer set for detecting the genus *Arachis* consisting of oligonucleotide having a sequence shown in SEQ ID NO: 21 and oligonucleotide having a sequence shown in SEQ ID NO: 26, 65, or 66.

**28.** The kit according to claim 27, further comprising a probe for detecting the genus *Arachis* having a sequence shown in SEQ ID NO: 34.

FIG.1A

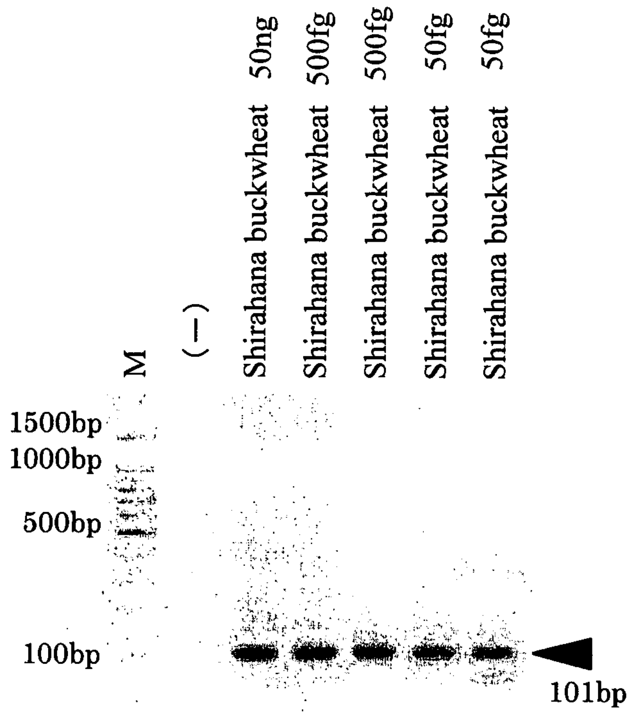
M
(−)
Shirahana buckwheat 50ng
Shirahana buckwheat 500fg
Shirahana buckwheat 500fg
Shirahana buckwheat 50fg
Shirahana buckwheat 50fg
1500bp
1000bp
500bp
100bp
101bp

FIG.1B

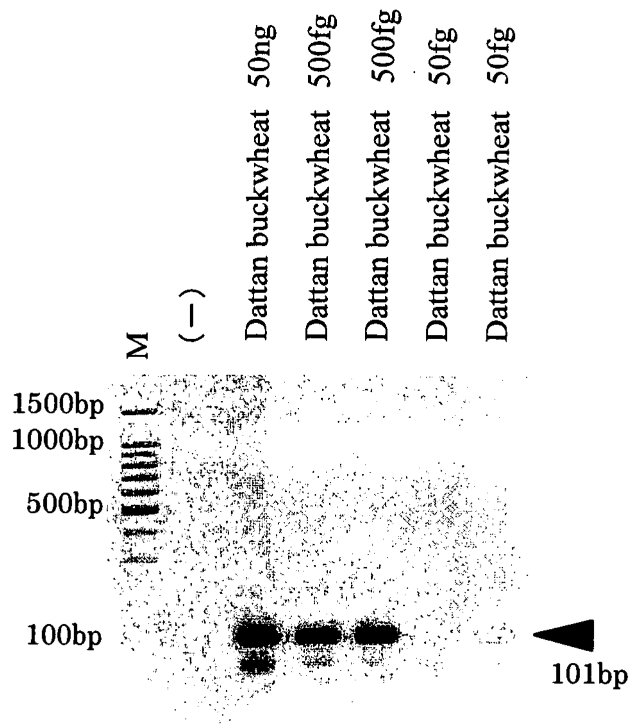
M
(−)
Dattan buckwheat 50ng
Dattan buckwheat 500fg
Dattan buckwheat 500fg
Dattan buckwheat 50fg
Dattan buckwheat 50fg
1500bp
1000bp
500bp
100bp
101bp

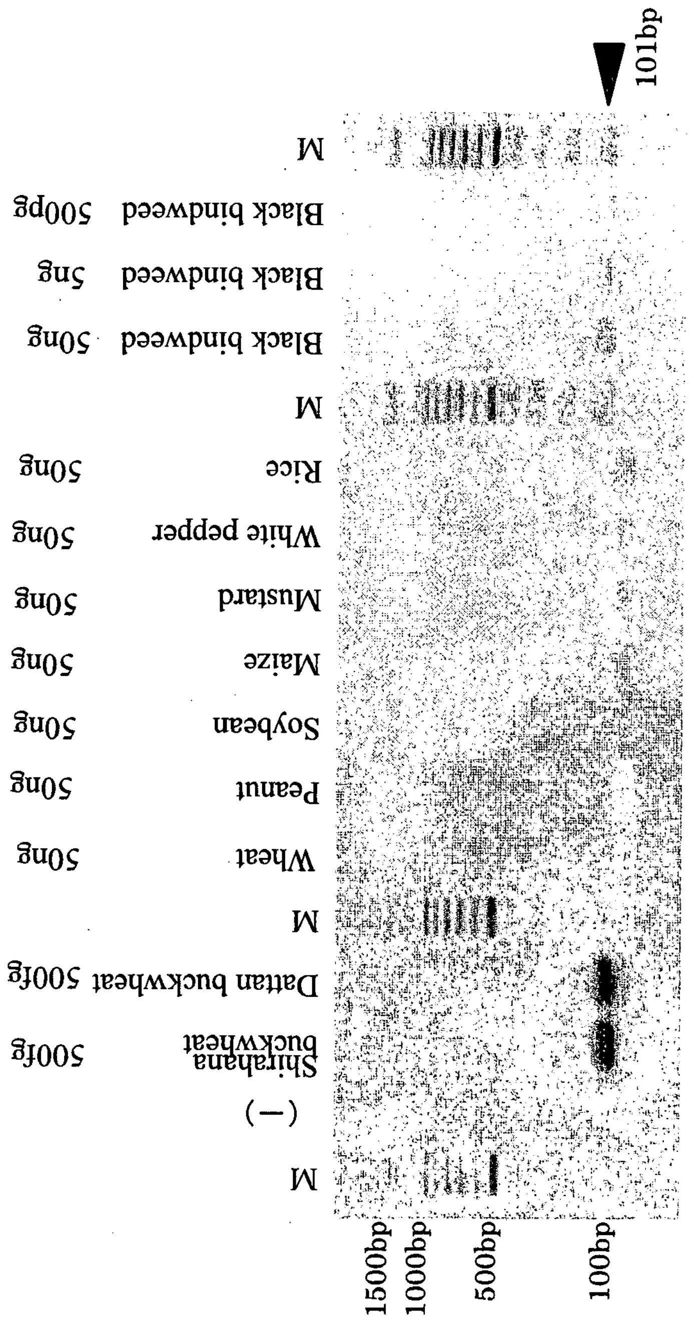

M
(−)
Shirahana buckwheat 500fg
Dattan buckwheat 500fg
M
Wheat 50ng
Peanut 50ng
Soybean 50ng
Maize 50ng
Mustard 50ng
White pepper 50ng
Rice 50ng
M
Black bindweed 50ng
Black bindweed 5ng
Black bindweed 500pg
M
101bp
1500bp
1000bp
500bp
100bp


FIG.2

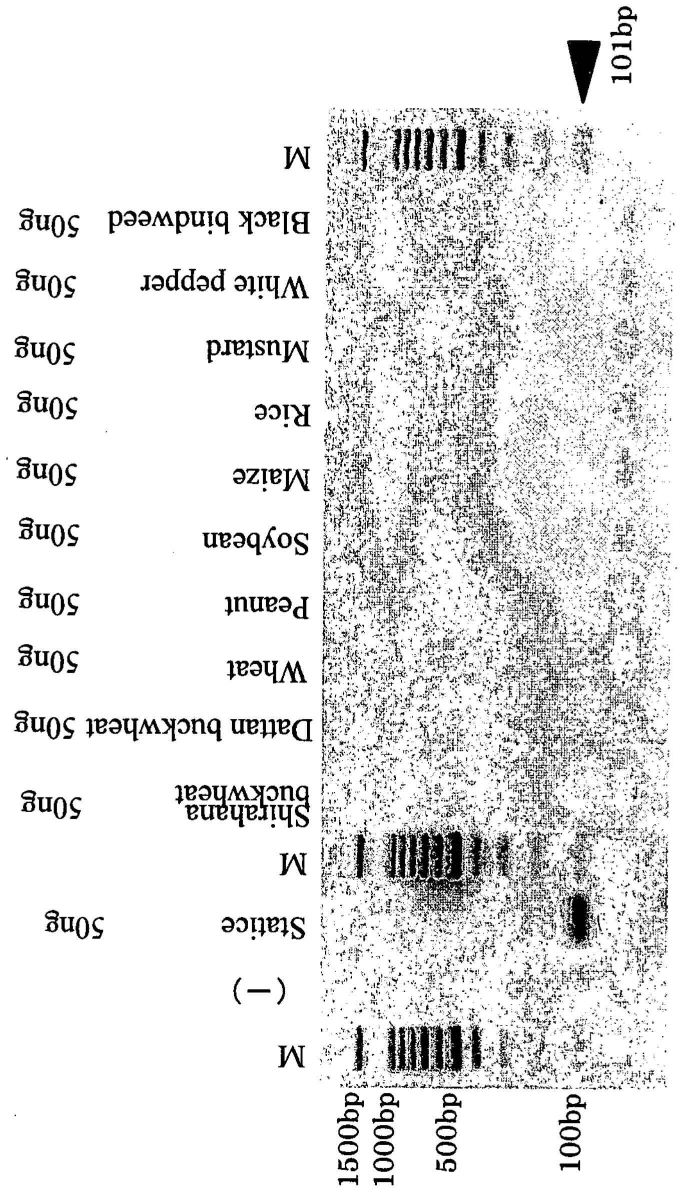
M
(−)
Statice 50ng
M
Shirahana buckwheat 50ng
Dattan buckwheat 50ng
Wheat 50ng
Peanut 50ng
Soybean 50ng
Maize 50ng
Rice 50ng
Mustard 50ng
White pepper 50ng
Black bindweed 50ng
M
1500bp
1000bp
500bp
100bp
101bp

FIG.3

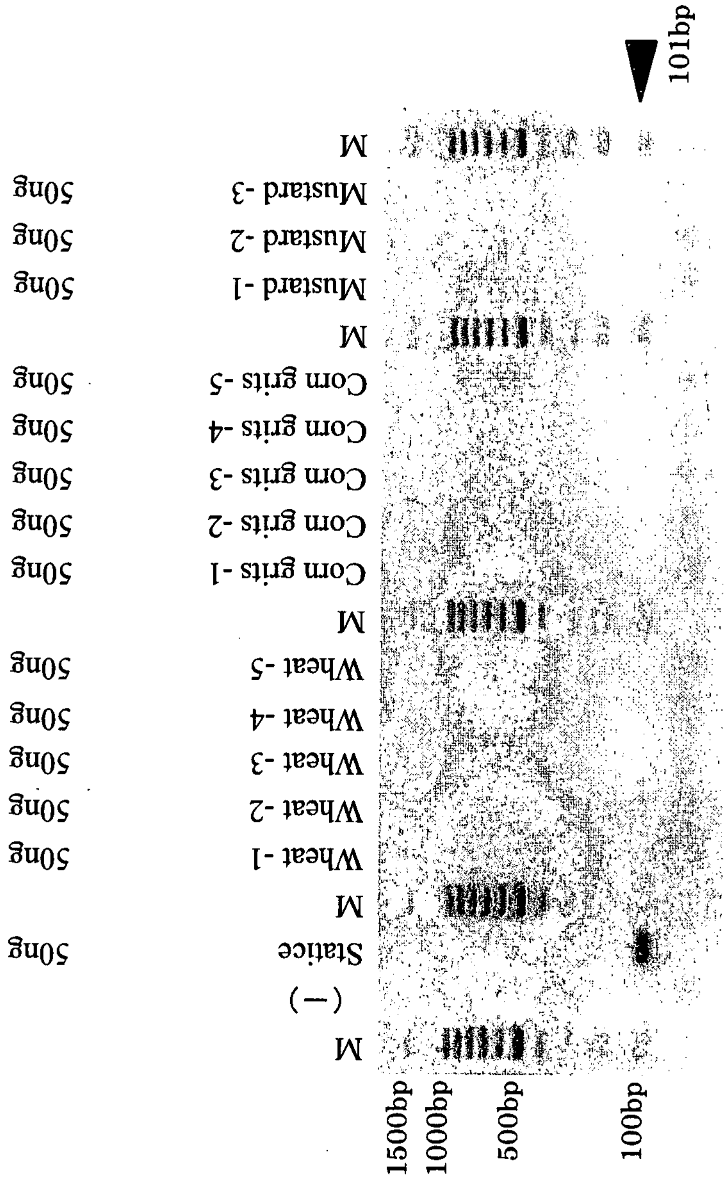
M
(−)
Statice 50ng
M
Wheat -1 50ng
Wheat -2 50ng
Wheat -3 50ng
Wheat -4 50ng
Wheat -5 50ng
M
Corn grits -1 50ng
Corn grits -2 50ng
Corn grits -3 50ng
Corn grits -4 50ng
Corn grits -5 50ng
M
Mustard -1 50ng
Mustard -2 50ng
Mustard -3 50ng
M
1500bp
1000bp
500bp
100bp
101bp

FIG.4

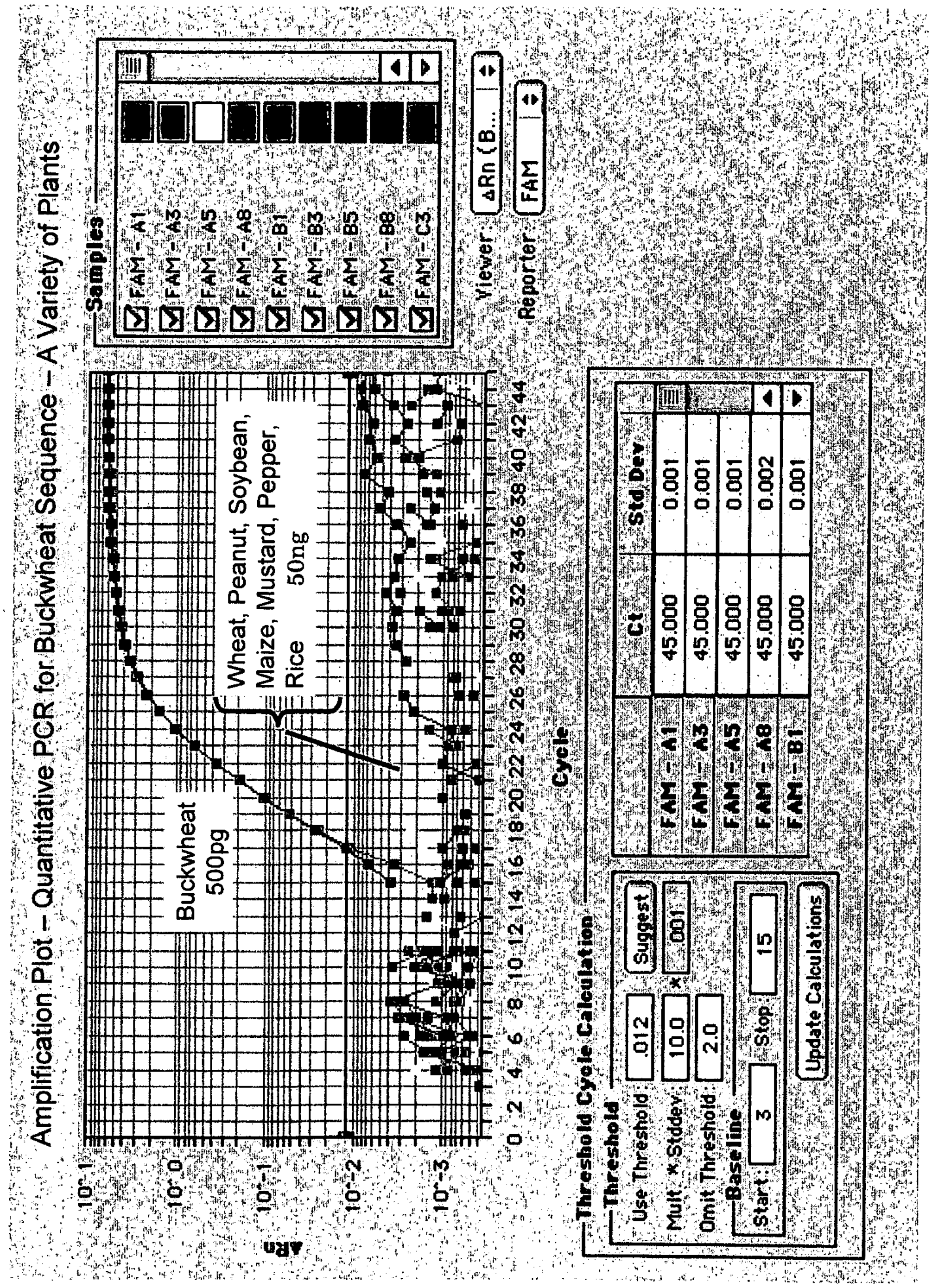
Amplification Plot – Quantitative PCR for Buckwheat Sequence – A Variety of Plants
Buckwheat 500pg
Wheat, Peanut, Soybean, Maize, Mustard, Pepper, Rice 50ng
ΔRn
Cycle
Samples
FAM - A1
FAM - A3
FAM - A5
FAM - A8
FAM - B1
FAM - B3
FAM - B5
FAM - B8
FAM - C3
Viewer ΔRn (B...
Reporter FAM
Threshold Cycle Calculation
Threshold
Use Threshold .012 Suggest
Mult. × Stddev 10.0 × .001
Omit Threshold 2.0
Baseline
Start: 3 Stop: 15
Update Calculations
Ct Std Dev
FAM - A1 45.000 0.001
FAM - A3 45.000 0.001
FAM - A5 45.000 0.001
FAM - A8 45.000 0.002
FAM - B1 45.000 0.001

FIG.5A

FIG.5B

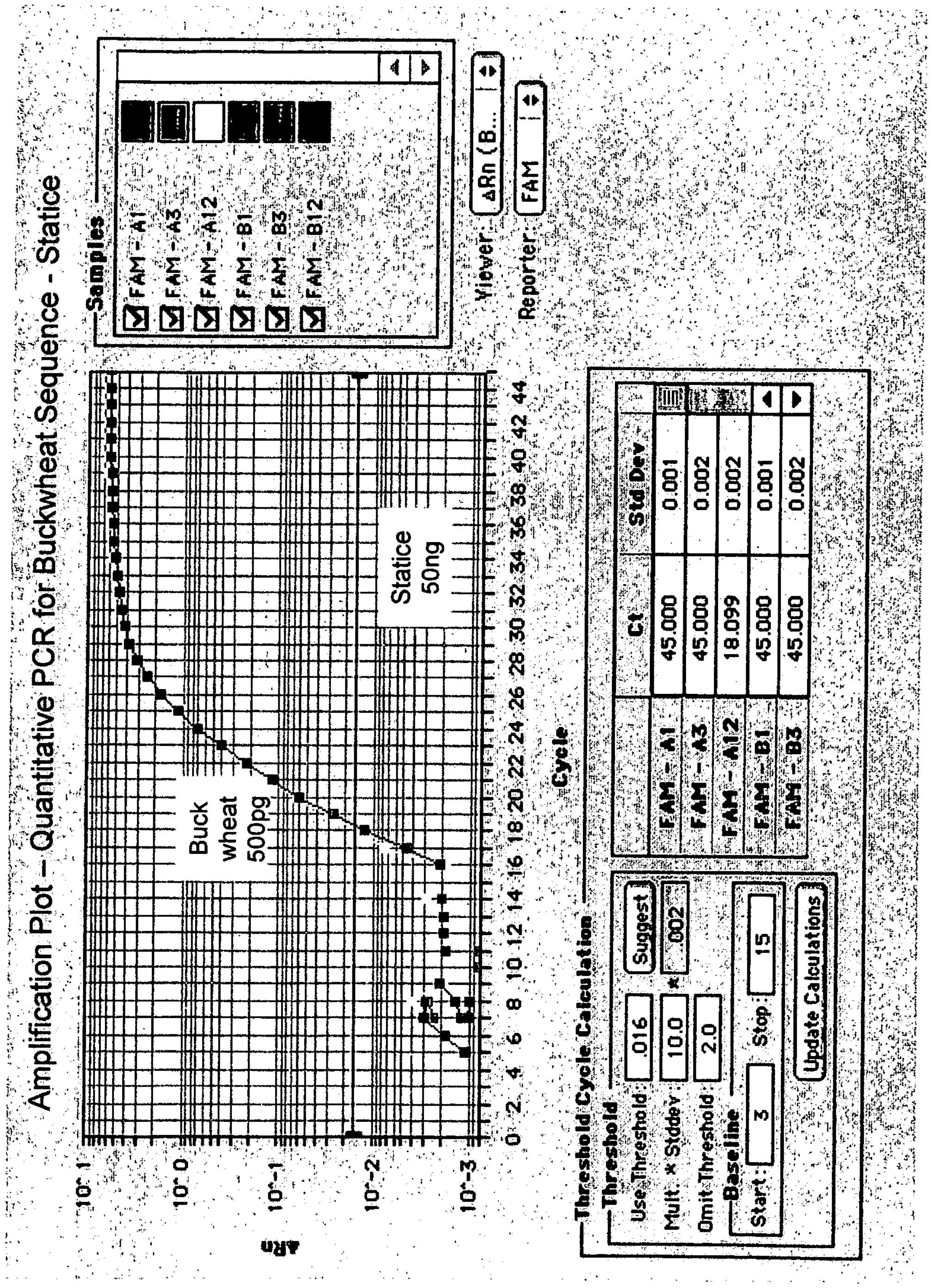
Amplification Plot – Quantitative PCR for Buckwheat Sequence - Statice
ΔRn
10^1
10^0
10^-1
10^-2
10^-3
0 2 4 6 8 10 12 14 16 18 20 22 24 26 28 30 32 34 36 38 40 42 44
Cycle
Buck wheat 500pg
Statice 50ng
Samples
FAM - A1
FAM - A3
FAM - A12
FAM - B1
FAM - B3
FAM - B12
Viewer: ΔRn (B...
Reporter: FAM
Threshold Cycle Calculation
Threshold
Use Threshold: .016
Suggest
Mult. * Stddev: 10.0 * .002
Omit Threshold: 2.0
Baseline
Start: 3
Stop: 15
Update Calculations
Ct
Std Dev
FAM - A1 45.000 0.001
FAM - A3 45.000 0.002
FAM - A12 18.099 0.002
FAM - B1 45.000 0.001
FAM - B3 45.000 0.002

FIG.6

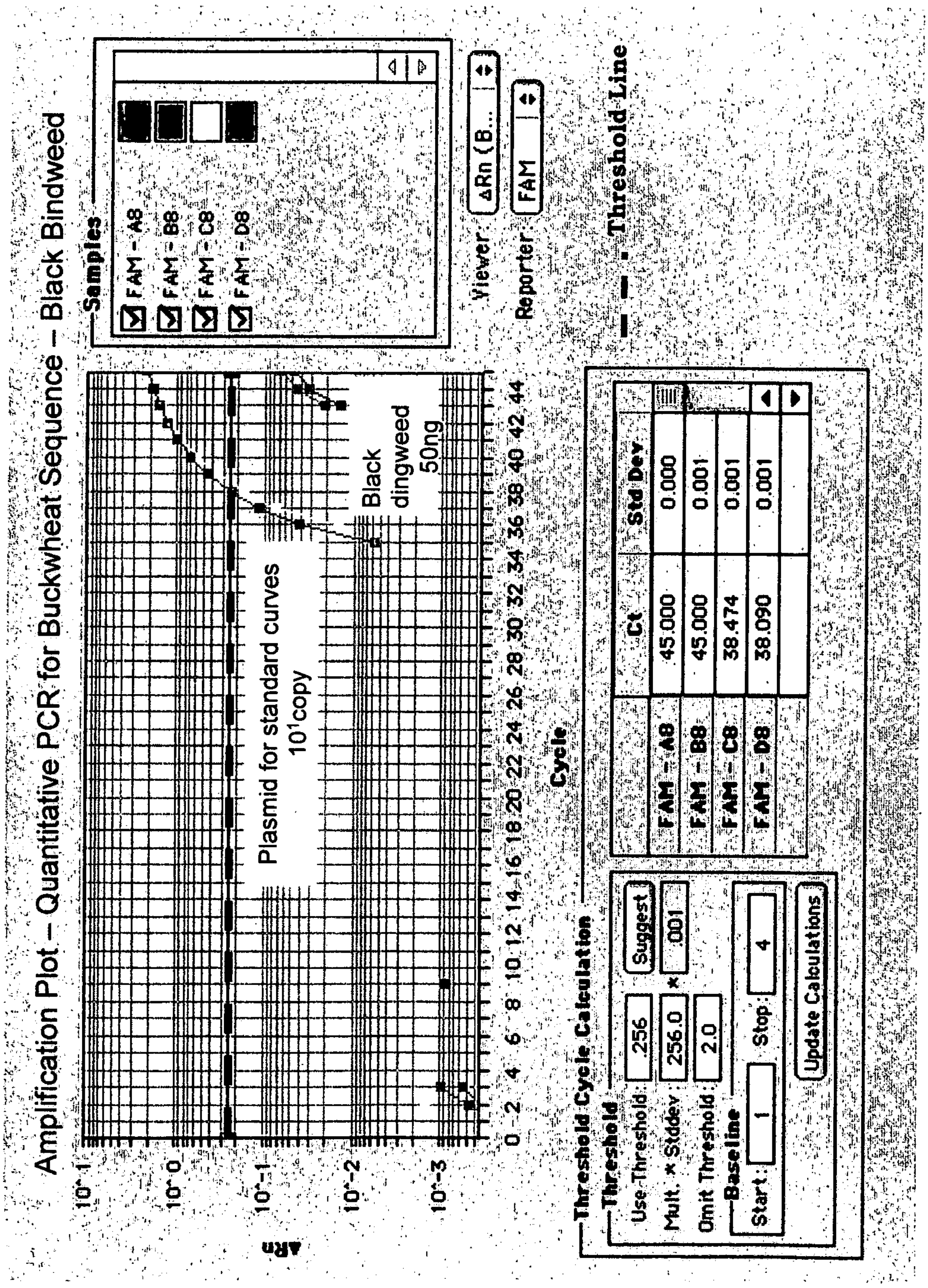
Amplification Plot – Quantitative PCR for Buckwheat Sequence – Black Bindweed
ΔRn
10^1
10^0
10^-1
10^-2
10^-3
0 2 4 6 8 10 12 14 16 18 20 22 24 26 28 30 32 34 36 38 40 42 44
Cycle
Plasmid for standard curves
10¹copy
Black
dingweed
50ng
Samples
FAM - A8
FAM - B8
FAM - C8
FAM - D8
Viewer
ΔRn (B...
Reporter
FAM
Threshold Line
Threshold Cycle Calculation
Threshold
Use Threshold: .256
Suggest
Mult. × Stddev: 256.0 × .001
Omit Threshold: 2.0
Baseline
Start: 1
Stop: 4
Update Calculations
Ct
Std Dev
FAM - A8 45.000 0.000
FAM - B8 45.000 0.001
FAM - C8 38.474 0.001
FAM - D8 38.090 0.001

FIG.7

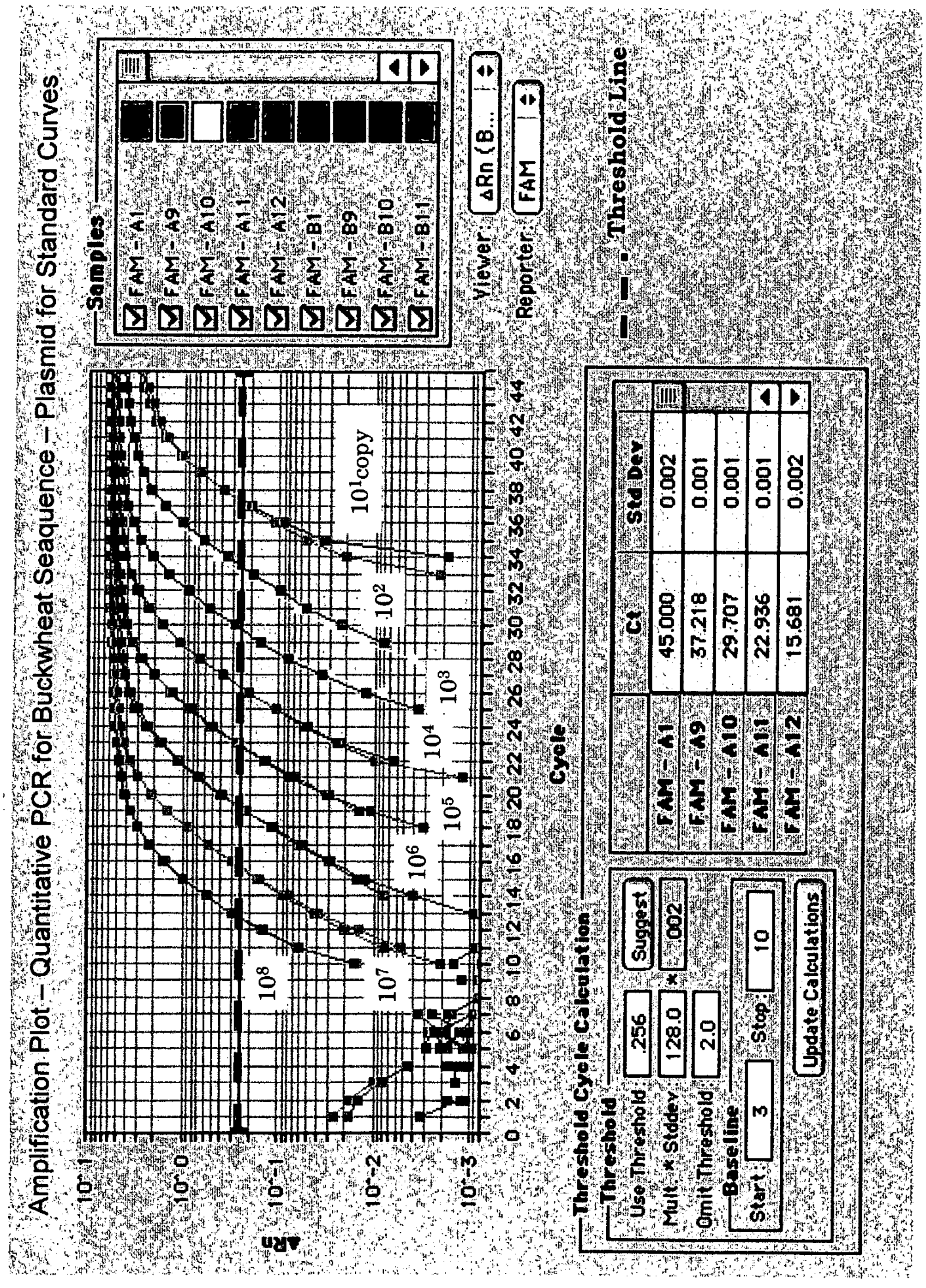

Amplification Plot – Quantitative PCR for Buckwheat Seaquence – Plasmid for Standard Curves
ΔRn
Cycle
10^8
10^7
10^6
10^5
10^4
10^3
10^2
10^1copy
Samples
FAM - A1
FAM - A9
FAM - A10
FAM - A11
FAM - A12
FAM - B1
FAM - B9
FAM - B10
FAM - B11
Viewer
ΔRn (B...
Reporter
FAM
Threshold Line
Threshold Cycle Calculation
Threshold
Use Threshold 256
Suggest
Mult. × Stddev 128.0 × .002
Omit Threshold 2.0
Baseline
Start 3
Stop 10
Update Calculations
Ct
Std Dev
FAM - A1 45.000 0.002
FAM - A9 37.218 0.001
FAM - A10 29.707 0.001
FAM - A11 22.936 0.001
FAM - A12 15.681 0.002

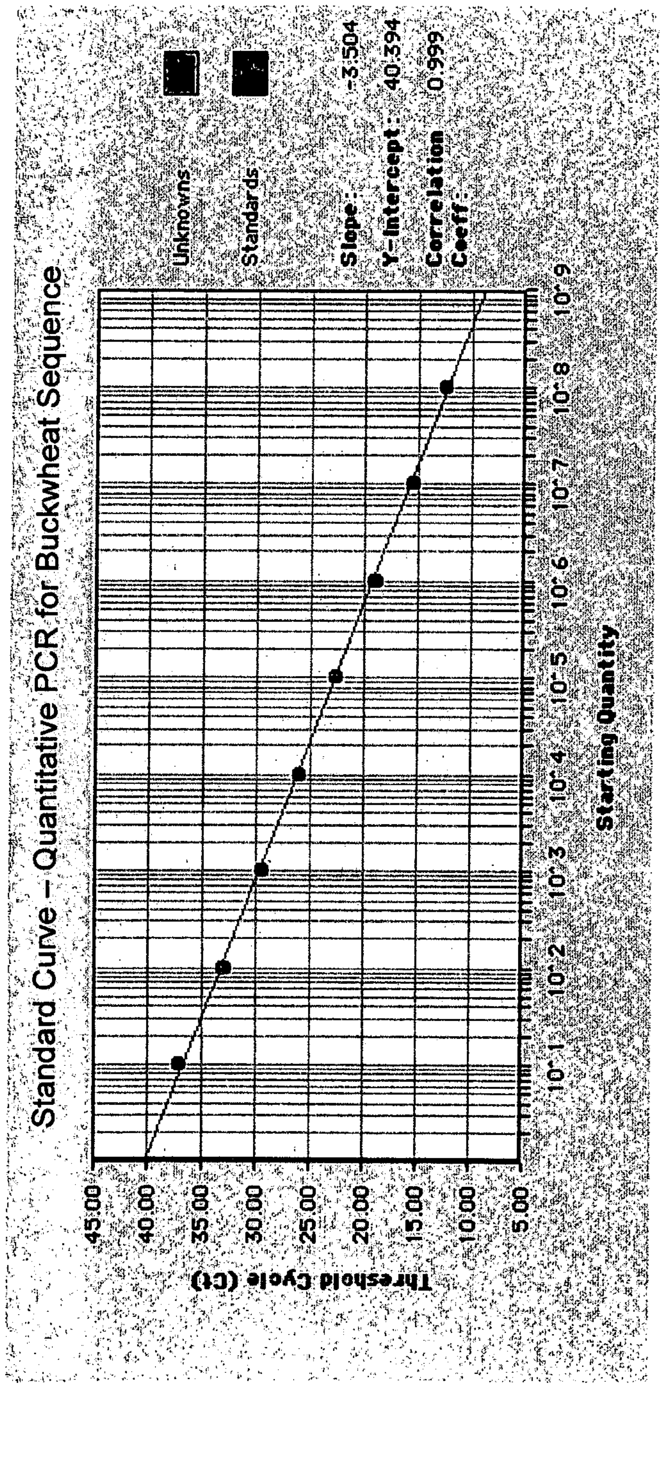
Standard Curve – Quantitative PCR for Buckwheat Sequence
Unknowns
Standards
Slope: -3.504
Y-Intercept: 40.394
Correlation Coeff: 0.999
Threshold Cycle (Ct)
45.00
40.00
35.00
30.00
25.00
20.00
15.00
10.00
5.00
10^1
10^2
10^3
10^4
10^5
10^6
10^7
10^8
10^9
Starting Quantity

FIG.8

FIG.9

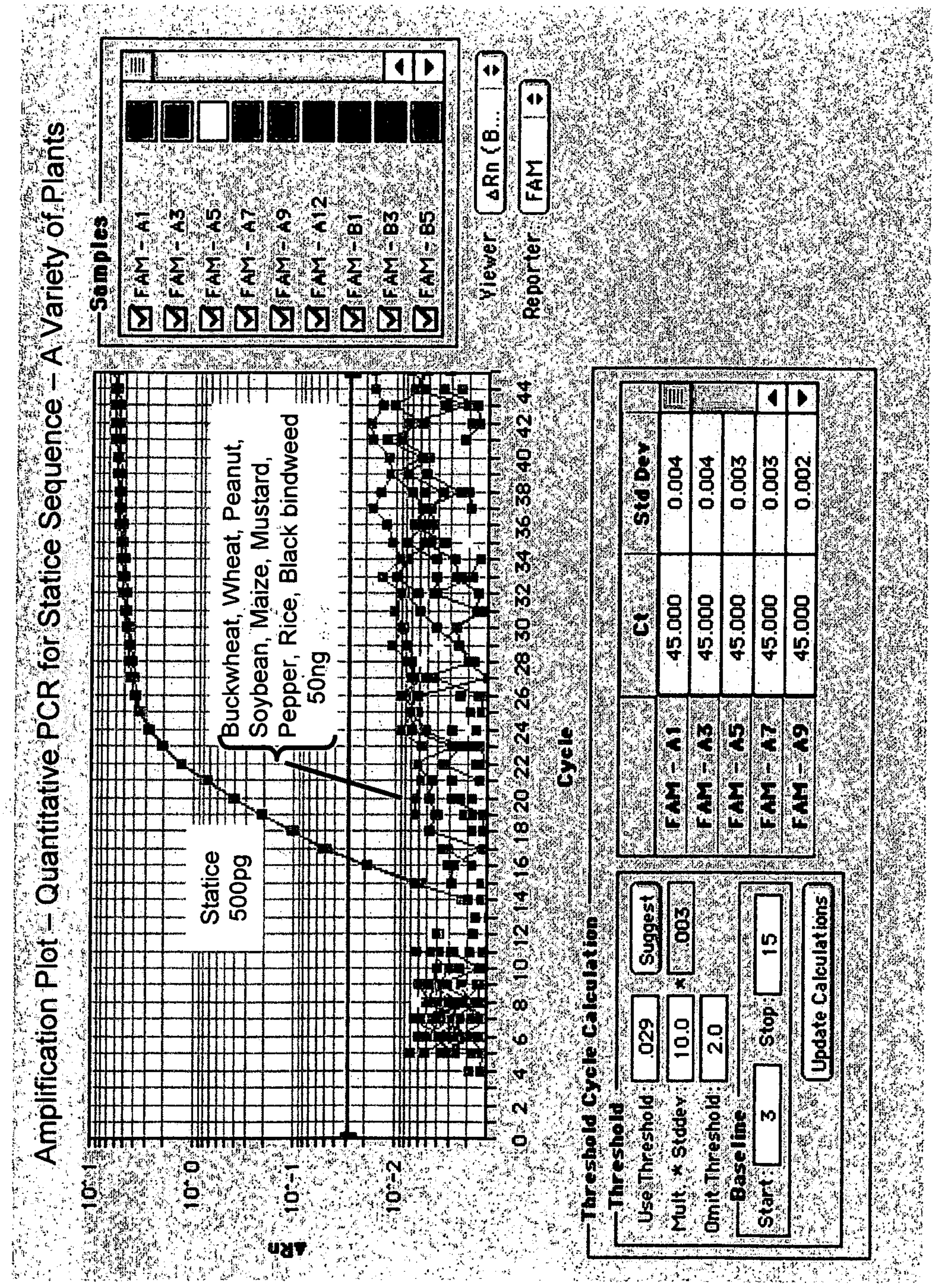
Amplification Plot – Quantitative PCR for Statice Sequence – A Variety of Plants
ΔRn
10^1
10^0
10^-1
10^-2
0 2 4 6 8 10 12 14 16 18 20 22 24 26 28 30 32 34 36 38 40 42 44
Cycle
Statice 500pg
Buckwheat, Wheat, Peanut, Soybean, Maize, Mustard, Pepper, Rice, Black bindweed 50ng
Samples
FAM - A1
FAM - A3
FAM - A5
FAM - A7
FAM - A9
FAM - A12
FAM - B1
FAM - B3
FAM - B5
Viewer
ΔRn (B...
Reporter
FAM
Threshold Cycle Calculation
Threshold
Use Threshold: .029
Suggest
Mult. * Stddev: 10.0 * .003
Omit Threshold: 2.0
Baseline
Start: 3
Stop: 15
Update Calculations
Ct
Std Dev
FAM - A1 45.000 0.004
FAM - A3 45.000 0.004
FAM - A5 45.000 0.003
FAM - A7 45.000 0.003
FAM - A9 45.000 0.002

FIG.10

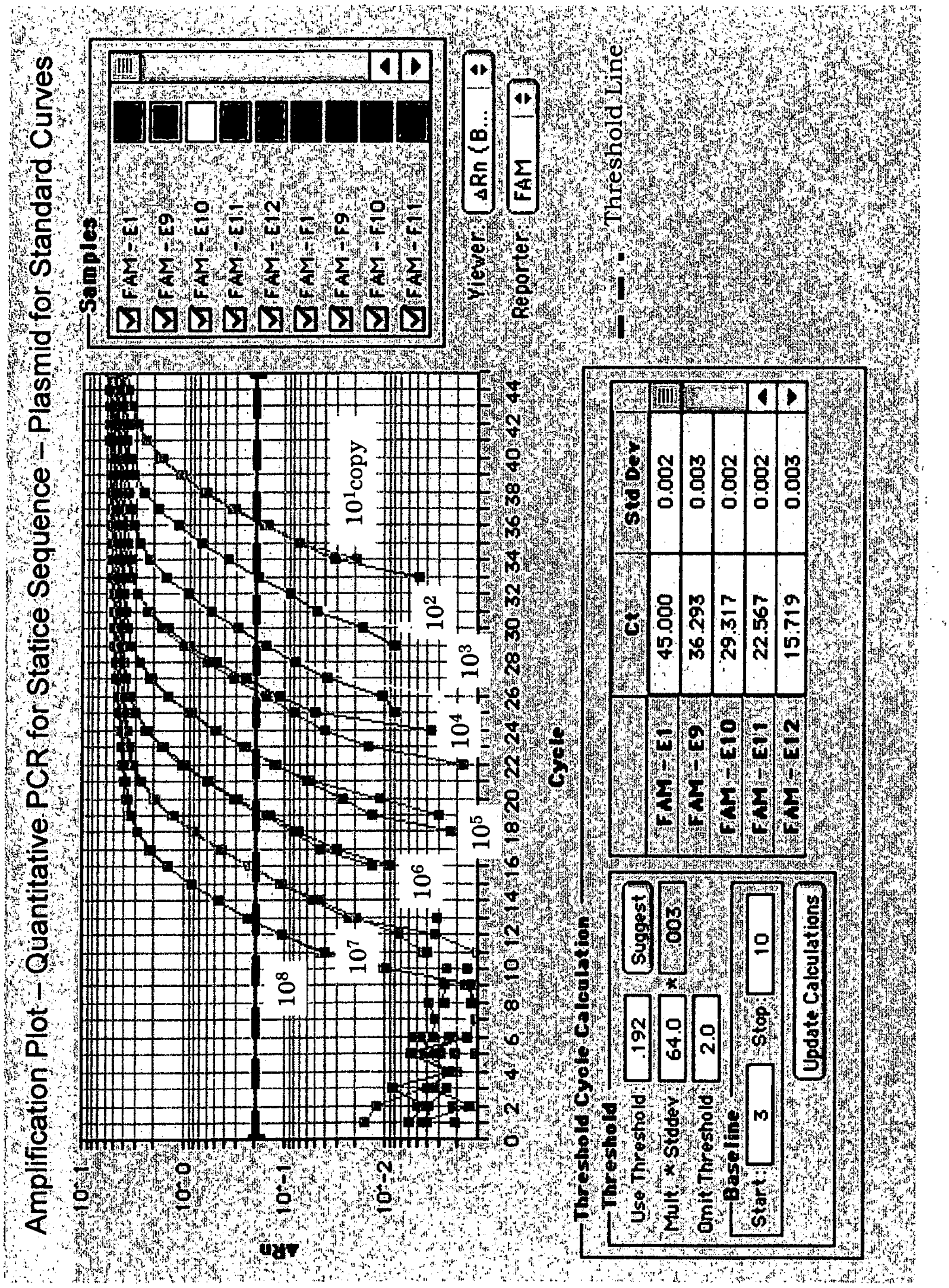
Amplification Plot – Quantitative PCR for Statice Sequence – Plasmid for Standard Curves
ΔRn
Cycle
$10^8$
$10^7$
$10^6$
$10^5$
$10^4$
$10^3$
$10^2$
$10^1$copy
Samples
FAM - E1
FAM - E9
FAM - E10
FAM - E11
FAM - E12
FAM - F1
FAM - F9
FAM - F10
FAM - F11
Viewer: ΔRn (B...
Reporter: FAM
Threshold Line
Threshold Cycle Calculation
Threshold
Use Threshold: .192
Suggest
Mult. * Stddev: 64.0 * .003
Omit Threshold: 2.0
Baseline
Start: 3
Stop: 10
Update Calculations
Ct
Std Dev
FAM - E1 45.000 0.002
FAM - E9 36.293 0.003
FAM - E10 29.317 0.002
FAM - E11 22.567 0.002
FAM - E12 15.719 0.003

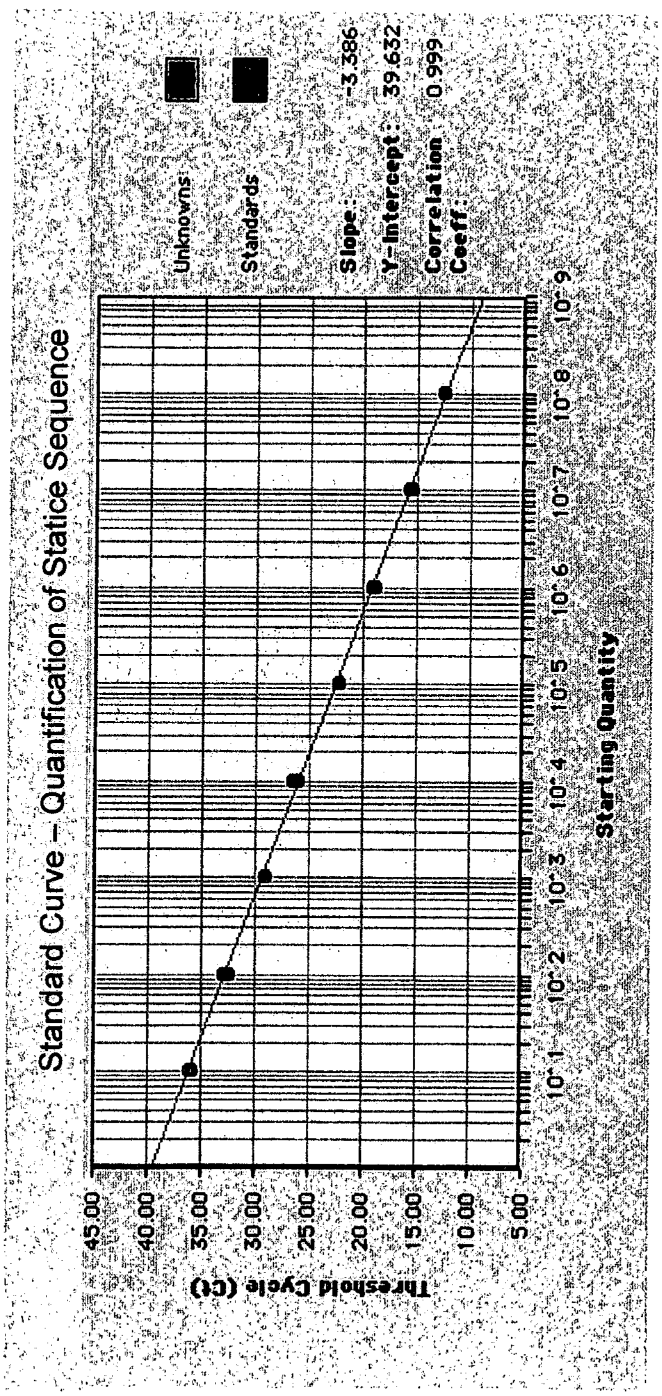
Standard Curve – Quantification of Statice Sequence
Threshold Cycle (Ct)
45.00
40.00
35.00
30.00
25.00
20.00
15.00
10.00
5.00
10^1
10^2
10^3
10^4
10^5
10^6
10^7
10^8
10^9
Starting Quantity
Unknowns
Standards
Slope: -3.386
Y-Intercept: 39.632
Correlation Coeff.: 0.999

FIG.11

FIG.12

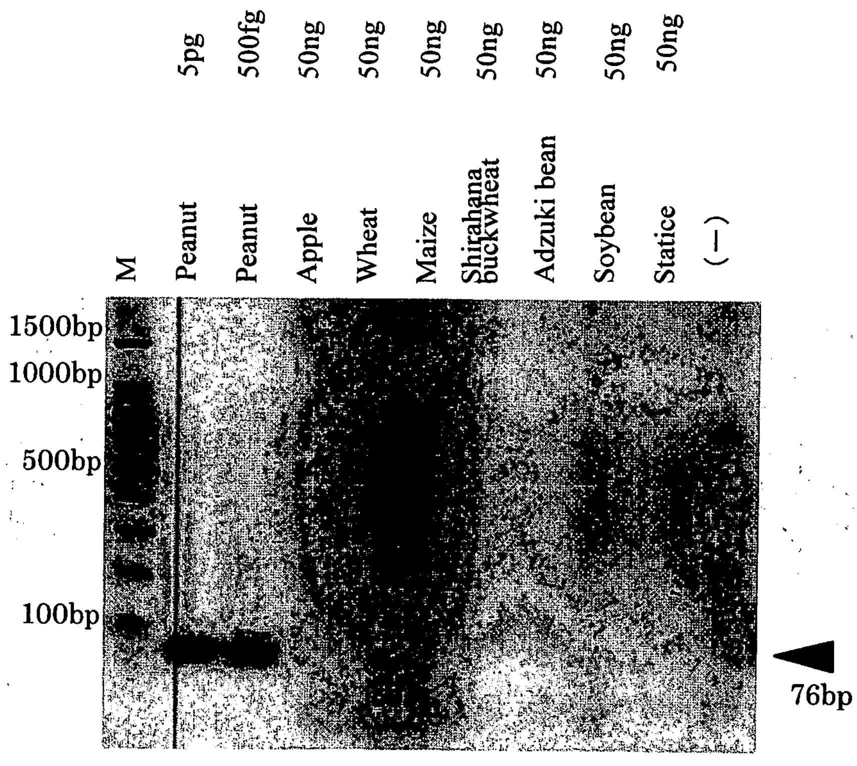
5pg
500fg
50ng
50ng
50ng
50ng
50ng
50ng
50ng
M
Peanut
Peanut
Apple
Wheat
Maize
Shirahana buckwheat
Adzuki bean
Soybean
Statice
(−)
1500bp
1000bp
500bp
100bp
76bp

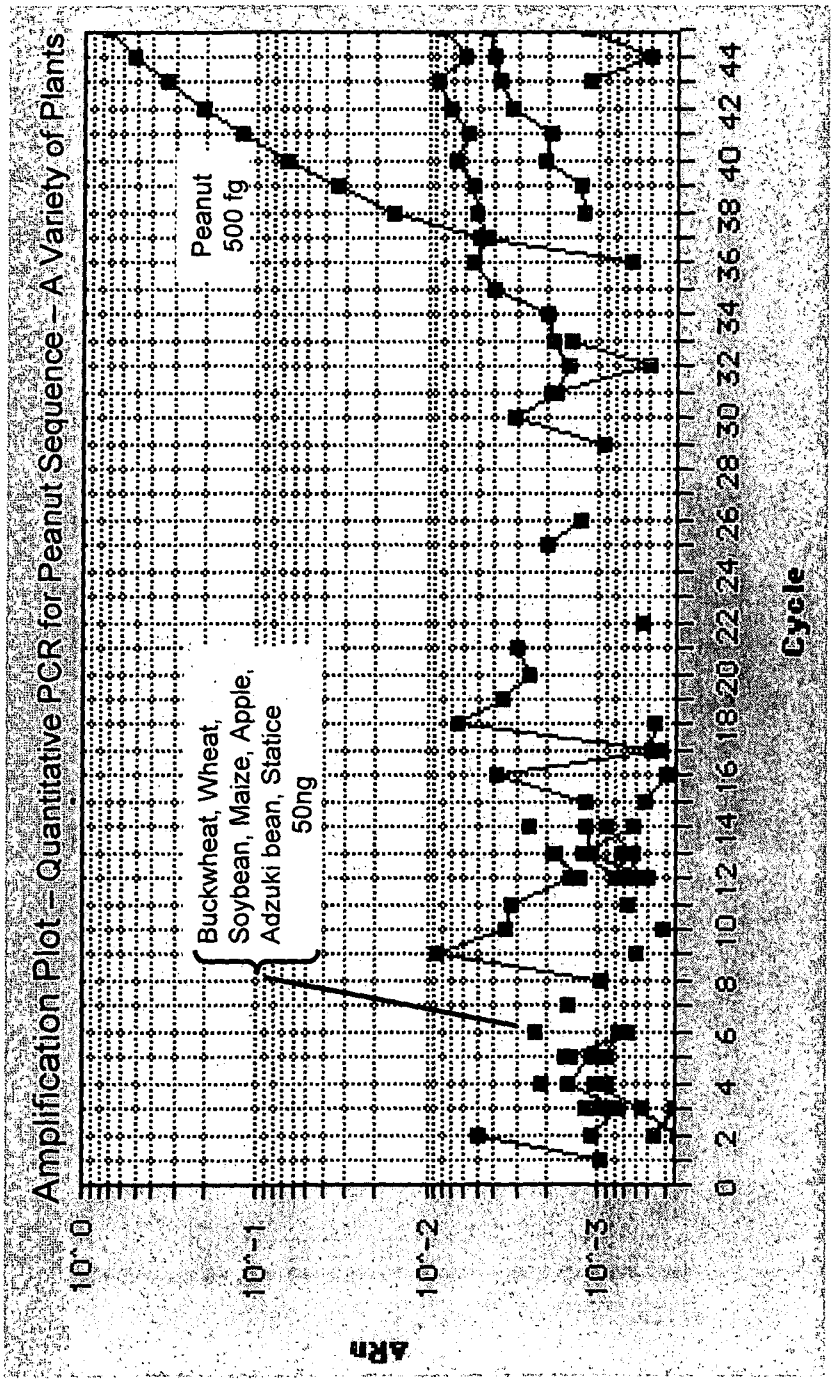
Amplification Plot – Quantitative PCR for Peanut Sequence – A Variety of Plants
Peanut
500 fg
Buckwheat, Wheat,
Soybean, Maize, Apple,
Adzuki bean, Statice
50ng
10^0
10^-1
10^-2
10^-3
ΔRn
0 2 4 6 8 10 12 14 16 18 20 22 24 26 28 30 32 34 36 38 40 42 44
Cycle

FIG.13

FIG.14

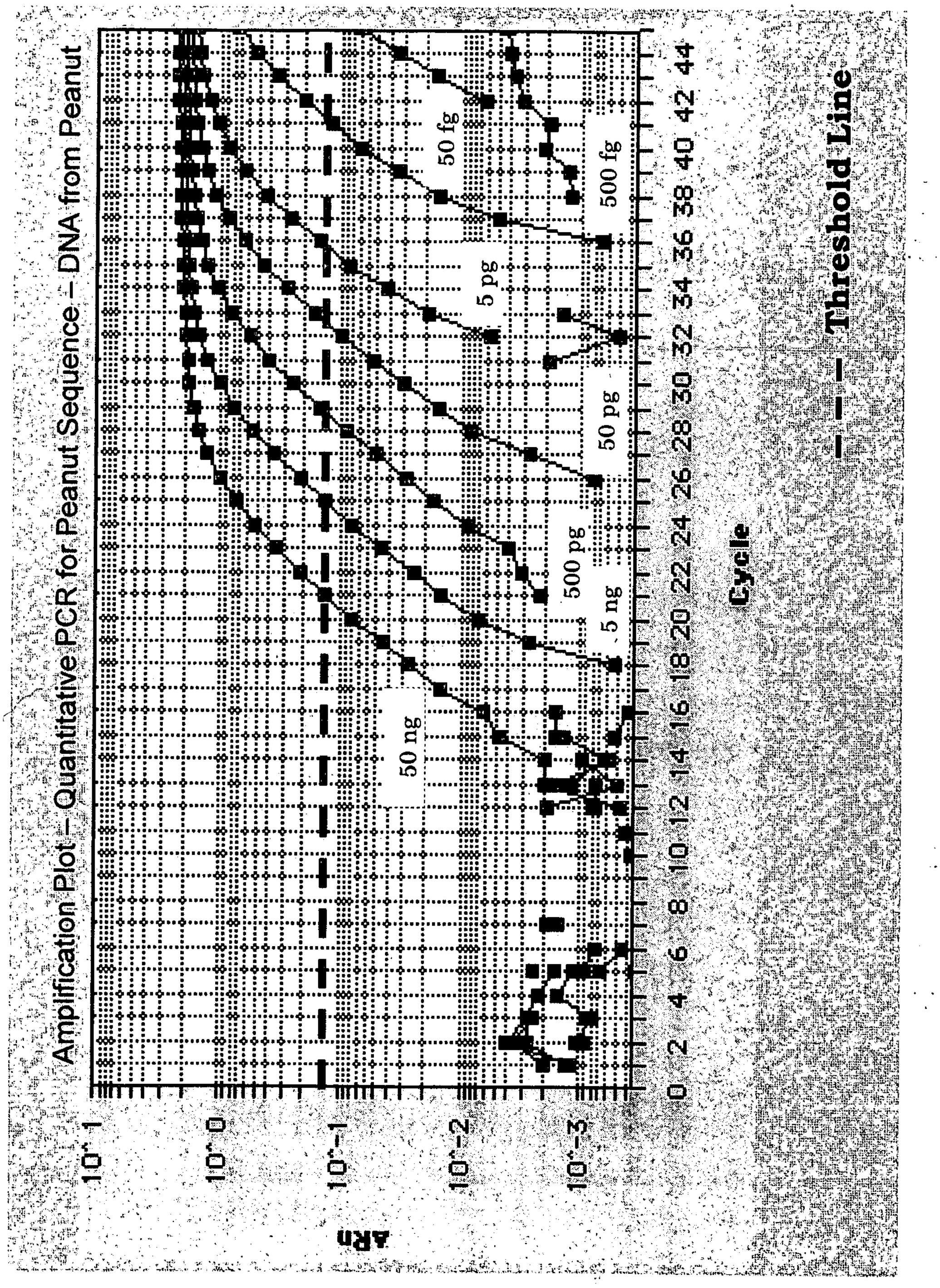
Amplification Plot – Quantitative PCR for Peanut Sequence – DNA from Peanut
ΔRn
10^1
10^0
10^-1
10^-2
10^-3
Cycle
0 2 4 6 8 10 12 14 16 18 20 22 24 26 28 30 32 34 36 38 40 42 44
50 ng
5 ng
500 pg
50 pg
5 pg
500 fg
50 fg
Threshold Line

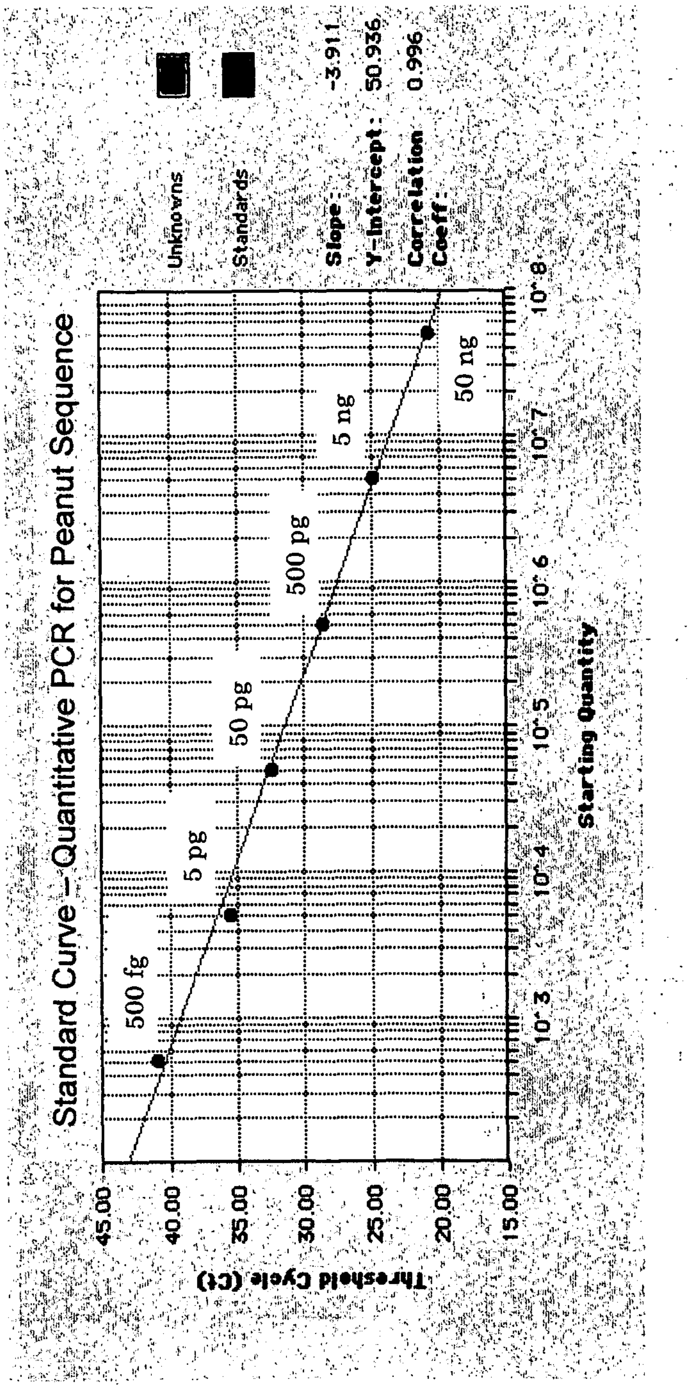
Standard Curve - Quantitative PCR for Peanut Sequence
Unknowns
Standards
Slope : -3.911
Y-Intercept : 50.936
Correlation Coeff : 0.996
500 fg
5 pg
50 pg
500 pg
5 ng
50 ng
Threshold Cycle (Ct)
45.00
40.00
35.00
30.00
25.00
20.00
15.00
10^3
10^4
10^5
10^6
10^7
10^8
Starting Quantity

FIG.15

**INTERNATIONAL SEARCH REPORT**

International application No.
PCT/JP2004/006913

A. CLASSIFICATION OF SUBJECT MATTER
Int.Cl[7] C12N15/29, C12Q1/68

According to International Patent Classification (IPC) or to both national classification and IPC

B. FIELDS SEARCHED

Minimum documentation searched (classification system followed by classification symbols)
Int.Cl[7] C12N15/29, C12N15/11-15/12, C12Q1/68

Documentation searched other than minimum documentation to the extent that such documents are included in the fields searched

Electronic data base consulted during the international search (name of data base and, where practicable, search terms used)
JSTPlus, WPI(DIALOG), BIOSIS(DIALOG), PUBMED, EMBL/DDBJ/Genebank/Geneseq

C. DOCUMENTS CONSIDERED TO BE RELEVANT

| Category* | Citation of document, with indication, where appropriate, of the relevant passages | Relevant to claim No. |
|---|---|---|
| X<br>A | "Allergy Busshitsu o Fukumu Shokuhin no Kensa Hoho ni Tsuite", Notice by Kaku Todofuken Chiji · Kaku Seireishi Shicho · Kaku Tokubetsuku Kucho Ate Kosei Rodosho Iyakukyoku Shokuhin Hoken Bucho, 06 November, 2002 (06.11.02), Shokuhatsu No.1106001 | 13,14,17<br>1-9,15,16,<br>18-28 |
| X<br>A | MATSUOKA T. et al., A multiplex PCR method of detecting recombinant DNAs from five lines of genetically modified maize, J.Food Hyg.Soc. Japan (2001), Vol.42, No.1, pages 24 to 32 | 13,14,17<br>1-9,15,16,<br>18-28 |
| A | HUBNER P. et al., Validation of PCR methods for quantitation of genetically modified plants in food, J.AOAC.Int. (2001), Vol.84, No.6, pages 1855 to 1864 | 1-9,13-28 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 August, 2004 (09.08.04) | 24 August, 2004 (24.08.04) |
| Name and mailing address of the ISA/<br>Japanese Patent Office<br><br>Facsimile No. | Authorized officer<br><br>Telephone No. |

Form PCT/ISA/210 (second sheet) (January 2004)

**INTERNATIONAL SEARCH REPORT**

International application No.
PCT/JP2004/006913

C (Continuation). DOCUMENTS CONSIDERED TO BE RELEVANT

| Category* | Citation of document, with indication, where appropriate, of the relevant passages | Relevant to claim No. |
|---|---|---|
| A | SHINDO Y. et al., Validation of real-time PCR analyses for line-specific quantitation of genetically modified maize and soybean using new reference molecules, J.AOAC.Int. (2002), Vol.85, No.5, pages 1119 to 1126 | 1-9,13-28 |
| A | KURIBARA H. et al., Novel reference molecules for quantitation of genetically modified maize and soybean, J.AOAC.Int. (2002), Vol.85, No.5, pages 1077 to 1089 | 1-9,13-28 |
| A | JP 2001-238700 A (Takara Shuzo Kabushiki Kaisha), 04 September, 2001 (04.09.01), & KR 200186586 A & US 2002/0100082 A | 1-9,13-28 |
| A | WO 02/34943 A1 (National Food Research Institute), 02 May, 2002 (02.05.02), & AU 200212678 A & EP 1335027 A1 & KR 2003051740 A & US 2004/0005605 A1 & BR 200114928 A | 1-9,13-28 |
| A | JP 2002-536024 A (BIOINSIDE GMBH.), 29 October, 2002 (29.10.02), & DE 19906169 A1 & WO 2000/47764 A2 & EP 1144676 A2 & DE 50002062 B & US 2003/0148278 A1 | 1-9,13-28 |

Form PCT/ISA/210 (continuation of second sheet) (January 2004)

**INTERNATIONAL SEARCH REPORT**

International application No.
PCT/JP2004/006913

**Box No. I Nucleotide and/or amino acid sequence(s) (Continuation of item1.b of the first sheet)**

1. With regard to any nucleotide and/or amino acid sequence disclosed in the international application and necessary to the claimed invention, the international search was carried out on the basis of:

   a. type of material
      - [X] a sequence listing
      - [ ] table(s) related to the sequence listing

   b. format of material
      - [ ] in written format
      - [X] in computer readable form

   c. time of filing/furnishing
      - [ ] contained in the international application as filed
      - [X] filed together with the international application in computer readable form
      - [ ] furnished subsequently to this Authority for the purposes of search

2. [X] In addition, in the case that more than one version or copy of a sequence listing and/or table relating thereto has been filed or furnished, the required statements that the information in the subsequent or additional copies is identical to that in the application as filed or does not go beyond the application as filed, as appropriate, were furnished.

3. Additional comments:

Form PCT/ISA/210 (continuation of first sheet (1)) (January 2004)

**INTERNATIONAL SEARCH REPORT**

International application No.
PCT/JP2004/006913

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

See extra sheet.

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.: 1-9 and 14-28.

**Remark on Protest**
☐ The additional search fees were accompanied by the applicant's protest.
☐ No protest accompanied the payment of additional search fees.

Form PCT/ISA/210 (continuation of first sheet (2)) (January 2004)

**INTERNATIONAL SEARCH REPORT**

International application No.
PCT/JP2004/006913

Continuation of Box No.III of continuation of first sheet(2)

The invention relating to the method of determining the amount of arbitrary plant claimed in claim 1 and claims 2 to 9 quoting the same is common to the kit for detection of specified plant specimen including primers for detection of standard plant specimen claimed in claim 13 and claims 14 to 28 quoting the same only in relation to primers for detection of standard plant specimen for use in the detection of specified plant.

Moreover, the primer set for statice detection claimed in claim 10, the primer set for buckwheat genus detection claimed in claim 11 and the primer set for peanut detection claimed in claim 12, as the primers without exception are not those employed only for the method of determining the amount of arbitrary plant involving the step of detecting the standard plant specimen and as the chemical structures of the primers are different from each other, are common to each other only in being the invention relating to primers for specific detection of individual plants.

However, the references 1 to 7 describe primers for specific detection of plants.

Consequently, being primers for specific detection of plants cannot be stated as constituting special technical features within the meaning of PCT Rule 13.2.

Therefore, the inventions of claims 1 to 28 cannot be stated as being a group of inventions linked with each other so as to form a single general inventive concept, and it appears that the invention group consists of four inventions, namely, those relating to the quantitative method and primer set therefor claimed in claims 1-9 and 14-28, the primer set for statice detection claimed in claim 10, the primer set for buckwheat genus detection claimed in claim 11 and the primer set for peanut detection claimed in claim 12.

Reference 1: JP 2001-136983 A
Reference 2: JP 2001-238700 A
Reference 3: JP 2002-536024 A
Reference 4: JP 2003-135082 A
Reference 5: WO 02/34943 A
Reference 6: MATSUOKA T. et al., A multiplex PCR method of detecting recombinant DNAs from five lines of genetically modified maize, J. Food Hyg. Soc. Japan (2001), Vol.42, No.1, pages 24 to 32
Reference 7: "Allergy Busshitsu o Fukumu Shokuhin no Kensa Hoho ni Tsuite", Notice by Kaku Todofuken Chiji · Kaku Seireishi Shicho · Kaku Tokubetsuku Kucho Ate Kosei Rodosho Iyakukyoku Shokuhin Hoken Bucho, 06 November, 2002 (06.11.02), Shokuhatsu No.1106001

Form PCT/ISA/210 (extra sheet) (January 2004)

SEQUENCE LISTING

<110> House Foods Corporation

<120> Quantitative PCR method of detecting specific plant genus in food or food ingredient

<130> PH-2153-PCT

<150> JP 2003-139513

<151> 2003-05-16

<160> 66

<170> PatentIn version 3.1

<210> 1

<211> 73

<212> DNA

<213> Fagopyrum esculentum

<400> 1

caacggatat ctcggctctc gcatcgatga agaacgtagc gaaatgcgat acttggtgtg 60

aattgcagaa tcc 73

<210> 2

<211> 27

<212> DNA

<213> Artificial

<220>

<223> PCR primer

<400> 2

gcatttcgct acgttcttca tcgatgc 27

<210> 3

<211> 26

<212> DNA

<213> Artificial

<220>

<223> PCR primer

<400> 3

atcgcatttc gctacgttct tcatcg 26

<210> 4

<211> 28

<212> DNA

<213> Artificial

<220>

<223> PCR primer

<400> 4

agtatcgcat ttcgctacgt tcttcatc 28

<210> 5

<211> 27

<212> DNA

<213> Artificial

<220>

<223> PCR primer

<400> 5

gcatcgatga agaacgtagc gaaatgc 27

<210> 6

<211> 26

<212> DNA

<213> Artificial

<220>

<223> PCR primer

<400> 6

cgatgaagaa cgtagcgaaa tgcgat 26

<210> 7

<211> 28

<212> DNA

<213> Artificial

<220>

<223> PCR primer

<400> 7 •

gatgaagaac gtagcgaaat gcgatact 28

<210> 8

<211> 71

<212> DNA

<213> Fagopyrum esculentum

<400> 8

acgaaccccg gcgcggactg cgccaaggac cacgaacaga agcgcgtccc gagcctcccg 60

gtccccgggc g 71

<210> 9

<211> 77

<212> DNA

<213> Fagopyrum esculentum

<400> 9

ccgggcggca cggcggcgtc gcgtcgtttc tacgaaacag aacgactctc ggcaacggat 60

atctcggctc tcgcatc 77

<210> 10

<211> 58

<212> DNA

<213> Fagopyrum esculentum

<400> 10

gccggaaggg cgagctcccc cgaaacacca agtacggcgg gcggaccccg aaggccat 58

<210> 11

<211> 25

<212> DNA

<213> Artificial

<220>

<223> PCR primer

<400> 11

ggaccacgaa cagaagcgcg tcccg 25

<210> 12

<211> 21

<212> DNA

<213> Artificial

<220>

<223> PCR primer

<400> 12

cacgaacaga agcgcgtccc g 21

<210> 13

<211> 21

<212> DNA

<213> Artificial

<220>

<223> PCR primer

<400> 13
ggaccacgaa cagaagcgcg t 21

<210> 14
<211> 22
<212> DNA
<213> Artificial

<220>
<223> PCR primer

<400> 14
cgccaaggac cacgaacaga ag 22

<210> 15
<211> 23
<212> DNA
<213> Artificial

<220>
<223> PCR primer

<400> 15
cgttgccgag agtcgttctg ttt 23

<210> 16
<211> 26

<212> DNA

<213> Artificial

<220>

<223> PCR primer

<400> 16

gtcgttctgt ttmktagaaa cgacgc 26

<210> 17

<211> 72

<212> DNA

<213> Arachis villosa

<400> 17

cgccccgtct caaacaagaa caaaaccccg gcgcggaaag cgccaaggaa gccaaacgtt 60

tctgctctcc cc 72

<210> 18

<211> 57

<212> DNA

<213> Arachis villosa

<400> 18

aacgtttctg ctctccccgc cggctccgga gacggcatcc ggtcggggcg acgagtg 57

<210> 19

<211> 60

<212> DNA

<213> Arachis villosa

<400> 19

ccgccggctc cggagacggc atccggtcgg ggcgacgagt gaccacaaga gttaagaacg 60

<210> 20

<211> 68

<212> DNA

<213> Arachis villosa

<400> 20

ggccggccgtg cgcggccgg cgcccccgtct caaacaagaa caaaaccccg gcgcggaaag 60

cgccaagg 68

<210> 21

<211> 20

<212> DNA

<213> Artificial

<220>

<223> PCR primer

<400> 21

gcggaaagcg ccaaggaagc 20

<210> 22

<211> 17

<212> DNA

<213> Artificial

<220>

<223> PCR primer

<400> 22

ggcgcggaaa gcgccaa 17

<210> 23

<211> 19

<212> DNA

<213> Artificial

<220>

<223> PCR primer

<400> 23

caaaaccccg gcgcggaaa 19

<210> 24

<211> 18

<212> DNA

<213> Artificial

<220>

<223> PCR primer

<400> 24

cggcttccgg agacggca 18

<210> 25

<211> 17

<212> DNA

<213> Artificial

<220>

<223> PCR primer

<400> 25

cggctccgga gacggca 17

<210> 26

<211> 17

<212> DNA

<213> Artificial

<220>

<223> PCR primer

<400> 26

cgtcgccccg accggat 17

<210> 27

<211> 18

<212> DNA

<213> Artificial

<220>

<223> PCR primer

<400> 27

tcgtcgcccc gaccggat 18

<210> 28

<211> 19

<212> DNA

<213> Artificial

<220>

<223> PCR primer

<400> 28

ctcgtcgccc cgaccggat 19

<210> 29

<211> 20

<212> DNA

<213> Artificial

<220>

<223> PCR primer

<400> 29

actcgtcgcc ccgaccggat 20

<210> 30

<211> 28

<212> DNA

<213> Artificial

<220>

<223> PCR primer

<400> 30

cgccccgtct caaacaagaa caaaaccc 28

<210> 31

<211> 26

<212> DNA

<213> Artificial

<220>

<223> PCR primer

<400> 31

ccccgtctca aacaagaaca aaaccc 26

<210> 32

<211> 20

<212> DNA

<213> Arachis villosa

<400> 32

cgacgagtga ccacaagagt 20

<210> 33

<211> 24

<212> DNA

<213> Arachis villosa

<400> 33

aacgactctc ggcaacggat atct 24

<210> 34

<211> 16

<212> DNA

<213> Artificial

<220>

<223> PCR probe

<400> 34

tgctctcccc gccggc 16

<210> 35

<211> 36

<212> DNA

<213> Arachis villosa

<400> 35

agaacaaaac cccggcgcgg aaagcgccaa ggaagc 36

<210> 36

<211> 53

<212> DNA

<213> Fagopyrum esculentum

<400> 36

agggcacgcc tgtctgggcg tcacgcaccg cgtcgccccc tccccctcct tcc 53

<210> 37

<211> 56

<212> DNA

<213> Fagopyrum esculentum

<400> 37

aagactacgc atcgcgtcgc gtcgccgcga gccccgggag gaaagacccg agagag 56

<210> 38

<211> 57

<212> DNA

<213> Arachis villosa

<400> 38

acgggctctt ggtggggagc ggcaccgcgg cagatggtgg tcgagaacaa ccctcgt 57

<210> 39

<211> 17

<212> DNA

<213> Artificial

<220>

<223> PCR primer

<400> 39

ccatctgccg cggtgcc 17

<210> 40

<211> 60

<212> DNA

<213> Triticum aestivum

<400> 40

tctcaacggg aatcgggatg cggcatctgg tccctcgtct ctcaagggac ggtggaccga 60

<210> 41

<211> 57

<212> DNA

<213> Triticum aestivum

<400> 41

taccgcgccg gacacagcgc atggtgggcg tcctcgcttt atcaatgcag tgcatcc 57

<210> 42

<211> 57

<212> DNA

<213> Triticum aestivum

<400> 42

taccgtgtcg aacacagcgc atggtgggcg tctttgcttt atcaactgca gtgcata 57

<210> 43

<211> 20

<212> DNA

<213> Artificial

<220>

<223> PCR primer

<400> 43

cggcatctgg tccctcgtct 20

<210> 44

<211> 17

<212> DNA

<213> Artificial

<220>

<223> PCR primer

<400> 44

gcgaggacgc ccaccat 17

<210> 45

<211> 17

<212> DNA

<213> Artificial

<220>

<223> PCR primer

<400> 45

gcaaagacgc ccaccat 17

<210> 46

<211> 58

<212> DNA

<213> Glycine max

<400> 46

gttgctgcgc ggggtgtatg ctgacctccc gcgagcaccc gcctcgtggt tggttgaa 58

<210> 47

<211> 65

<212> DNA

<213> Glycine max

<400> 47

gttcatggcc gacttcgccg tgataaaatg gtggatgagc cacgctcgag accaatcacg 60

tgcga 65

<210> 48

<211> 62

<212> DNA

<213> Glycine max

<400> 48

gttcatggcc gacttcgccg tgataaaatg gatgagccac gctcgaccaa acgtgcgacc 60

gg 62

<210> 49

<211> 18

<212> DNA

<213> Artificial

<220>

<223> PCR primer

<400> 49

ctgacctccc gcgagcac 18

<210> 50

<211> 25

<212> DNA

<213> Artificial

<220>

<223> PCR primer

<400> 50

gcgtggctca tccaccattt tatca 25

<210> 51

<211> 25

<212> DNA

<213> Artificial

<220>

<223> PCR primer

<400> 51

gcgttgctca tccaccattt tatca 25

<210> 52

<211> 25

<212> DNA

<213> Artificial

<220>

<223> PCR primer

<400> 52

gcgttgctca tccaccattt tgtca 25

<210> 53

<211> 25

<212> DNA

<213> Artificial

<220>

<223> PCR primer

<400> 53

gcattgctca tccaccattt tgtca 25

<210> 54

<211> 25

<212> DNA

<213> Artificial

<220>

<223> PCR primer

<400> 54

gcgctgctca tccgccattt tgtca 25

<210> 55

<211> 25

<212> DNA

<213> Artificial

<220>

<223> PCR primer

<400> 55

gcgctgctca tccaccattt tgtca 25

<210> 56

<211> 22

<212> DNA

<213> Artificial

<220>

<223> PCR primer

<400> 56
gcgtggctca tccattttat ca 22

<210> 57
<211> 24
<212> DNA
<213> Artificial

<220>
<223> PCR primer

<400> 57
ttggacgtgt atcccttgtg gttc 24

<210> 58
<211> 24
<212> DNA
<213> Artificial

<220>
<223> PCR primer

<400> 58
cacgaaggtg aaagttgcgt tcat 24

<210> 59
<211> 16
<212> DNA

<213> Artificial

<220>

<223> PCR probe

<400> 59

tgtgcgacgc ggaatg 16

<210> 60

<211> 28

<212> DNA

<213> Artificial

<220>

<223> PCR primer

<400> 60

tctagacgcc aaggaccacg aacagaag 28

<210> 61

<211> 32

<212> DNA

<213> Artificial

<220>

<223> PCR primer

<400> 61

caaaagcttc gttgccgaga gtcgttctgt tt 32

<210> 62

<211> 22

<212> DNA

<213> Artificial

<220>

<223> PCR primer

<400> 62

acgaagcttt tggacgtgta tcccttgtgg ttc 33

<210> 63

<211> 30

<212> DNA

<213> Artificial

<220>

<223> PCR primer

<400> 63

ggatcccacg aaggtgaaag ttgcgttcat 30

<210> 64

<211> 13

<212> DNA

<213> Artificial

<220>

<223> PCR probe

<400> 64
cgggacgcgc ttc 13

<210> 65
<211> 19
<212> DNA
<213> Artificial

<220>
<223> PCR primer

<400> 65
tcgtcgcccc gaccggatg 19

<210> 66
<211> 17
<212> DNA
<213> Artificial

<220>
<223> PCR primer

<400> 66
gtcgccccga ccggatg 17